# EUROPEAN PATENT SPECIFICATION

(11) **EP 3 019 621 B1**
(45) Date of publication and mention of the grant of the patent: **28.11.2018**
(21) Application number: 14738496.0
(22) Date of filing: 10.07.2014
(51) Int. Cl.: C12P 21/00, C12N 9/10, C07K 16/00, C12N 15/80

(54) **PRODUCTION OF GLYCOPROTEINS HAVING INCREASED N-GLYCOSYLATION SITE OCCUPANCY**
HERSTELLUNG VON GLYKOPROTEINEN MIT ERHÖHTER N-GLYKOSYLIERUNGSSTELLENBELEGUNG
PRODUCTION DE GLYCOPROTÉINES AYANT UN TAUX D'OCCUPATION DE SITE DE N-GLYCOSYLATION ACCRU

(30) Priority: 10.07.2013 EP 13175997
(43) Date of publication of application: 18.05.2016
(73) Proprietor: Glykos Finland Oy, 00790 Helsinki (FI)
(72) Inventor: NATUNEN, Jari, FI-01520 Vantaa (FI); LANDOWSKI, Christopher, FI-00650 Helsinki (FI); SALOHEIMO, Markku, FI-00390 Helsinki (FI); OSTERMEIER, Christian, CH-4002 Basel (CH); SOMMER, Benjamin Patrick, CH-4002 Basel (CH); WAHL, Ramon, CH-4002 Basel (CH)
(74) Representative: Cabinet Plasseraud
(86) International application number: PCT/EP2014/064818
(87) International publication number: WO 2015/004239

(56) References cited:
- WO-A1-2010/049177
- WO-A1-2011/106389
- WO-A2-2013/062939
- OWEN P. WARD: "Production of recombinant proteins by filamentous fungi", BIOTECHNOLOGY ADVANCES, vol. 30, no. 5, 1 September 2012 (2012-09-01), pages 1119-1139, XP055088348, ISSN: 0734-9750, DOI: 10.1016/j.biotechadv.2011.09.012
- BYUNG-KWON CHOI ET AL: "Improvement of-glycan site occupancy of therapeutic glycoproteins produced in", APPLIED MICROBIOLOGY AND BIOTECHNOLOGY, SPRINGER, BERLIN, DE, vol. 95, no. 3, 10 May 2012 (2012-05-10), pages 671-682, XP035084625, ISSN: 1432-0614, DOI: 10.1007/S00253-012-4067-3 cited in the application
- KAREN DE POURCQ ET AL: "Engineering of glycosylation in yeast and other fungi: current state and perspectives", APPLIED MICROBIOLOGY AND BIOTECHNOLOGY, SPRINGER, BERLIN, DE, vol. 87, no. 5, 29 June 2010 (2010-06-29), pages 1617-1631, XP019841719, ISSN: 1432-0614 cited in the application
- IRINA S. DRUZHININA ET AL: "Novel traits of Trichoderma predicted through the analysis of its secretome", FEMS MICROBIOLOGY LETTERS, vol. 337, no. 1, 18 December 2012 (2012-12-18), pages 1-9, XP055088413, ISSN: 0378-1097, DOI: 10.1111/j.1574-6968.2012.02665.x
- DIEGO MARTINEZ ET AL: "Genome sequencing and analysis of the biomass-degrading fungus Trichoderma reesei (syn. Hypocrea jecorina)", NATURE BIOTECHNOLOGY, NATURE PUBLISHING GROUP, NEW YORK, NY, US, vol. 26, no. 5, 1 May 2008 (2008-05-01), pages 553-560, XP002689190, ISSN: 1087-0156, DOI: 10.1038/NBT1403 [retrieved on 2008-05-04]
- KAINZ E. ET AL: "N-Glycan Modification in Aspergillus Species", APPLIED AND ENVIRONMENTAL MICROBIOLOGY, vol. 74, no. 4, 14 December 2007 (2007-12-14), pages 1076-1086, XP055041797, ISSN: 0099-2240, DOI: 10.1128/AEM.01058-07

## Description

### TECHNICAL FIELD

The present disclosure relates to compositions and methods useful for the production of heterologous proteins, e.g recombinant antibodies, in filamentous fungal cells.

### BACKGROUND

Posttranslational modification of eukaryotic proteins, particularly therapeutic proteins such as immunoglobulins, is often necessary for proper protein folding and function. Because standard prokaryotic expression systems lack the proper machinery necessary for such modifications, alternative expression systems have to be used in production of these therapeutic proteins. Even where eukaryotic proteins do not have posttranslational modifications, prokaryotic expression systems often lack necessary chaperone proteins required for proper folding. Yeast and fungi are attractive options for expressing proteins as they can be easily grown at a large scale in simple media, which allows low production costs, and yeast and fungi have posttranslational machinery and chaperones that perform similar functions as found in mammalian cells. Moreover, tools are available to manipulate the relatively simple genetic makeup of yeast and fungal cells as well as more complex eukaryotic cells such as mammalian or insect cells (De Pourcq et al., Appl Microbiol Biotechnol, 87(5):1617-31).

However, posttranslational modifications occurring in yeast and fungi may still be a concern for the production of recombinant therapeutic protein. In particular, insufficient N-glycosylation is one of the biggest hurdles to overcome in the production of biopharmaceuticals for human applications in fungi.

N-glycosylation, which refers to the attachment of sugar molecule to a nitrogen atom of an asparagine side chain, has been shown to modulate the pharmacokinetics and pharmacodynamics of therapeutic proteins.

When recombinant proteins are expressed in filamentous fungal cells such as *Trichoderma* fungus cells, the proportion of N-glycosylation sites that are indeed glycosylated is generally lower than for the same protein expressed in a mammalian system, such as CHO cells.

WO2011/106389, entitled "Methods for increasing N-glycosylation site occupancy on therapeutic glycoproteins produced in *Pichia pastoris",* describes *Pichia pastoris* cells that overexpress heterologous single-subunit oligotransferase, and are able to produce glycoproteins with improved N-glycosylation.

Similarly, Choi et al. describe improved N-glycosylation of recombinant proteins by heterologous expression of heterologous single-subunit oligotransferase (Choi et al., Appl Microbiol Biotechnol, 95(3): 671-82).

The same authors have also described, in WO2013062939, methods for increasing N-glycan occupancy and reducing production of hybrid N-glycans in Pichia pastoris strains lacking alpha-1,3 mannosyltransferase activity (Alg3p disruption).

Reports of fungal cell expression systems expressing human-like fucosylated N-glycans are lacking. Indeed, due to the industry's focus on mammalian cell culture technology for such a long time, the fungal cell expression systems such as *Trichoderma* are not as well established for therapeutic protein production as mammalian cell culture and therefore suffer from drawbacks when expressing mammalian proteins. In particular, a need remains in the art for improved filamentous fungal cells, such as *Trichoderma* fungus cells, that can stably produce heterologous proteins with increased N-glycosylation site occupancy, preferably at high levels of expression.

### SUMMARY

The invention is defined in the claims.

The present disclosure relates to improved methods for producing glycoproteins with increased N-glycosylation site occupancy in filamentous fungal expression systems, and more specifically, glycoproteins, such as antibodies or related immunoglobulins or fusion proteins.

The present disclosure is based in part on the surprising discovery that filamentous fungal cells, such as *Trichoderma* cells, can be genetically modified to express oligosaccharyl transferase activity, without adversely affecting yield of produced glycoproteins.

Accordingly, in a first aspect, the disclosure relates to a filamentous fungal cell comprising
i. one or more mutation that reduces or eliminates one or more endogenous protease activity compared to a parental filamentous fungal cell which does not have said mutation(s),
ii. a polynucleotide encoding a heterologous catalytic subunit of oligosaccharyl transferase, and
iii. a polynucleotide encoding a heterologous glycoprotein,
wherein said catalytic subunit of oligosaccharyl transferase is selected from *Leishmania* oligosaccharyl transferase catalytic subunits.

In one embodiment, said filamentous fungal cell has at least a two-fold reduction, preferably at least a three-fold reduction, even more preferably at least a four-fold reduction, at least a five-fold reduction, in total protease activity compared to a parental filamentous fungal cell which does not have the protease-deficient mutations(s).

In one embodiment of the disclosure, said filamentous fungal cell is a *Trichoderma, Neurospora, Myceliophthora, Chrysosporium, Aspergillus,* or *Fusarium* cell.

In one embodiment of the invention, the polynucleotide encoding the heterologous catalytic subunit of oliogaccharyl transferase comprises a nucleic acid sequence selected from the group consisting of SEQ ID NO: 2, SEQ ID NO: 9, SEQ ID NO: 88 and SEQ ID NO: 90 or a polynucleotide encoding a functional variant polypeptide having at least 50%, at least 60%, at least 70% identity, at least 80% identity, at least 90% identity, or at least 95% identity with SEQ ID NO: 1, SEQ ID NO: 8, SEQ ID NO: 89 or SEQ ID NO: 91, said functional variant polypeptide having oligosaccharyltransferase activity.

In another embodiment, said polynucleotide encoding the heterologous catalytic subunit of oligosaccharyl transferase is under the control of a promoter for constitutive expression of said oligosaccharyl transferase in said cell.

In one embodiment of the disclosure, the N-glycosylation site occupancy of the heterologous glycoprotein expressed in filamentous fungal cell is at least 80%, at least 90%, at least 95%, at least 99%, or 100%.

In a specific embodiment, the N-glycosylation site occupancy of the heterologous glycoprotein is at least 95% and Man3, Man5, G0, G1 and/or G2 glycoforms represent at least 50% of total neutral N-glycans of the heterologous glycoprotein.

In one embodiment of the disclosure, the filamentous fungal cell is a *Trichoderma* cell, for example, *Trichoderma reesei,* and said cell comprises mutations that reduce or eliminate the activity of
- the three endogenous proteases pep1, tsp1, and slp1;
- the three endogenous proteases gap1, slp1, and pep1;
- the three endogenous proteases selected from the group consisting of pep1, pep2, pep3, pep4, pep5, pep8, pep9, pep11, pep12, tsp1, slp1, slp2, slp3, slp7, gap1 and gap2;
- three to six proteases selected from the group consisting of pep1, pep2, pep3, pep4, pep5, tsp1, slp1, slp2, slp3, gap1 and gap2;
- seven to ten proteases selected from the group consisting of pep1, pep2, pep3, pep4, pep5, pep7, pep8, pep9, tsp1, slp1, slp2, slp3, slp5, slp6, slp7, slp8, tpp1, gap1 and gap2.

In one embodiment, the fungal cell further comprises a mutation in the gene encoding ALG3 that reduces or eliminates the corresponding ALG3 expression compared to the level of expression of ALG3 gene in a parental cell which does not have such mutation.

In one embodiment, the fungal cell further comprises a polynucleotide encoding an N-acetylglucosaminyltransferase I catalytic domain and an N-acetylglucosaminyltransferase II catalytic domain.

In one embodiment, the fungal cell further comprises one or more polynucleotides encoding a polypeptide selected from the group consisting of:
i. α1, 2 mannosidase;
ii. N-acetylglucosaminyltransferase I catalytic domain;
iii. α-mannosidase II;
iv. N-acetylglucosaminyltransferase II catalytic domain;
v. β1,4 galactosyltransferase; and,
vi. fucosyltransferase.

In one embodiment of the disclosure, the heterologous glycoprotein is a mammalian glycoprotein.

In a specific embodiment, said mammalian glycoprotein is selected from the group consisting of an antibody, an immunoglobulin or a protein fusion comprising Fc fragment of an immunoglobulin.

In another specific embodiment, said mammalian glycoprotein is a therapeutic antibody.

In another aspect, the disclosure also relates to a method of increasing N-glycosylation site occupancy of heterologous glycoprotein produced in a filamentous fungal host cell, comprising:
a) providing a filamentous fungal host cell, for example a *Trichoderma* cell, having a *Leishmania* STT3D gene encoding a catalytic subunit of oligosaccharyl transferase, or a functional variant thereof, and a polynucleotide encoding a heterologous glycoprotein,
b) culturing the host cell under appropriate conditions for expression of the STT3D gene or its functional variant, or said functional variant, and the production of the heterologous glycoprotein;
wherein the expressed heterologous glycoproteins exhibit increased N-glycosylation site occupancy compared to the heterologous glycoproteins expressed in a corresponding parental filamentous fungal cell which does not express said oligosaccharyl transferase catalytic subunit.

The disclosure also relates to a method of producing a heterologous glycoprotein composition, with increased N-glycosylation site occupancy, comprising:
a) providing a filamentous fungal cell, for example a *Trichoderma* cell, having a *Leishmania* STT3D gene encoding a catalytic subunit of oligosaccharyl transferase, or a functional variant thereof, and a polynucleotide encoding a heterologous glycoprotein,
b) culturing the cell under appropriate conditions for expression of the STT3D gene or its functional variant, and the production of the heterologous glycoprotein composition; and,
c) recovering and, optionally, purifying the heterologous glycoprotein composition.

In certain embodiments of the method of the disclosure, said *Leishmania* STT3D gene encoding a catalytic subunit of oligosaccharyl transferase comprises a nucleic acid sequence selected from the group consisting of SEQ ID NO: 2, SEQ ID NO: 9, SEQ ID NO: 88 and SEQ ID NO: 90, or a polynucleotide encoding a functional variant polypeptide having at least 50%, at least 60%, at least 70% identity, at least 80% identity, at least 90% identity, or at least 95% identity with SEQ ID NO: 1, SEQ ID NO: 8, SEQ ID NO: 89 or SEQ ID NO: 91, said functional variant polypeptide having oligosaccharyltransferase activity.

In one embodiment, said polynucleotide encoding said heterologous glycoprotein further comprises a polynucleotide encoding CBH1 catalytic domain and linker as a carrier protein and/or cbh1 promoter.

In one embodiment of the disclosure, the culturing is in a medium comprises a protease inhibitor.

In a specific embodiment, the culturing is in a medium comprising one or two protease inhibitors selected from SBTI and chymostatin.

In one embodiment of the method of the disclosure, the N-glycosylation site occupancy of the produced glycoprotein composition is at least 80%, at least 90%, at least 95%, at least 99%, or 100%.

In one aspect, the disclosure also relates to a glycoprotein composition obtainable by the method described above.

In one aspect, the disclosure relates to an antibody composition obtainable by the method described above.

In one embodiment the disclosure relates to the antibody composition described above, wherein N-glycosylation site occupancy is at least 80%, at least 90%, at least 95%, at least 99%, or 100%.

In one embodiment the disclosure relates to the antibody composition described above, wherein said antibody composition further comprises, as a major glycoform, either:
i. Manα3[Manα6(Manα3)Manα6]Manβ4GlcNAβ4GlcNAc (Man5 glycoform);
ii. GlcNAcβ2Manα3[Manα6(Manα3)Manα6]Manβ4GlcNAβ4GlcNAc (GlcNAcMan5 glycoform);
iii. Manα6(Manα3)Manβ4GlcNAβ4GlcNAc (Man3 glycoform);
iv. Manα6(GlcNAcβ2Manα3)Manβ4GlcNAβ4GlcNAc (GlcNAcMan3 glycoform); or,
v. complex type N-glycans selected from the G0, G1, or G2 glycoform.

### DESCRIPTION OF THE FIGURES

**Figure 1****.** Schematic expression cassette design for *Leishmania major* STT3 targeted to the xylanase 1 locus.
**Figure 2****.** Example spectra of parental strain M317 (pyr4- of M304) and L. major STT3 clone 26B-a (M421). K means lysine.
**Figure 3****.** Schematic map of the STT3 expression cassettes.
**Figure 4****.** Glycan structures produced in Δalg3 strains.
**Figure 5****.** Normalized protease activity data from culture supernatants from the protease deletion supernatants and the parent strain. Protease activity was measured at pH 5.5 in first 5 strains and at pH 4.5 in the last three deletion strains. Protease activity is against green fluorescent casein. The six protease deletion strain has only 6% of the wild type parent strain and the 7 protease deletion strain protease activity was about 40% less than the 6 protease deletion strain activity.

### DETAILED DESCRIPTION

### Definitions

As used herein, an "expression system" or a "host cell" refers to the cell that is genetically modified to enable the transcription, translation and proper folding of a polypeptide or a protein of interest, typically of mammalian protein.

The term "polynucleotide" or "oligonucleotide" or "nucleic acid" as used herein typically refers to a polymer of at least two nucleotides joined together by a phosphodiester bond and may consist of either ribonucleotides or deoxynucleotides or their derivatives that can be introduced into a host cell for genetic modification of such host cell. For example, a polynucleotide may encode a coding sequence of a protein, and/or comprise control or regulatory sequences of a coding sequence of a protein, such as enhancer or promoter sequences or terminator. A polynucleotide may for example comprise native coding sequence of a gene or their fragments, or variant sequences that have been optimized for optimal gene expression in a specific host cell (for example to take into account codon bias).

As used herein, the term, "optimized" with reference to a polynucleotide means that a polynucleotide has been altered to encode an amino acid sequence using codons that are preferred in the production cell or organism, for example, a filamentous fungal cell such as a *Trichoderma* cell. Heterologous nucleotide sequences that are transfected in a host cell are typically optimized to retain completely or as much as possible the amino acid sequence originally encoded by the original (not optimized) nucleotide sequence. The optimized sequences herein have been engineered to have codons that are preferred in the corresponding production cell or organism, for example the filamentous fungal cell. The amino acid sequences encoded by optimized nucleotide sequences may also be referred to as optimized.

As used herein, a "peptide" or a "polypeptide" is an amino acid sequence including a plurality of consecutive polymerized amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide or a combination of more than one peptide or polypeptide assembled together by covalent or non-covalent bonds. Unless specified, the term "protein" may encompass one or more amino acid sequences with their post-translation modifications, and in particular with either O-mannosylation or N-glycan modifications.

As used herein, the term "glycoprotein" refers to a protein which comprises at least one N-linked glycan attached to at least one asparagine residue of a protein, or at least one mannose attached to at least one serine or threonine resulting in O-mannosylation. Since glycoproteins as produced in a host cell expression system are usually produced as a mixture of different glycosylation patterns, the terms "glycoprotein" or "glycoprotein composition" encompass the mixtures of glycoproteins as produced by a host cell, with different glycosylation patterns, unless specifically defined.

The terms "N-glycosylation" or "oligosaccharyl transferase activity" are used herein to refer to the covalent linkage of at least an oligosaccharide chain to the side-chain amide nitrogen of asparagine residue (Asn) of a polypeptide.

As used herein, "glycan" refers to an oligosaccharide chain that can be linked to a carrier such as an amino acid, peptide, polypeptide, lipid or a reducing end conjugate. In certain embodiments, the disclosure relates to N-linked glycans ("N-glycan") conjugated to a polypeptide N-glycosylation site such as -Asn-Xaa-Ser/Thr- by N-linkage to side-chain amide nitrogen of asparagine residue (Asn), where Xaa is any amino acid residue except Pro. The disclosure may further relate to glycans as part of dolichol-phospho-oligosaccharide (Dol-P-P-OS) precursor lipid structures, which are precursors of N-linked glycans in the endoplasmic reticulum of eukaryotic cells. The precursor oligosaccharides are linked from their reducing end to two phosphate residues on the dolichol lipid. For example, α3-mannosyltransferase Alg3 modifies the Dol-P-P-oligosaccharide precursor of N-glycans. Generally, the glycan structures described herein are terminal glycan structures, where the non-reducing residues are not modified by other monosaccharide residue or residues.

As used throughout the present disclosure, glycolipid and carbohydrate nomenclature is essentially according to recommendations by the IUPAC-IUB Commission on Biochemical Nomenclature (e.g. Carbohydrate Res. 1998, 312, 167; Carbohydrate Res. 1997, 297, 1; Eur. J. Biochem. 1998, 257, 29). It is assumed that Gal (galactose), Glc (glucose), GlcNAc (N-acetylglucosamine), GalNAc (N-acetylgalactosamine), Man (mannose), and Neu5Ac are of the D-configuration, Fuc of the L-configuration, and all the monosaccharide units in the pyranose form (D-Galp, D-Glcp, D-GlcpNAc, D-GalpNAc, D-Manp, L-Fucp, D-Neup5Ac). The amine group is as defined for natural galactose and glucosamines on the 2-position of GalNAc or GlcNAc. Glycosidic linkages are shown partly in shorter and partly in longer nomenclature, the linkages of the sialic acid SA/Neu5X-residues α3 and α6 mean the same as α2-3 and α2-6, respectively, and for hexose monosaccharide residues α1-3, α1-6, β1-2, β1-3, β1-4, and β1-6 can be shortened as α3, α6, β2, β3, β4, and β6, respectively. Lactosamine refers to type II N-acetyllactosamine, Galβ4GlcNAc, and/or type I N-acetyllactosamine. Galβ3GlcNAc and sialic acid (SA) refer to N-acetylneuraminic acid (Neu5Ac), N-glycolylneuraminic acid (Neu5Gc), or any other natural sialic acid including derivatives of Neu5X. Sialic acid is referred to as NeuNX or Neu5X, where preferably X is Ac or Gc. Occasionally Neu5Ac/Gc/X may be referred to as NeuNAc/NeuNGc/NeuNX.

The sugars typically constituting N-glycans found in mammalian glycoprotein, include, without limitation, N-acetylglucosamine (abbreviated hereafter as "GlcNAc"), mannose (abbreviated hereafter as "Man"), glucose (abbreviated hereafter as "Glc"), galactose (abbreviated hereafter as "Gal"), and sialic acid (abbreviated hereafter as "Neu5Ac"). N-glycans share a common pentasaccharide referred to as the "core" structure Man₃GlcNAc₂ (Manα6(Manα3)Manβ4GlcNAβ4GlcNAc, referred to as Man3).

In some embodiments Man3 glycan or its derivative Manα6(GlcNAcβ2Manα3)Manβ4GlcNAβ4GlcNAc is the major glycoform. When a fucose is attached to the core structure, preferably α6-linked to reducing end GlcNAc, the N-glycan or the core of N-glycan, may be represented as Man₃GlcNAc₂(Fuc). In an embodiment the major N-glycan is Manα3[Manα6(Manα3)Manα6]Manβ4GlcNAβ4GlcNAc (Man5).

Preferred hybrid type N-glycans comprise GlcNAcβ2Manα3[Manα6(Manα3)Manα6]Manβ4GlcNAβ4GlcNAc ("GlcNAcMan5"), or b4-galactosylated derivatives thereof Galβ4GlcNAcMan3, G1, G2, or GalGlcNAcMan5 glycoform.

A "complex N-glycan" refers to a N-glycan which has at least one GlcNAc residue, optionally by GlcNAcβ2-residue, on terminal 1,3 mannose arm of the core structure and at least one GlcNAc residue, optionally by GlcNAcβ2-residue, on terminal 1,6 mannose arm of the core structure.

Such complex N-glycans include, without limitation, GlcNAc₂Man₃GlcNAc₂ (also referred as GO glycoform), Gal₁GlcNAc₂Man₃GlcNAc₂ (also referred as G1 glycoform), and Gal₂GlcNAc₂Man₃GlcNAc₂ (also referred as G2 glycoform), and their core fucosylated glycoforms FG0, FG1 and FG2, respectively GlcNAc₂Man₃GlcNAc₂(Fuc), Gal₁GlcNAc₂Man₃GlcNAc₂(Fuc), and Gal₂GlcNAc₂Man₃GlcNAc₂(Fuc).

As used herein, the expression "neutral N-glycan" has its general meaning in the art. It refers to non-sialylated N-glycans. In contrast, sialylated N-glycans are acidic.

"Increased" or "Reduced activity of an endogenous enzyme": The filamentous fungal cell may have increased or reduced levels of activity of various endogenous enzymes. A reduced level of activity may be provided by inhibiting the activity of the endogenous enzyme with an inhibitor, an antibody, or the like. In certain embodiments, the filamentous fungal cell is genetically modified in ways to increase or reduce activity of various endogenous enzymes. "Genetically modified" refers to any recombinant DNA or RNA method used to create a prokaryotic or eukaryotic host cell that expresses a polypeptide at elevated levels, at lowered levels, or in a mutated form. In other words, the host cell has been transfected, transformed, or transduced with a recombinant polynucleotide molecule, and thereby been altered so as to cause the cell to alter expression of a desired protein.

"Genetic modifications" which result in a decrease or deficiency in gene expression, in the function of the gene, or in the function of the gene product (i.e., the protein encoded by the gene) can be referred to as inactivation (complete or partial), knock-out, deletion, disruption, interruption, blockage, silencing, or down-regulation, or attenuation of expression of a gene. For example, a genetic modification in a gene which results in a decrease in the function of the protein encoded by such gene, can be the result of a complete deletion of the gene (i.e., the gene does not exist, and therefore the protein does not exist), a mutation in the gene which results in incomplete (disruption) or no translation of the protein (e.g., the protein is not expressed), or a mutation in the gene which decreases or abolishes the natural function of the protein (e.g., a protein is expressed which has decreased or no enzymatic activity or action). More specifically, reference to decreasing the action of proteins discussed herein generally refers to any genetic modification in the host cell in question, which results in decreased expression and/or functionality (biological activity) of the proteins and includes decreased activity of the proteins (e.g., decreased catalysis), increased inhibition or degradation of the proteins as well as a reduction or elimination of expression of the proteins. For example, the action or activity of a protein can be decreased by blocking or reducing the production of the protein, reducing protein action, or inhibiting the action of the protein. Combinations of some of these modifications are also possible. Blocking or reducing the production of a protein can include placing the gene encoding the protein under the control of a promoter that requires the presence of an inducing compound in the growth medium. By establishing conditions such that the inducer becomes depleted from the medium, the expression of the gene encoding the protein (and therefore, of protein synthesis) could be turned off. Blocking or reducing the action of a protein could also include using an excision technology approach similar to that described in U.S. Pat. No. 4,743,546. To use this approach, the gene encoding the protein of interest is cloned between specific genetic sequences that allow specific, controlled excision of the gene from the genome. Excision could be prompted by, for example, a shift in the cultivation temperature of the culture, as in U.S. Pat. No. 4,743,546, or by some other physical or nutritional signal.

In general, according to the present disclosure, an increase or a decrease in a given characteristic of a mutant or modified protein (e.g., enzyme activity) is made with reference to the same characteristic of a parent (i.e., normal, not modified) protein that is derived from the same organism (from the same source or parent sequence), which is measured or established under the same or equivalent conditions. Similarly, an increase or decrease in a characteristic of a genetically modified host cell (e.g., expression and/or biological activity of a protein, or production of a product) is made with reference to the same characteristic of a wild-type host cell of the same species, and preferably the same strain, under the same or equivalent conditions. Such conditions include the assay or culture conditions (e.g., medium components, temperature, pH, etc.) under which the activity of the protein (e.g., expression or biological activity) or other characteristic of the host cell is measured, as well as the type of assay used, the host cell that is evaluated, etc. As discussed above, equivalent conditions are conditions (e.g., culture conditions) which are similar, but not necessarily identical (e.g., some conservative changes in conditions can be tolerated), and which do not substantially change the effect on cell growth or enzyme expression or biological activity as compared to a comparison made under the same conditions.

Preferably, a genetically modified host cell that has a genetic modification that increases or decreases (reduces) the activity of a given protein (e.g., a protease) has an increase or decrease, respectively, in the activity or action (e.g., expression, production and/or biological activity) of the protein, as compared to the activity of the protein in a parent host cell (which does not have such genetic modification), of at least about 5%, and more preferably at least about 10%, 15%, 20%, 25%, 30%, 35%, 40%, 45%, 50%, 55 60%, 65%, 70%, 75 80%, 85 90%, 95%, or any percentage, in whole integers between 5% and 100% (e.g., 6%, 7%, 8%, etc.).

In another aspect of the disclosure, a genetically modified host cell that has a genetic modification that increases or decreases (reduces) the activity of a given protein (e.g., a protease) has an increase or decrease, respectively, in the activity or action (e.g., expression, production and/or biological activity) of the protein, as compared to the activity of the wild-type protein in a parent host cell, of at least about 2-fold, and more preferably at least about 5-fold, 10-fold, 20-fold, 30-fold, 40-fold, 50-fold, 75-fold, 100-fold, 125-fold, 150-fold, or any whole integer increment starting from at least about 2-fold (e.g., 3-fold, 4-fold, 5-fold, 6-fold, etc.).

As used herein, the terms "identical" or "per cent identity," in the context of two or more nucleic acid or amino acid sequences, refers to two or more sequences or subsequences that are the same. Two sequences are "substantially identical" if two sequences have a specified percentage of amino acid residues or nucleotides that are the same (i.e., 29% identity, optionally 30%, 40%, 45%, 50%, 55%, 60%, 65%, 70%, 75%, 80%, 85%, 90%, 95%, 99% or 100% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200, or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. When comparing two sequences for identity, it is not necessary that the sequences be contiguous, but any gap would carry with it a penalty that would reduce the overall percent identity. For blastn, the default parameters are Gap opening penalty=5 and Gap extension penalty=2. For blastp, the default parameters are Gap opening penalty=11 and Gap extension penalty=1.

A "comparison window," as used herein, includes reference to a segment of any one of the number of contiguous positions including, but not limited to from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, e.g., by the local homology algorithm of Smith and Waterman (1981), by the homology alignment algorithm of Needleman and Wunsch (1970) J Mol Biol 48(3):443-453, by the search for similarity method of Pearson and Lipman (1988) Proc Natl Acad Sci USA 85(8):2444-2448, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection [see, e.g., Brent et al., (2003) Current Protocols in Molecular Biology, John Wiley & Sons, Inc. (Ringbou Ed)].

Two examples of algorithms that are suitable for determining percent sequence identity and sequence similarity are the BLAST and BLAST 2.0 algorithms, which are described in Altschul et al. (1997) Nucleic Acids Res 25(17):3389-3402 and Altschul et al. (1990) J. Mol Biol 215(3)-403-410, respectively. Software for performing BLAST analyses is publicly available through the National Center for Biotechnology Information. The BLASTN program (for nucleotide sequences) uses as defaults a word length (W) of 11, an expectation (E) or 10, M=5, N=-4, and a comparison of both strands. For amino acid sequences, the BLASTP program uses as defaults a word length of 3, and expectation (E) of 10, and the BLOSUM62 scoring matrix [see Henikoff and Henikoff, (1992) Proc Natl Acad Sci USA 89(22):10915-10919] alignments (B) of 50, expectation (E) of 10, M=5, N=-4, and a comparison of both strands.

The BLAST algorithm also performs a statistical analysis of the similarity between two sequences (see, e.g., Karlin and Altschul, (1993) Proc Natl Acad Sci USA 90(12):5873-5877). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

"Functional variant" or "functional homologous gene" as used herein refers to a coding sequence or a protein having sequence similarity with a reference sequence, typically, at least 30%, 40%, 50%, 60%, 70%, 80%, 90% or 95% identity with the reference coding sequence or protein, and retaining substantially the same function as said reference coding sequence or protein. A functional variant may retain the same function but with reduced or increased activity. Functional variants include natural variants, for example, homologs from different species or artificial variants, resulting from the introduction of a mutation in the coding sequence. Functional variant may be a variant with only conservatively modified mutations.

"Conservatively modified mutations" as used herein include individual substitutions, deletions or additions to an encoded amino acid sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the disclosure. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

### Filamentous fungal cells

As used herein, "filamentous fungal cells" include cells from all filamentous forms of the subdivision Eumycota and Oomycota (as defined by Hawksworth et al., In, Ainsworth and Bisby's Dictionary of The Fungi, 8th edition, 1995, CAB International, University Press, Cambridge, UK). Filamentous fungal cells are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

Preferably, the filamentous fungal cell is not adversely affected by the transduction of the necessary nucleic acid sequences, the subsequent expression of the proteins (e.g., mammalian proteins), or the resulting intermediates. General methods to disrupt genes of and cultivate filamentous fungal cells are disclosed, for example, for Penicillium, in Kopke et al. (2010) Appl Environ Microbiol. 76(14):4664-74. doi: 10.1128/AEM.00670-10, for Aspergillus, in Maruyama and Kitamoto (2011), Methods in Molecular Biology, vol. 765, DOI10.1007/978-1-61779-197-0_27; for Neurospora, in Collopy et al. (2010) Methods Mol Biol. 2010;638:33-40. doi: 10.1007/978-1-60761-611-5_3; and for *Myceliophthora* or *Chrysosporium* PCT/NL2010/000045 and PCT/EP98/06496.

Examples of suitable filamentous fungal cells include, without limitation, cells from an *Acremonium, Aspergillus, Fusarium, Humicola, Mucor, Myceliophthora, Neurospora, Penicillium, Scytalidium, Thielavia, Tolypocladium,* or *Trichoderma*/*Hypocrea* strain.

In certain embodiments, the filamentous fungal cell is from a *Trichoderma* sp., *Acremonium, Aspergillus, Aureobasidium, Cryptococcus, Chrysosporium, Chrysosporium lucknowense, Filibasidium, Fusarium, Gibberella, Magnaporthe, Mucor, Myceliophthora, Myrothecium, Neocallimastix, Neurospora, Paecilomyces, Penicillium, Piromyces, Schizophyllum, Talaromyces, Thermoascus, Thielavia,* or *Tolypocladium* strain.

In some embodiments, the filamentous fungal cell is a *Myceliophthora or Chrysosporium, Neurospora, Aspergillus, Fusarium* or *Trichoderma* strain.

Aspergillus fungal cells of the present disclosure may include, without limitation, *Aspergillus aculeatus, Aspergillus awamori, Aspergillus clavatus, Aspergillus flavus, Aspergillus foetidus, Aspergillus fumigatus, Aspergillus japonicus, Aspergillus nidulans, Aspergillus niger, Aspergillus oryzae,* or *Aspergillus terreus.*

*Neurospora* fungal cells of the present disclosure may include, without limitation, *Neurospora crassa.*

*Myceliophthora* fungal cells of the present disclosure may include, without limitation, *Myceliophthora thermophila.*

In a preferred embodiment, the filamentous fungal cell is a *Trichoderma* fungal cell. *Trichoderma* fungal cells of the present disclosure may be derived from a wild-type *Trichoderma* strain or a mutant thereof. Examples of suitable *Trichoderma* fungal cells include, without limitation, *Trichoderma harzianum, Trichoderma koningii, Trichoderma longibrachiatum, Trichoderma reesei, Trichoderma atroviride, Trichoderma virens, Trichoderma viride;* and alternative sexual form thereof (i.e., *Hypocrea*).

In a more preferred embodiment, the filamentous fungal cell is a *Trichoderma reesei,* and for example, strains derived from ATCC 13631 (QM 6a), ATCC 24449 (radiation mutant 207 of QM 6a), ATCC 26921 (QM 9414; mutant of ATCC 24449), VTT-D-00775 (Selinheimo et al., FEBS J., 2006, 273: 4322-4335), Rut-C30 (ATCC 56765), RL-P37 (NRRL 15709) or T. harzianum isolate T3 (Wolffhechel, H., 1989).

The disclosure described herein relates to a filamentous fungal cell, for example selected from *Trichoderma, Neurospora, Myceliophthora* or a *Chrysosporium* cells, such as *Trichoderma reesei* fungal cell, comprising:
i. one or more mutation that reduces or eliminates one or more endogenous protease activity compared to a parental filamentous fungal cell which does not have said mutation(s),
ii. a polynucleotide encoding a heterologous catalytic subunit of oligosaccharyl transferase, and
iii. a polynucleotide encoding a heterologous glycoprotein,
wherein said catalytic subunit of oligosaccharyl transferase is selected from *Leishmania* oligosaccharyl transferase catalytic subunits.

### Proteases with reduced activity

It has been found that reducing protease activity enables to increase substantially the production of heterologous mammalian protein. Indeed, such proteases found in filamentous fungal cells that express a heterologous protein normally catalyse significant degradation of the expressed recombinant protein. Thus, by reducing the activity of proteases in filamentous fungal cells that express a heterologous protein, the stability of the expressed protein is increased, resulting in an increased level of production of the protein, and in some circumstances, improved quality of the produced protein (e.g., full-length instead of degraded).

Proteases include, without limitation, aspartic proteases, trypsin-like serine proteases, subtilisin proteases, glutamic proteases, and sedolisin proteases. Such proteases may be identified and isolated from filamentous fungal cells and tested to determine whether reduction in their activity affects the production of a recombinant polypeptide from the filamentous fungal cell. Methods for identifying and isolating proteases are well known in the art, and include, without limitation, affinity chromatography, zymogram assays, and gel electrophoresis. An identified protease may then be tested by deleting the gene encoding the identified protease from a filamentous fungal cell that expresses a recombinant polypeptide, such a heterologous or mammalian polypeptide, and determining whether the deletion results in a decrease in total protease activity of the cell, and an increase in the level of production of the expressed recombinant polypeptide. Methods for deleting genes, measuring total protease activity, and measuring levels of produced protein are well known in the art and include the methods described herein.

### Aspartic Proteases

Aspartic proteases are enzymes that use an aspartate residue for hydrolysis of the peptide bonds in polypeptides and proteins. Typically, aspartic proteases contain two highly-conserved aspartate residues in their active site which are optimally active at acidic pH. Aspartic proteases from eukaryotic organisms such as *Trichoderma* fungi include pepsins, cathepsins, and renins. Such aspartic proteases have a two-domain structure, which is thought to arise from ancestral gene duplication. Consistent with such a duplication event, the overall fold of each domain is similar, though the sequences of the two domains have begun to diverge. Each domain contributes one of the catalytic aspartate residues. The active site is in a cleft formed by the two domains of the aspartic proteases. Eukaryotic aspartic proteases further include conserved disulfide bridges, which can assist in identification of the polypeptides as being aspartic acid proteases.
Ten aspartic proteases have been identified in *Trichoderma* fungal cells: *pep1* (tre74156); *pep2* (tre53961); *pep3* (tre121133); *pep4* (tre77579), *pep5* (tre81004), and *pep7* (tre58669), *pep8* (tre122076), *pep9* (tre79807), *pep11* (121306), and *pep12* (tre119876).
Examples of suitable aspartic proteases include, without limitation, *Trichoderma reesei* pep1 (SEQ ID NO: 22), *Trichoderma reesei* pep2 (SEQ ID NO: 18), *Trichoderma reesei* pep3 (SEQ ID NO: 19); *Trichoderma reesei* pep4 (SEQ ID NO: 20), *Trichoderma reesei* pep5 (SEQ ID NO: 21) and *Trichoderma reesei* pep7 (SEQ ID NO:23), *Trichoderma reesei* EGR48424 pep8 (SEQ ID NO:85), *Trichoderma reesei pep9* (SEQ ID NO:87), *Trichoderma reesei* EGR49498 *pep11* (SEQ ID NO:86), *Trichoderma reesei* EGR52517 *pep12* (SEQ ID NO:35), and homologs thereof. Examples of homologs of pep1, pep2, pep3, pep4, pep5, pep7, pep8, pep11 and pep12 proteases identified in other organisms are also described in PCT/EP/2013/050186.

### Trypsin-Like Serine Proteases

Trypsin-like serine proteases are enzymes with substrate specificity similar to that of trypsin. Trypsin-like serine proteases use a serine residue for hydrolysis of the peptide bonds in polypeptides and proteins. Typically, trypsin-like serine proteases cleave peptide bonds following a positively-charged amino acid residue. Trypsin-like serine proteases from eukaryotic organisms such as *Trichoderma* fungi include trypsin 1, trypsin 2, and mesotrypsin. Such trypsin-like serine proteases generally contain a catalytic triad of three amino acid residues (such as histidine, aspartate, and serine) that form a charge relay that serves to make the active site serine nucleophilic. Eukaryotic trypsin-like serine proteases further include an "oxyanion hole" formed by the backbone amide hydrogen atoms of glycine and serine, which can assist in identification of the polypeptides as being trypsin-like serine proteases.

One trypsin-like serine protease has been identified in *Trichoderma* fungal cells: tsp1 (tre73897). As discussed in PCT/EP/2013/050186, tsp1 has been demonstrated to have a significant impact on expression of recombinant glycoproteins, such as immunoglobulins.

Examples of suitable tsp1 proteases include, without limitation, *Trichoderma reesei* tsp1 (SEQ ID NO: 24) and homologs thereof. Examples of homologs of tsp1 proteases identified in other organisms are described in PCT/EP/2013/050186.

### Subtilisin Proteases

Subtilisin proteases are enzymes with substrate specificity similar to that of subtilisin. Subtilisin proteases use a serine residue for hydrolysis of the peptide bonds in polypeptides and proteins. Generally, subtilisin proteases are serine proteases that contain a catalytic triad of the three amino acids aspartate, histidine, and serine. The arrangement of these catalytic residues is shared with the prototypical subtilisin from Bacillus licheniformis. Subtilisin proteases from eukaryotic organisms such as *Trichoderma* fungi include furin, MBTPS1, and TPP2. Eukaryotic trypsin-like serine proteases further include an aspartic acid residue in the oxyanion hole.

Seven subtilisin proteases have been identified in *Trichoderma* fungal cells: slp1 (tre51365); slp2 (tre123244); slp3 (tre123234); slp5 (tre64719), slp6 (tre121495), slp7 (tre123865), and slp8 (tre58698). Subtilisin protease *slp7* resembles also sedolisin protease *tpp1.*

Examples of suitable slp proteases include, without limitation, *Trichoderma reesei* slp1 (SEQ ID NO: 25), slp2 (SEQ ID NO: 26); slp3 (SEQ ID NO: 27); slp5 (SEQ ID NO: 28), slp6 (SEQ ID NO: 29), slp7 (SEQ ID NO: 30), and slp8 (SEQ ID NO: 31), and homologs thereof. Examples of homologs of slp proteases identified in other organisms are described in in PCT/EP/2013/050186.

### Glutamic Proteases

Glutamic proteases are enzymes that hydrolyse the peptide bonds in polypeptides and proteins. Glutamic proteases are insensitive to pepstatin A, and so are sometimes referred to as pepstatin insensitive acid proteases. While glutamic proteases were previously grouped with the aspartic proteases and often jointly referred to as acid proteases, it has been recently found that glutamic proteases have very different active site residues than aspartic proteases.

Two glutamic proteases have been identified in *Trichoderma* fungal cells: gap1 (tre69555) and gap2 (tre106661).

Examples of suitable gap proteases include, without limitation, *Trichoderma reesei* gap1 (SEQ ID NO: 32), *Trichoderma reesei* gap2 (SEQ ID NO: 33), and homologs thereof. Examples of homologs of gap proteases identified in other organisms are described in PCT/EP/2013/050186.

### Sedolisin Proteases and homologs of proteases

Sedolisin proteases are enzymes that use a serine residue for hydrolysis of the peptide bonds in polypeptides and proteins. Sedolisin proteases generally contain a unique catalytic triad of serine, glutamate, and aspartate. Sedolisin proteases also contain an aspartate residue in the oxyanion hole. Sedolisin proteases from eukaryotic organisms such as *Trichoderma* fungi include tripeptidyl peptidase.

Examples of suitable tpp1 proteases include, without limitation, *Trichoderma reesei* tpp1 tre82623 (SEQ ID NO: 34) and homologs thereof. Examples of homologs of tpp1 proteases identified in other organisms are described in PCT/EP/2013/050186.

As used in reference to protease, the term "homolog" refers to a protein which has protease activity and exhibit sequence similarity with a known (reference) protease sequence. Homologs may be identified by any method known in the art, preferably, by using the BLAST tool to compare a reference sequence to a single second sequence or fragment of a sequence or to a database of sequences. As described in the "Definitions" section, BLAST will compare sequences based upon percent identity and similarity.

Preferably, a homologous protease has at least 30% identity with (optionally 30%, 40%, 45%, 50%, 55%, 60%, 65%, 70%, 75%, 80%, 85%, 90%, 95%, 99% or 100% identity over a specified region, or, when not specified, over the entire sequence), when compared to one of the protease sequences listed above, including *T. reesei pep1, pep2, pep3, pep4, pep5, pep7, pep8, pep9, pep11, pep12, tsp1, slp1, slp2, slp3, slp5, slp6, slp7, slp8, tpp1, gap1* and *gap2.* Corresponding homologous proteases from *N. crassa* and *M*. *thermophila* are shown in SEQ ID NO: 136-169.

### Reducing the Activity of Proteases in the filamentous fungal cell of the disclosure

The filamentous fungal cells according to the disclosure have reduced activity of at least one endogenous protease, typically 2, 3, 4, 5 or more, in order to improve the stability and production of the protein with increased N-glycosylation site occupancy in said filamentous fungal cell, preferably in a *Trichoderma* cell.

Total protease activity can be measured according to standard methods in the art and, for example, as described herein using protease assay kit (QuantiCleave protease assay kit, Pierce #23263) with succinylated casein as substrate.

The activity of proteases found in filamentous fungal cells can be reduced by any method known to those of skill in the art. In some embodiments reduced activity of proteases is achieved by reducing the expression of the protease, for example, by promoter modification or RNAi.

In further embodiments, the reduced or eliminated expression of the proteases is the result of anti-sense polynucleotides or RNAi constructs that are specific for each of the genes encoding each of the proteases. In one embodiment, an RNAi construct is specific for a gene encoding an aspartic protease such as a pep-type protease, a trypsin-like serine proteases such as a tsp1, a glutamic protease such as a gap-type protease, a subtilisin protease such as a slp-type protease, or a sedolisin protease such as a tpp1 or a slp7 protease. In one embodiment, an RNAi construct is specific for the gene encoding a slp-type protease. In one embodiment, an RNAi construct is specific for the gene encoding slp2, slp3, slp5 or slp6. In one embodiment, an RNAi construct is specific for two or more proteases. In one embodiment, two or more proteases are any one of the pep-type proteases, any one of the trypsin-like serine proteasess, any one of the slp-type proteases, any one of the gap-type proteases and/or any one of the sedolisin proteases. In one embodiment, two or more proteases are slp2, slp3, slp5 and/or slp6. In one embodiment, RNAi construct comprises any one of the following nucleic acid sequences (see also PCT/EP/2013/050186).

| **RNAi Target sequence** |
|---|
| GCACACTTTCAAGATTGGC (SEQ ID NO: 15) |
| GTACGGTGTTGCCAAGAAG (SEQ ID NO: 16) |
| |
| |

In other embodiments, reduced activity of proteases is achieved by modifying the gene encoding the protease. Examples of such modifications include, without limitation, a mutation, such as a deletion or disruption of the gene encoding said endogenous protease activity.

Accordingly, the disclosure relates to a filamentous fungal cell, such as a *Trichoderma* cell, which has a mutation that reduces or eliminates at least one endogenous protease activity compared to a parental filamentous fungal cell which does not have such protease deficient mutation, said filamentous fungal cell further comprising a polynucleotide encoding a heterologous catalytic subunit of oligosaccharyl transferase from *Leishmania.*

Deletion or disruption mutation includes without limitation knock-out mutation, a truncation mutation, a point mutation, a missense mutation, a substitution mutation, a frameshift mutation, an insertion mutation, a duplication mutation, an amplification mutation, a translocation mutation, or an inversion mutation, and that results in a reduction in the corresponding protease activity. Methods of generating at least one mutation in a protease encoding gene of interest are well known in the art and include, without limitation, random mutagenesis and screening, site-directed mutagenesis, PCR mutagenesis, insertional mutagenesis, chemical mutagenesis, and irradiation.

In certain embodiments, a portion of the protease encoding gene is modified, such as the region encoding the catalytic domain, the coding region, or a control sequence required for expression of the coding region. Such a control sequence of the gene may be a promoter sequence or a functional part thereof, i.e., a part that is sufficient for affecting expression of the gene. For example, a promoter sequence may be inactivated resulting in no expression or a weaker promoter may be substituted for the native promoter sequence to reduce expression of the coding sequence. Other control sequences for possible modification include, without limitation, a leader sequence, a propeptide sequence, a signal sequence, a transcription terminator, and a transcriptional activator.

Protease encoding genes that are present in filamentous fungal cells may also be modified by utilizing gene deletion techniques to eliminate or reduce expression of the gene. Gene deletion techniques enable the partial or complete removal of the gene thereby eliminating their expression. In such methods, deletion of the gene may be accomplished by homologous recombination using a plasmid that has been constructed to contiguously contain the 5' and 3' regions flanking the gene.

The protease encoding genes that are present in filamentous fungal cells may also be modified by introducing, substituting, and/or removing one or more nucleotides in the gene, or a control sequence thereof required for the transcription or translation of the gene. For example, nucleotides may be inserted or removed for the introduction of a stop codon, the removal of the start codon, or a frame-shift of the open reading frame. Such a modification may be accomplished by methods known in the art, including without limitation, site-directed mutagenesis and peR generated mutagenesis (see, for example, Botstein and Shortie, 1985, Science 229: 4719; Lo et al., 1985, Proceedings of the National Academy of Sciences USA 81: 2285; Higuchi et al., 1988, Nucleic Acids Research 16: 7351; Shimada, 1996, Meth. Mol. Bioi. 57: 157; Ho et al., 1989, Gene 77: 61; Horton et al., 1989, Gene 77: 61; and Sarkar and Sommer, 1990, BioTechniques 8: 404).

Additionally, protease encoding genes that are present in filamentous fungal cells may be modified by gene disruption techniques by inserting into the gene a disruptive nucleic acid construct containing a nucleic acid fragment homologous to the gene that will create a duplication of the region of homology and incorporate construct nucleic acid between the duplicated regions. Such a gene disruption can eliminate gene expression if the inserted construct separates the promoter of the gene from the coding region or interrupts the coding sequence such that a nonfunctional gene product results. A disrupting construct may be simply a selectable marker gene accompanied by 5' and 3' regions homologous to the gene. The selectable marker enables identification of transformants containing the disrupted gene.

Protease encoding genes that are present in filamentous fungal cells may also be modified by the process of gene conversion (see, for example, Iglesias and Trautner, 1983, Molecular General Genetics 189:5 73-76). For example, in the gene conversion a nucleotide sequence corresponding to the gene is mutagenized in vitro to produce a defective nucleotide sequence, which is then transformed into a *Trichoderma* strain to produce a defective gene. By homologous recombination, the defective nucleotide sequence replaces the endogenous gene. It may be desirable that the defective nucleotide sequence also contains a marker for selection of transformants containing the defective gene.

Protease encoding genes of the present disclosure that are present in filamentous fungal cells that express a recombinant polypeptide may also be modified by established anti-sense techniques using a nucleotide sequence complementary to the nucleotide sequence of the gene (see, for example, Parish and Stoker, 1997, FEMS Microbiology Letters 154: 151-157). In particular, expression of the gene by filamentous fungal cells may be reduced or inactivated by introducing a nucleotide sequence complementary to the nucleotide sequence of the gene, which may be transcribed in the strain and is capable of hybridizing to the mRNA produced in the cells. Under conditions allowing the complementary anti-sense nucleotide sequence to hybridize to the mRNA, the amount of protein translated is thus reduced or eliminated.

Protease encoding genes that are present in filamentous fungal cells may also be modified by random or specific mutagenesis using methods well known in the art, including without limitation, chemical mutagenesis (see, for example, Hopwood, The Isolation of Mutants in Methods in Microbiology (J.R. Norris and D.W. Ribbons, eds.) pp. 363-433, Academic Press, New York, 25 1970). Modification of the gene may be performed by subjecting filamentous fungal cells to mutagenesis and screening for mutant cells in which expression of the gene has been reduced or inactivated. The mutagenesis, which may be specific or random, may be performed, for example, by use of a suitable physical or chemical mutagenizing agent, use of a suitable oligonucleotide, subjecting the DNA sequence to peR generated mutagenesis, or any combination thereof. Examples of physical and chemical mutagenizing agents include, without limitation, ultraviolet (UV) irradiation, hydroxylamine, N-methyl-N'-nitro-N-nitrosoguanidine (MNNG), N-methyl-N'-nitrosogaunidine (NTG) O-methyl hydroxylamine, nitrous acid, ethyl methane sulphonate (EMS), sodium bisulphite, formic acid, and nucleotide analogues. When such agents are used, the mutagenesis is typically performed by incubating the filamentous fungal cells, such as *Trichoderma* cells, to be mutagenized in the presence of the mutagenizing agent of choice under suitable conditions, and then selecting for mutants exhibiting reduced or no expression of the gene.

In certain embodiments, the at least one mutation or modification in a protease encoding gene of the present disclosure results in a modified protease that has no detectable protease activity. In other embodiments, the at least one modification in a protease encoding gene of the present disclosure results in a modified protease that has at least 25% less, at least 50% less, at least 75% less, at least 90%, at least 95%, or a higher percentage less protease activity compared to a corresponding non-modified protease.

The filamentous fungal cells or *Trichoderma* fungal cells of the present disclosure may have reduced or no detectable protease activity of at least three, or at least four proteases selected from the group consisting of pep1, pep2, pep3, pep4, pep5, pep8, pep9, pep11, pep12, tsp1, slp1, slp2, slp3, slp5, slp6, slp7, gap1 and gap2. In preferred embodiment, a filamentous fungal cell according to the disclosure is a filamentous fungal cell which has a deletion or disruption in at least 3 or 4 endogenous proteases, resulting in no detectable activity for such deleted or disrupted endogenous proteases and further comprising a polynucleotide encoding a heterologous catalytic subunit of oligosaccharyl transferase from *Leishmania.*

In certain embodiments, the filamentous fungal cell or *Trichoderma* cell, has reduced or no detectable protease activity in pep1, tsp1, and slp1. In other embodiments, the filamentous fungal cell or *Trichoderma* cell, has reduced or no detectable protease activity in gap1, slp1, and pep1. In certain embodiments, the filamentous fungal cell or *Trichoderma* cell, has reduced or no detectable protease activity in slp2, pep1 and gap1. In certain embodiments, the filamentous fungal cell or *Trichoderma* cell, has reduced or no detectable protease activity in slp2, pep1, gap1 and pep4. In certain embodiments, the filamentous fungal cell or *Trichoderma* cell, has reduced or no detectable protease activity in slp2, pep1, gap1, pep4 and slp1. In certain embodiments, the filamentous fungal cell or *Trichoderma* cell, has reduced or no detectable protease activity in slp2, pep1, gap1, pep4, slp1, and slp3. In certain embodiments, the filamentous fungal cell or *Trichoderma* cell, has reduced or no detectable protease activity in slp2, pep1, gap1, pep4, slp1, slp3, and pep3. In certain embodiments, the filamentous fungal cell or *Trichoderma* cell, has reduced or no detectable protease activity in slp2, pep1, gap1, pep4, slp1, slp3, pep3 and pep2. In certain embodiments, the filamentous fungal cell or *Trichoderma* cell, has reduced or no detectable protease activity in slp2, pep1, gap1, pep4, slp1, slp3, pep3, pep2 and pep5. In certain embodiments, the filamentous fungal cell or *Trichoderma* cell, has reduced or no detectable protease activity in slp2, pep1, gap1, pep4, slp1, slp3, pep3, pep2, pep5 and tsp1. In certain embodiments, the filamentous fungal cell or *Trichoderma* cell, has reduced or no detectable protease activity in slp2, pep1, gap1, pep4, slp1, slp3, pep3, pep2, pep5, tsp1 and slp7. In certain embodiments, the filamentous fungal cell or *Trichoderma* cell, has reduced or no detectable protease activity in slp2, pep1, gap1, pep4, slp1, slp3, pep3, pep2, pep5, tsp1, slp7 and slp8. In certain embodiments, the filamentous fungal cell or *Trichoderma* cell, has reduced or no detectable protease activity in slp2, pep1, gap1, pep4, slp1, slp3, pep3, pep2, pep5, tsp1, slp7, slp8 and gap2. In certain embodiments, the filamentous fungal cell or *Trichoderma* cell, has reduced or no detectable protease activity in at least three endogenous proteases selected from the group consisting of pep1, pep2, pep3, pep4, pep5, pep8, pep9, pep11, pep12, tsp1, slp2, slp3, slp7, gap1 and gap2. In certain embodiments, the filamentous fungal cell or *Trichoderma* cell, has reduced or no detectable protease activity in at least three to six endogenous proteases selected from the group consisting of pep1, pep2, pep3, pep4, pep5, tsp1, slp1, slp2, slp3, gap1 and gap2. In certain embodiments, the filamentous fungal cell or *Trichoderma* cell, has reduced or no detectable protease activity in at least seven to ten endogenous proteases selected from the group consisting of pep1, pep2, pep3, pep4, pep5, pep7, pep8, tsp1, slp1, slp2, slp3, slp5, slp6, slp7, slp8, tpp1, gap1 and gap2.

### Expression of heterologous catalytic subunits of oligosaccharyl transferase in filamentous fungal cells

As used herein, the expression "oligosaccharyl transferase" or OST refers to the enzymatic complex that transfers a 14-sugar oligosaccharide from dolichol to nascent protein. It is a type of glycosyltransferase. The sugar Glc3Man9GlcNAc2 is attached to an asparagine (Asn) residue in the sequence Asn-X-Ser or Asn-X-Thr where X is any amino acid except proline. This sequence is called a glycosylation sequon. The reaction catalyzed by OST is the central step in the N-linked glycosylation pathway.

In most eukaryotes, OST is a hetero-oligomeric complex composed of eight different proteins, in which the STT3 component is believed to be the catalytic subunit.

According to the present disclosure, the heterologous catalytic subunit of oligosaccharyl transferase is selected from *Leishmania* oligosaccharyl transferase catalytic subunits. There are four STT3 paralogues in the parasitic protozoa *Leishmania,* named STT3A, STT3B, STT3C and STT3D.

In one embodiment, the heterologous catalytic subunit of oligosaccharyl transferase is STT3D from *Leishmania major* (having the amino acid sequence as set forth in SEQ ID No:1).

In another embodiment, the heterologous catalytic subunit of oligosaccharyl transferase is STT3D from *Leishmania infantum* (having the amino acid sequence as set forth in SEQ ID No:8).

In another embodiment, the heterologous catalytic subunit of oligosaccharyl transferase is STT3D from *Leishmania braziliensis* (having the amino acid sequence as set forth in SEQ ID No:89).

In another embodiment, the heterologous catalytic subunit of oligosaccharyl transferase is STT3D from *Leishmania mexicana* (having the amino acid sequence as set forth in SEQ ID No:91).

In yet another embodiment, the heterologous catalytic subunit of oligosaccharyl transferase is a functional variant polypeptide having at least 50%, preferably at least 60%, even more preferably at least 70%, 80%, 90%, 95% identity with SEQ ID NO:1, SEQ ID NO:8, SEQ ID NO: 89 or SEQ ID NO: 91.

In yet another embodiment, the heterologous catalytic subunit of oligosaccharyl transferase is a functional variant polypeptide having at least 50%, preferably at least 60%, even more preferably at least 70%, 80%, 90%, 95% identity with SEQ ID NO:1 or SEQ ID NO:8.

In one embodiment of the disclosure, the polynucleotide encoding heterologous catalytic subunit of oligosaccharyl transferase comprises SEQ ID NO:2.

SEQ ID NO:2 is a codon-optimized version of the STT3D gene from L major (gi389594572|XM_003722461.1).

In one embodiment of the disclosure, the polynucleotide encoding heterologous catalytic subunit of oligosaccharyl transferase comprises SEQ ID NO:9.

SEQ ID NO:9 is a codon-optimized version of the STT3D gene from *L major* (gi339899220|XM_003392747.1|).

In one embodiment of the disclosure, the polynucleotide encoding heterologous catalytic subunit of oligosaccharyl transferase comprises SEQ ID NO:88 or a variant or SEQ ID NO: 88 which has been codon-optimized for expression in filamentous fungal cells such as Trichoderma reesei.

In one embodiment of the disclosure, the polynucleotide encoding heterologous catalytic subunit of oligosaccharyl transferase comprises SEQ ID NO:90 or a variant or SEQ ID NO: 90 which has been codon-optimized for expression in filamentous fungal cells such as Trichoderma reesei.

In one embodiment of the disclosure, the polynucleotide encoding a heterologous catalytic subunit of oligosaccharyl transferase comprises a polynucleotide encoding a functional variant polypeptide of STT3D from *Leishmania major, Leishmania infantum, Leishmania braziliens or Leishmania mexicana* having at least 50%, preferably at least 60%, even more preferably at least 70%, 80%, 90%, 95% identity with SEQ ID NO:1, SEQ ID NO:8, SEQ ID NO: 89 or SEQ ID NO: 91.

In one embodiment of the disclosure, the polynucleotide encoding a heterologous catalytic subunit of oligosaccharyl transferase comprises a polynucleotide encoding a functional variant polypeptide of STT3D from *Leishmania major* or *Leishmania infantum* having at least 50%, preferably at least 60%, even more preferably at least 70%, 80%, 90%, 95% identity with SEQ ID NO:1 or SEQ ID NO:8.

In one embodiment of the disclosure, the polynucleotide encoding a heterologous catalytic subunit of oligosaccharyl transferase is under the control of a promoter for the constitutive expression of said oligosaccharyl transferase is said filamentous fungal cell.

Promoters that may be used for expression of the oligosaccharyl transferase include constitutive promoters such as gpd or cDNA1, promoters of endogenous glycosylation enzymes and glycosyltransferases such as mannosyltransferases that synthesize N-glycans in the Golgi or ER, and inducible promoters of high-yield endogenous proteins such as the cbh1 promoter.

In one embodiment of the disclosure, said promoter is the cDNA1 promoter from *Trichoderma reesei.*

### Increasing N-glycosylation site occupancy in filamentous fungal cell of the disclosure

The filamentous fungal cells according to the disclosure have increased oligosaccharide transferase activity, in order to increase N-glycosylation site occupancy.

The N-glycosylation site occupancy can be measured by standard methods in the art (for example, Schulz and Aebi (2009) Analysis of Glycosylation Site Occupancy Reveals a Role for Ost3p and Ost6p in Site-specific N-Glycosylation Efficiency, Molecular & Cellular Proteomics, 8:357-364, or Millward et al. (2008), Effect of constant and variable domain glycosylation on pharmacokinetics of therapeutic antibodies in mice, Biologicals, 36:41-47, Forno et al. (2004) N- and O-linked carbohydrates and glycosylation site occupancy in recombinant human granulocyte-macrophage colony-stimulating factor secreted by a Chinese hamster ovary cell line, Eur. J. Biochem. 271: 907-919) or methods as described herein in the Examples.

The N-glycosylation site occupancy refers to the molar percentage (or mol%) of the heterologous glycoproteins that are N-glycosylated with respect to the total number of heterologous glycoprotein produced by the filamentous fungal cell (as described in Example 1 below).

In one embodiment of the disclosure, the N-glycosylation site occupancy is at least 95%, and Man3, Man5, G0, G1 and/or G2 glycoforms represent at least 50% of total neutral N-glycans of the heterologous glycoprotein.

The percentage of various glycoforms with respect to the total neutral N-glycans of the heterologous glycoprotein can be measured for example as described in WO2012069593.

In an embodiment, the heterologous protein with increased N-glycosylation site occupancy is selected from the group consisting of:
a) an immunoglobulin, such as IgG,
b) a light chain or heavy chain of an immunoglobulin,
c) a heavy chain or a light chain of an antibody,
d) a single chain antibody,
e) a camelid antibody,
f) a monomeric or multimeric single domain antibody,
g) a FAb-fragment, a FAb2-fragment, and,
h) their antigen-binding fragments.

### Methods for producing glycoproteins with increased N-glycosylation site occupancy and mammalian-like N-glycans

The filamentous fungal cells according to the present disclosure may be useful in particular for producing heterologous glycoprotein composition, such as antibody composition, with increased N-glycosylation site occupancy and mammalian-like N-glycans, such as complex N-glycans. Accordingly, in one aspect, the filamentous fungal cell is further genetically modified to produce a mammalian-like N-glycan, thereby enabling *in vivo* production of glycoprotein or antibody composition with increased N-glycosylation site occupancy and with mammalian-like N-glycan as major glycoforms of said glycoprotein or antibody.

In certain embodiments, this aspect includes methods of producing glycoproteins or antibodies with mammalian-like N-glycans in a *Trichoderma* cell.

In certain embodiment, the glycoprotein or antibody comprises, as a major glycoform, the mammalian-like N-glycan having the formula [{Galβ4}ₓGlcNAcβ2]_{z}Manα3([{Galβ4}_{y}GlcNAcβ2]_{w}Manα6)Manβ4GlcNAcβ[Fucα6]ₐGlcNAc, where () defines a branch in the structure, where [] or {} define a part of the glycan structure either present or absent in a linear sequence, and where a, x, y, z and w are 0 or 1, independently. In an embodiment w and z are 1, and x and y are 0 for a non-galactosylated G0 structure; both x and y are 1 for a G2 structure; and only either one of x or y is 1 for a G1 structure. When a is 1, the structure is core fucosylated such as a FG0, FG1 or FG2 glycan.

In certain embodiments, the glycoprotein or antibody comprises, as a major glycoform, mammalian-like N-glycan selected from the group consisting of:
i.Manα3[Manα6(Manα3)Manα6]Manβ4GlcNAβ4GlcNAc (Man5 glycoform);
ii.GlcNAcβ2Manα3[Manα6(Manα3)Manα6]Manβ4GlcNAβ4GlcNAc (GlcNAcMan5 glycoform);
iii.Manα6(Manα3)Manβ4GlcNAβ4GlcNAc (Man3 glycoform);
iv.Manα6(GlcNAcβ2Manα3)Manβ4GlcNAβ4GlcNAc (GlcNAcMan3) or,
v.complex type N-glycans selected from the G0, G1, or G2 glycoform.

In an embodiment, the glycoprotein or antibody composition with mammalian-like N-glycans, preferably produced by an alg3 knock-out strain, include glycoforms that essentially lack or are devoid of glycans Manα3[Manα6(Manα3)Manα6]Manβ4GlcNAβ4GlcNAc (Man5). In specific embodiments, the filamentous fungal cell produces heterologous glycoproteins or antibodies with, as major glycoform, the trimannosyl N-glycan structure Manα3[Manα6]Manβ4GlcNAcβ4GlcNAc. In other embodiments, the filamentous fungal cell produces glycoproteins or antibodies with, as major glycoform, the GO N-glycan structure GlcNAcβ2Manα3[GlcNAcβ2Manα6]Manβ4GlcNAcβ4GlcNAc.

In certain embodiments, the filamentous fungal cell of the disclosure produces glycoprotein or antibody composition with a mixture of different N-glycans.

In some embodiments, Man3GlcNAc2 N-glycan (i.e. Manα3[Manα6]Manβ4GlcNAcβ4GlcNAc) represents at least 10%, at least 20%, at least at least 30%, at least 40%, at least 50%, at least 60%, at least 70%, at least 80%, at least 90% or more of total (mol%) neutral N-glycans of the heterologous glycoprotein or antibody, as expressed in a filamentous fungal cells of the disclosure.

In other embodiments, GlcNAc2Man3 N-glycan (for example G0 GlcNAcβ2Manα3[GlcNAcβ2Manα6]Manβ4GlcNAcβ4GlcNAc) represents at least 10%, at least 20%, at least at least 30%, at least 40%, at least 50%, at least 60%, at least 70%, at least 80%, at least 90% or more of total (mol%) neutral N-glycans of the heterologous glycoprotein or antibody, as expressed in a filamentous fungal cells of the disclosure.

In other embodiments, GalGlcNAc2Man3GlcNAc2 N-glycan (for example G1 N-glycan) represents at least 10%, at least 20%, at least at least 30%, at least 40%, at least 50%, at least 60%, at least 70%, at least 80%, at least 90% or more of total (mol%) neutral N-glycans of the heterologous glycoprotein or antibody, as expressed in a filamentous fungal cells of the disclosure.

In other embodiments, Gal2GlcNAc2Man3GlcNAc2 N-glycan (for example G2 N-glycan) represents at least 10%, at least 20%, at least at least 30%, at least 40%, at least 50%, at least 60%, at least 70%, at least 80%, at least 90% or more of total (mol%) neutral N-glycans of the heterologous glycoprotein or antibody, as expressed in a filamentous fungal cells of the disclosure.

In other embodiments, complex type N-glycan represents at least 10%, at least 20%, at least at least 30%, at least 40%, at least 50%, at least 60%, at least 70%, at least 80%, at least 90% or more of total (mol%) neutral N-glycans of a heterologous glycoprotein or antibody, as expressed in a filamentous fungal cells of the disclosure.

In other embodiments, hybrid type N-glycan represents at least 10%, at least 20%, at least at least 30%, at least 40%, at least 50%, at least 60%, at least 70%, at least 80%, at least 90% or more of total (mol%) neutral N-glycans of a heterologous glycoprotein or antibody, as expressed in a filamentous fungal cells of the disclosure.

In other embodiments, less than 0.5%, 0.1%, 0.05%, or less than 0.01% of the N-glycan of the heterologous glycoprotein composition or antibody composition produced by the host cell of the disclosure, comprises galactose. In certain embodiments, none of N-glycans comprise galactose.

The Neu5Gc and Gala- (non-reducing end terminal Galα3Galβ4GlcNAc) structures are known xenoantigenic (animal derived) modifications of antibodies which are produced in animal cells such as CHO cells. The structures may be antigenic and, thus, harmful even at low concentrations. The filamentous fungi of the present disclosure lack biosynthetic pathways to produce the terminal Neu5Gc and Gala- structures. In an embodiment that may be combined with the preceding embodiments less than 0.1%, 0.01 %, 0.001 % or 0 % of the N-glycans and/or O-glycans of the glycoprotein or antibody composition comprises Neu5Gc and/or Gala- structure. In an embodiment that may be combined with the preceding embodiments, less than 0.1%, 0.01 %, 0.001 % or 0 % of the N-glycans and/or O-glycans of the heterologous glycoprotein or antibody composition comprises Neu5Gc and/or Gala- structure.

The filamentous fungal cells of the present disclosure lack genes to produce fucosylated heterologous proteins. In an embodiment that may be combined with the preceding embodiments, less than 0.1%, 0.01 %, 0.001 %, or 0 % of the N-glycan of the glycoprotein or antibody composition comprises core fucose structures.

The terminal Galβ4GlcNAc structure of N-glycan of mammalian cell produced glycans affects bioactivity of antibodies and Galβ3GlcNAc may be xenoantigen structure from plant cell produced proteins. In an embodiment that may be combined with one or more of the preceding embodiments, less than 0.1%, 0.01 %, 0.001 %, or 0 % of N-glycan of the heterologous glycoprotein or antibody composition comprises terminal galactose epitopes Galβ3/4GlcNAc.

Glycation is a common post-translational modification of proteins, resulting from the chemical reaction between reducing sugars such as glucose and the primary amino groups on protein. Glycation occurs typically in neutral or slightly alkaline pH in cell cultures conditions, for example, when producing antibodies in CHO cells and analysing them (see, for example, Zhang et al. (2008) Unveiling a glycation hot spot in a recombinant humanized monoclonal antibody. Anal Chem. 80(7):2379-2390). As filamentous fungi of the present disclosure are typically cultured in acidic pH, occurrence of glycation is reduced. In an embodiment that may be combined with the preceding embodiments, less than 1.0%, 0.5%, 0.1%, 0.01 %, 0.001 %, or 0 % of the heterologous glycoprotein or antibody composition comprises glycation structures.

In one embodiment, the glycoprotein composition, such as an antibody is devoid of one, two, three, four, five, or six of the structures selected from the group of Neu5Gc, terminal Galα3Galβ4GlcNAc, terminal Galβ4GlcNAc, terminal Galβ3GlcNAc, core linked fucose and glycation structures.

In certain embodiments, such glycoprotein protein with mammalian-like N-glycan, as produced in the filamentous fungal cell of the disclosure, is a therapeutic protein. Therapeutic proteins may include immunoglobulin, or a protein fusion comprising a Fc fragment or other therapeutic glycoproteins, such as antibodies, erythropoietins, interferons, growth hormones, albumins or serum albumin, enzymes, or blood-clotting factors and may be useful in the treatment of humans or animals. For example, the glycoproteins with mammalian-like N-glycan as produced by the filamentous fungal cell according to the disclosure may be a therapeutic glycoprotein such as rituximab.

Methods for producing glycoproteins with mammalian-like N-glycans in filamentous fungal cells are also described for example in WO2012/069593.

In one aspect, the filamentous fungal cell according to the disclosure as described above, is further genetically modified to mimick the traditional pathway of mammalian cells, starting from Man5 N-glycans as acceptor substrate for GnTI, and followed sequentially by GnT1, mannosidase II and GnTII reaction steps (hereafter referred as the "traditional pathway" for producing GO glycoforms). In one variant, a single recombinant enzyme comprising the catalytic domains of GnTI and GnTII, is used.

Alternatively, in a second aspect, the filamentous fungal cell according to the disclosure as described above is further genetically modified to have alg3 reduced expression, allowing the production of core Man₃GlcNAc₂ N-glycans, as acceptor substrate for GnTI and GnTII subsequent reactions and bypassing the need for mannosidase α1,2 or mannosidase II enzymes (the reduced "alg3" pathway). In one variant, a single recombinant enzyme comprising the catalytic domains of GnTI and GnTII, is used.

In such embodiments for mimicking the traditional pathway for producing glycoproteins with mammalian-like N-glycans, a Man₅ expressing filamentous fungal cell, such as *T. reesei* strain, may be transformed with a GnTI or a GnTII/GnTI fusion enzyme using random integration or by targeted integration to a known site known not to affect Man5 glycosylation. Strains that synthesise GlcNAcMan5 N-glycan for production of proteins having hybrid type glycan(s) are selected. The selected strains are further transformed with a catalytic domain of a mannosidase II-type mannosidase capable of cleaving Man5 structures to generate GlcNAcMan3 for production of proteins having the corresponding GlcNAcMan3 glycoform or their derivative(s). In certain embodiments, mannosidase II-type enzymes belong to glycoside hydrolase family 38 (cazy.org/GH38_all.html). Characterized enzymes include enzymes listed in cazy.org/GH38_characterized.html. Especially useful enzymes are Golgi-type enzymes that cleaving glycoproteins, such as those of subfamily α-mannosidase II (Man2A1;ManA2). Examples of such enzymes include human enzyme AAC50302, D. melanogaster enzyme (Van den Elsen J.M. et al (2001) EMBO J. 20: 3008-3017), those with the 3D structure according to PDB-reference 1HTY, and others referenced with the catalytic domain in PDB. For cytoplasmic expression, the catalytic domain of the mannosidase is typically fused with an N-terminal targeting peptide (for example as disclosed in the above Section) or expressed with endogenous animal or plant Golgi targeting structures of animal or plant mannosidase II enzymes. After transformation with the catalytic domain of a mannosidase II-type mannosidase, strains are selected that produce GlcNAcMan3 (if GnTI is expressed) or strains are selected that effectively produce GlcNAc2Man3 (if a fusion of GnTI and GnTII is expressed). For strains producing GlcNAcMan3, such strains are further transformed with a polynucleotide encoding a catalytic domain of GnTII and transformant strains that are capable of producing GlcNAc2Man3GlcNAc2 are selected.

In such embodiment for mimicking the traditional pathway, the filamentous fungal cell is a filamentous fungal cell as defined in previous sections, and further comprising one or more polynucleotides encoding a polypeptide selected from the group consisting of:
i) α1,2 mannosidase,
ii) N-acetylglucosaminyltransferase I catalytic domain,
iii) α mannosidase II,
iv) N-acetylglucosaminyltransferase II catalytic domain,
v) β1,4 galactosyltransferase, and,
vi) fucosyltransferase.

In embodiments using the reduced *alg3* pathway, the filamentous fungal cell, such as a *Trichoderma* cell, has a reduced level of activity of a dolichyl-P-Man:Man(5)GlcNAc(2)-PP-dolichyl mannosyltransferase compared to the level of activity in a parent host cell. Dolichyl-P-Man:Man(5)GlcNAc(2)-PP-dolichyl mannosyltransferase (EC 2.4.1.130) transfers an alpha-D-mannosyl residue from dolichyl-phosphate D-mannose into a membrane lipid-linked oligosaccharide. Typically, the dolichyl-P-Man:Man(5)GlcNAc(2)-PP-dolichyl mannosyltransferase enzyme is encoded by an *alg3* gene. In certain embodiments, the filamentous fungal cell for producing glycoproteins with mammalian-like N-glycans has a reduced level of expression of an *alg3* gene compared to the level of expression in a parent strain.

More preferably, the filamentous fungal cell comprises a mutation of *alg3.* The ALG3 gene may be mutated by any means known in the art, such as point mutations or deletion of the entire alg3 gene. For example, the function of the alg3 protein is reduced or eliminated by the mutation of *alg3.* In certain embodiments, the *alg3* gene is disrupted or deleted from the filamentous fungal cell, such as *Trichoderma* cell. In certain embodiments, the filamentous fungal cell is a *T. reesei* cell. SEQ ID NOs: 36 and 37 provide, the nucleic acid and amino acid sequences of the *alg3* gene in *T. reesei,* respectively. In an embodiment the filamentous fungal cell is used for the production of a glycoprotein, wherein the glycan(s) comprise or consist of Manα3[Manα6]Manβ4GlcNAcβ4GlcNAc, and/or a non-reducing end elongated variant thereof.

In certain embodiments, the filamentous fungal cell has a reduced level of activity of an alpha-1,6-mannosyltransferase compared to the level of activity in a parent strain. Alpha-1,6-mannosyltransferase (EC 2.4.1.232) transfers an alpha-D-mannosyl residue from GDP-mannose into a protein-linked oligosaccharide, forming an elongation initiating alpha-(1->6)-D-mannosyl-D-mannose linkage in the Golgi apparatus. Typically, the alpha-1,6-mannosyltransferase enzyme is encoded by an *och1* gene. In certain embodiments, the filamentous fungal cell has a reduced level of expression of an *och1* gene compared to the level of expression in a parent filamentous fungal cell. In certain embodiments, the *och1* gene is deleted from the filamentous fungal cell.

The filamentous fungal cells used in the methods of producing glycoprotein with mammalian-like N-glycans may further contain a polynucleotide encoding an N-acetylglucosaminyltransferase I catalytic domain (GnTI) that catalyzes the transfer of N-acetylglucosamine to a terminal Manα3 and a polynucleotide encoding an N-acetylglucosaminyltransferase II catalytic domain (GnTII), that catalyses N-acetylglucosamine to a terminal Manα6 residue of an acceptor glycan to produce a complex N-glycan. In one embodiment, said polynucleotides encoding GnTI and GnTII are linked so as to produce a single protein fusion comprising both catalytic domains of GnTI and GnTII.

As disclosed herein, N-acetylglucosaminyltransferase I (GlcNAc-TI; GnTI; EC 2.4.1.101) catalyzes the reaction UDP-N-acetyl-D-glucosamine + 3-(alpha-D-mannosyl)-beta-D-mannosyl-R <=> UDP + 3-(2-(N-acetyl-beta-D-glucosaminyl)-alpha-D-mannosyl)-beta-D-mannosyl-R, where R represents the remainder of the N-linked oligosaccharide in the glycan acceptor. An N-acetylglucosaminyltransferase I catalytic domain is any portion of an N-acetylglucosaminyltransferase I enzyme that is capable of catalyzing this reaction. GnTI enzymes are listed in the CAZy database in the glycosyltransferase family 13 (cazy.org/GT13_all). Enzymatically characterized species includes *A. thaliana* AAR78757.1 (US6 653 459), *C*. *elegans* AAD03023.1 (Chen S. et al J. Biol.Chem 1999;274(1):288-97), *D*. *melanogaster* AAF57454.1 (Sarkar & Schachter Biol Chem. 2001 Feb;382(2):209-17); *C*. *griseus* AAC52872.1 (Puthalakath H. et al J. Biol.Chem 1996 271(44):27818-22); *H. sapiens* AAA52563.1 (Kumar R. et al Proc Natl Acad Sci U S A. 1990 Dec;87(24):9948-52); *M. auratus* AAD04130.1 (Opat As et al Biochem J. 1998 Dec 15;336 (Pt 3):593-8), (including an example of deactivating mutant), Rabbit, *O. cuniculus* AAA31493.1 (Sarkar M et al. Proc Natl Acad Sci U S A. 1991 Jan 1;88(1):234-8). Amino acid sequences for N-acetylglucosaminyltransferase I enzymes from various organisms are described for example in PCT/EP2011/070956. Additional examples of characterized active enzymes can be found at cazy.org/GT13_characterized. The 3D structure of the catalytic domain of rabbit GnTI was defined by X-ray crystallography in Unligil UM et al. EMBO J. 2000 Oct 16;19(20):5269-80. The Protein Data Bank (PDB) structures for GnTI are 1FO8, 1FO9, 1FOA, 2AM3, 2AM4, 2AM5, and 2APC. In certain embodiments, the N-acetylglucosaminyltransferase I catalytic domain is from the human N-acetylglucosaminyltransferase I enzyme (SEQ ID NO: 38) or variants thereof. In certain embodiments, the N-acetylglucosaminyltransferase I catalytic domain contains a sequence that is at least 70%, at least 75%, at least 80%, at least 85%, at least 90%, at least 95%, at least 96%, at least 97%, at least 98%, at least 99%, or 100% identical to amino acid residues 84-445 of SEQ ID NO: 38. In some embodiments, a shorter sequence can be used as a catalytic domain (e.g. amino acid residues 105-445 of the human enzyme or amino acid residues 107-447 of the rabbit enzyme; Sarkar et al. (1998) Glycoconjugate J 15:193-197). Additional sequences that can be used as the GnTI catalytic domain include amino acid residues from about amino acid 30 to 445 of the human enzyme or any C-terminal stem domain starting between amino acid residue 30 to 105 and continuing to about amino acid 445 of the human enzyme, or corresponding homologous sequence of another GnTI or a catalytically active variant or mutant thereof. The catalytic domain may include N-terminal parts of the enzyme such as all or part of the stem domain, the transmembrane domain, or the cytoplasmic domain.

As disclosed herein, N-acetylglucosaminyltransferase II (GlcNAc-TII; GnTII; EC 2.4.1.143) catalyzes the reaction UDP-N-acetyl-D-glucosamine + 6-(alpha-D-mannosyl)-beta-D-mannosyl-R <=> UDP + 6-(2-(N-acetyl-beta-D-glucosaminyl)-alpha-D-mannosyl)-beta-D-mannosyl-R, where R represents the remainder of the N-linked oligosaccharide in the glycan acceptor. An N-acetylglucosaminyltransferase II catalytic domain is any portion of an N-acetylglucosaminyltransferase II enzyme that is capable of catalyzing this reaction. Amino acid sequences for N-acetylglucosaminyltransferase II enzymes from various organisms are listed in WO2012069593. In certain embodiments, the N-acetylglucosaminyltransferase II catalytic domain is from the human N-acetylglucosaminyltransferase II enzyme (SEQ ID NO: 39) or variants thereof. Additional GnTII species are listed in the CAZy database in the glycosyltransferase family 16 (cazy.org/GT16_all). Enzymatically characterized species include GnTII of *C*. *elegans, D. melanogaster, Homo sapiens* (NP_002399.1), *Rattus norvegicus, Sus scrofa* (cazy.org/GT16_characterized). In certain embodiments, the N-acetylglucosaminyltransferase II catalytic domain contains a sequence that is at least 70%, at least 75%, at least 80%, at least 85%, at least 90%, at least 95%, at least 96%, at least 97%, at least 98%, at least 99%, or 100% identical to amino acid residues from about 30 to about 447 of SEQ ID NO: 39. The catalytic domain may include N-terminal parts of the enzyme such as all or part of the stem domain, the transmembrane domain, or the cytoplasmic domain.

In embodiments where the filamentous fungal cell contains a fusion protein of the disclosure, the fusion protein may further contain a spacer in between the N-acetylglucosaminyltransferase I catalytic domain and the N-acetylglucosaminyltransferase II catalytic domain. In certain embodiments, the spacer is an EGIV spacer, a 2xG4S spacer, a 3xG4S spacer, or a CBHI spacer. In other embodiments, the spacer contains a sequence from a stem domain.

For ER/Golgi expression the N-acetylglucosaminyltransferase I and/or N-acetylglucosaminyltransferase II catalytic domain is typically fused with a targeting peptide or a part of an ER or early Golgi protein, or expressed with an endogenous ER targeting structures of an animal or plant N-acetylglucosaminyltransferase enzyme. In certain preferred embodiments, the N-acetylglucosaminyltransferase I and/or N-acetylglucosaminyltransferase II catalytic domain contains any of the targeting peptides of the disclosure as described in the section entitled "Targeting sequences". Preferably, the targeting peptide is linked to the N-terminal end of the catalytic domain. In some embodiments, the targeting peptide contains any of the stem domains of the disclosure as described in the section entitled "Targeting sequences". In certain preferred embodiments, the targeting peptide is a Kre2/Mnt1 targeting peptide. In other embodiments, the targeting peptide further contains a transmembrane domain linked to the N-terminal end of the stem domain or a cytoplasmic domain linked to the N-terminal end of the stem domain. In embodiments where the targeting peptide further contains a transmembrane domain, the targeting peptide may further contain a cytoplasmic domain linked to the N-terminal end of the transmembrane domain.

The filamentous fungal cells may also contain a polynucleotide encoding a UDP-GlcNAc transporter. The polynucleotide encoding the UDP-GlcNAc transporter may be endogenous (i.e., naturally present) in the host cell, or it may be heterologous to the filamentous fungal cell.

In certain embodiments, the filamentous fungal cell may further contain a polynucleotide encoding a α-1,2-mannosidase. The polynucleotide encoding the α-1,2-mannosidase may be endogenous in the host cell, or it may be heterologous to the host cell. Heterologous polynucleotides are especially useful for a host cell expressing high-mannose glycans transferred from the Golgi to the ER without effective exo-α-2-mannosidase cleavage. The α-1,2-mannosidase may be a mannosidase I type enzyme belonging to the glycoside hydrolase family 47 (cazy.org/GH47_all.html). In certain embodiments the α-1,2-mannosidase is an enzyme listed at cazy.org/GH47_characterized.html. In particular, the α-1,2-mannosidase may be an ER-type enzyme that cleaves glycoproteins such as enzymes in the subfamily of ER α-mannosidase I EC 3.2.1.113 enzymes. Examples of such enzymes include human α-2-mannosidase 1B (AAC26169), a combination of mammalian ER mannosidases, or a filamentous fungal enzyme such as α-1,2-mannosidase (MDS1) (*T. reesei* AAF34579; Maras M et al J Biotech. 77, 2000, 255, or Trire 45717). For ER expression, the catalytic domain of the mannosidase is typically fused with a targeting peptide, such as HDEL, KDEL, or part of an ER or early Golgi protein, or expressed with an endogenous ER targeting structures of an animal or plant mannosidase I enzyme.

In certain embodiments, the filamentous fungal cell may also further contain a polynucleotide encoding a galactosyltransferase. Galactosyltransferases transfer β-linked galactosyl residues to terminal N-acetylglucosaminyl residue. In certain embodiments the galactosyltransferase is a β-1,4-galactosyltransferase. Generally, β-1,4-galactosyltransferases belong to the CAZy glycosyltransferase family 7 (cazy.org/GT7_all.html) and include β-N-acetylglucosaminyl-glycopeptide β-1,4-galactosyltransferase (EC 2.4.1.38), which is also known as N-acetylactosamine synthase (EC 2.4.1.90). Useful subfamilies include β4-GalT1, β4-GalT-II, -III, -IV, -V, and -VI, such as mammalian or human β4-GalTI or β4GalT-II, -III, -IV, -V, and -VI or any combinations thereof. β4-GalT1, β4-GalTII, or β4-GalTIII are especially useful for galactosylation of terminal GlcNAcβ2-structures on N-glycans such as GlcNAcMan3, GlcNAc2Man3, or GlcNAcMan5 (Guo S. et al. Glycobiology 2001, 11:813-20). The three-dimensional structure of the catalytic region is known (e.g. (2006) J.Mol.Biol. 357: 1619-1633), and the structure has been represented in the PDB database with code 2FYD. The CAZy database includes examples of certain enzymes. Characterized enzymes are also listed in the CAZy database at cazy.org/GT7_characterized.html. Examples of useful β4GalT enzymes include β4GalT1, e.g. bovine Bos taurus enzyme AAA30534.1 (Shaper N.L. et al Proc. Natl. Acad. Sci. U.S.A. 83 (6), 1573-1577 (1986)), human enzyme (Guo S. et al. Glycobiology 2001, 11:813-20), and *Mus musculus* enzyme AAA37297 (Shaper, N.L. et al. 1998 J. Biol. Chem. 263 (21), 10420-10428); β4GalTII enzymes such as human β4GalTII BAA75819.1, Chinese hamster *Cricetulus griseus* AAM77195, *Mus musculus* enzyme BAA34385, and Japanese Medaka fish *Oryzias latipes* BAH36754; and β4GalTIII enzymes such as human β4GalTIII BAA75820.1, Chinese hamster *Cricetulus griseus* AAM77196 and *Mus musculus* enzyme AAF22221.

The galactosyltransferase may be expressed in the plasma membrane of the host cell. A heterologous targeting peptide, such as a Kre2 peptide described in Schwientek J.Biol. Chem 1996 3398, may be used. Promoters that may be used for expression of the galactosyltransferase include constitutive promoters such as gpd, promoters of endogenous glycosylation enzymes and glycosyltransferases such as mannosyltransferases that synthesize N-glycans in the Golgi or ER, and inducible promoters of high-yield endogenous proteins such as the cbh1 promoter.

In certain embodiments of the disclosure where the filamentous fungal cell contains a polynucleotide encoding a galactosyltransferase, the filamentous fungal cell also contains a polynucleotide encoding a UDP-Gal 4 epimerase and/or UDP-Gal transporter. In certain embodiments of the disclosure where the filamentous fungal cell contains a polynucleotide encoding a galactosyltransferase, lactose may be used as the carbon source instead of glucose when culturing the host cell. The culture medium may be between pH 4.5 and 7.0 or between 5.0 and 6.5. In certain embodiments of the disclosure where the filamentous fungal cell contains a polynucleotide encoding a galactosyltransferase and a polynucleotide encoding a UDP-Gal 4 epimerase and/or UDP-Gal transporter, a divalent cation such as Mn2+, Ca2+ or Mg2+ may be added to the cell culture medium.

Accordingly, in certain embodiments, the filamentous fungal cell of the disclosure, for example, selected among *Neurospora, Trichoderma, Myceliophthora, Aspergillus, Fusarium* or *Chrysosporium* cell, and more preferably *Trichoderma reesei* cell, may comprise the following features:
a) a mutation in at least one endogenous protease that reduces or eliminates the activity of said endogenous protease, preferably the protease activity of two or three or more endogenous proteases is reduced, for example, pep1, tsp1, gap1 and/or slp1 proteases, in order to improve production or stability of a heterologous glycoprotein to be produced,
b) a polynucleotide encoding a heterologous catalytic subunit of oligosaccharyl transferase, preferably of SEQ ID NO:2 or NO:9,
c) a polynucleotide encoding a glycoprotein having at least one asparagine, preferably a heterologous glycoprotein, such as an immunoglobulin, an antibody, or a protein fusion comprising Fc fragment of an immunoglobulin.
d) optionally, a deletion or disruption of the alg3 gene,
e) optionally, a polynucleotide encoding N-acetylglucosaminyltransferase I catalytic domain and a polynucleotide encoding N-acetylglucosaminyltransferase II catalytic domain,
f) optionally, a polynucleotide encoding β1,4 galactosyltransferase,
g) optionally, a polynucleotide or polynucleotides encoding UDP-Gal 4 epimerase and/or transporter.

### Targeting Sequences

In certain embodiments, recombinant enzymes, such as α1,2 mannosidases, GnTI, or other glycosyltransferases introduced into the filamentous fungal cells, include a targeting peptide linked to the catalytic domains. The term "linked" as used herein means that two polymers of amino acid residues in the case of a polypeptide or two polymers of nucleotides in the case of a polynucleotide are either coupled directly adjacent to each other or are within the same polypeptide or polynucleotide but are separated by intervening amino acid residues or nucleotides. A "targeting peptide", as used herein, refers to any number of consecutive amino acid residues of the recombinant protein that are capable of localizing the recombinant protein to the endoplasmic reticulum (ER) or Golgi apparatus (Golgi) within the host cell. The targeting peptide may be N-terminal or C-terminal to the catalytic domains. In certain embodiments, the targeting peptide is N-terminal to the catalytic domains. In certain embodiments, the targeting peptide provides binding to an ER or Golgi component, such as to a mannosidase II enzyme. In other embodiments, the targeting peptide provides direct binding to the ER or Golgi membrane.

Components of the targeting peptide may come from any enzyme that normally resides in the ER or Golgi apparatus. Such enzymes include mannosidases, mannosyltransferases, glycosyltransferases, Type 2 Golgi proteins, and MNN2, MNN4, MNN6, MNN9, MNN10, MNS1, KRE2, VAN1, and OCH1 enzymes. Such enzymes may come from a yeast or fungal species such as those of *Acremonium, Aspergillus, Aureobasidium, Cryptococcus, Chrysosporium, Chrysosporium lucknowense, Filobasidium, Fusarium, Gibberella, Humicola, Magnaporthe, Mucor, Myceliophthora, Myrothecium, Neocallimastix, Neurospora, Paecilomyces, Penicillium, Piromyces, Schizophyllum, Talaromyces, Thermoascus, Thielavia, Tolypocladium,* and *Trichoderma.* Sequences for such enzymes can be found in the GenBank sequence database.

In certain embodiments the targeting peptide comes from the same enzyme and organism as one of the catalytic domains of the recombinant protein. For example, if the recombinant protein includes a human GnTII catalytic domain, the targeting peptide of the recombinant protein is from the human GnTII enzyme. In other embodiments, the targeting peptide may come from a different enzyme and/or organism as the catalytic domains of the recombinant protein.

Examples of various targeting peptides for use in targeting proteins to the ER or Golgi that may be used for targeting the recombinant enzymes, include: Kre2/Mnt1 N-terminal peptide fused to galactosyltransferase (Schwientek, JBC 1996, 3398), HDEL for localization of mannosidase to ER of yeast cells to produce Man5 (Chiba, JBC 1998, 26298-304; Callewaert, FEBS Lett 2001, 173-178), OCH1 targeting peptide fused to GnTI catalytic domain (Yoshida et al, Glycobiology 1999, 53-8), yeast N-terminal peptide of Mns1 fused to α2-mannosidase (Martinet et al, Biotech Lett 1998, 1171), N-terminal portion of Kre2 linked to catalytic domain of GnTI or β4GalT (Vervecken, Appl. Environ Microb 2004, 2639-46), various approaches reviewed in Wildt and Gerngross (Nature Rev Biotech 2005, 119), full-length GnTI in Aspergillus nidulans (Kalsner et al, Glycocon. J 1995, 360-370), full-length GnTI in Aspergillus oryzae (Kasajima et al, Biosci Biotech Biochem 2006, 2662-8), portion of yeast Sec12 localization structure fused to C. elegans GnTI in Aspergillus (Kainz et al 2008), N-terminal portion of yeast Mnn9 fused to human GnTI in Aspergillus (Kainz et al 2008), N-terminal portion of Aspergillus Mnn10 fused to human GnTI (Kainz et al, Appl. Environ Microb 2008, 1076-86), and full-length human GnTI in *T. reesei* (Maras et al, FEBS Lett 1999, 365-70).

In certain embodiments the targeting peptide is an N-terminal portion of the Mnt1/Kre2 targeting peptide having the amino acid sequence of SEQ ID NO: 40 (for example encoded by the polynucleotide of SEQ ID NO:41). In certain embodiments, the targeting peptide is selected from human GNT2, KRE2, KRE2-like, Och1, Anp1, Van1 as shown in the Table 1 below:

**Table 1: Amino acid sequence of targeting peptides**

| **Protein** | **TreID** | **Amino acid sequence** |
|---|---|---|
| human GNT2 | - | |
| KRE2 | 21576 | |
| KRE2-like | 69211 | |
| Och1 | 65646 | MLNPRRALIAAAFILTVFFLISRSHNSESASTS (SEQ ID NO:45) |
| Anp1 | 82551 | |
| Van1 | 81211 | |

Further examples of sequences that may be used for targeting peptides include the targeting sequences as described in WO2012/069593.

Uncharacterized sequences may be tested for use as targeting peptides by expressing enzymes of the glycosylation pathway in a host cell, where one of the enzymes contains the uncharacterized sequence as the sole targeting peptide, and measuring the glycans produced in view of the cytoplasmic localization of glycan biosynthesis (e.g. as in Schwientek JBC 1996 3398), or by expressing a fluorescent reporter protein fused with the targeting peptide, and analysing the localization of the protein in the Golgi by immunofluorescence or by fractionating the cytoplasmic membranes of the Golgi and measuring the location of the protein.

### Methods for producing a glycoprotein having increased N-glycosylation site occupancy

The filamentous fungal cells as described above are useful in methods for producing a glycoprotein composition with increased N-glycosylation site occupancy.

Accordingly, in another aspect, the disclosure relates to a method for producing a glycoprotein composition with increased N-glycosylation site occupancy, comprising
a) providing a filamentous fungal cell, for example a *Trichoderma* cell, having a *Leishmania* STT3D gene encoding a catalytic subunit of oligosaccharyl transferase, or a functional variant thereof, and a polynucleotide encoding a heterologous glycoprotein,
b) culturing the cell under appropriate conditions for expression of the STT3D gene or its functional variant, and the production of the heterologous glycoprotein; and,
c) recovering said glycoprotein composition and, optionally, purifying the heterologous glycoprotein composition.

In specific embodiments of the method, the filamentous fungal cell comprises one or more mutation that reduces or eliminates one or more endogenous protease activity compared to a parental filamentous fungal cell which does not have said mutation(s), as described above.

In methods of the disclosure, certain growth media include, for example, common commercially-prepared media such as Luria-Bertani (LB) broth, Sabouraud Dextrose (SD) broth or Yeast medium (YM) broth. Other defined or synthetic growth media may also be used and the appropriate medium for growth of the particular host cell will be known by someone skilled in the art of microbiology or fermentation science. Culture medium typically has the *Trichoderma reesei* minimal medium (Penttilä et al., 1987, Gene 61, 155-164) as a basis, supplemented with substances inducing the production promoter such as lactose, cellulose, spent grain or sophorose. Temperature ranges and other conditions suitable for growth are known in the art (see, e.g., Bailey and Ollis 1986). In certain embodiments the pH of cell culture is between 3.5 and 7.5, between 4.0 and 7.0, between 4.5 and 6.5, between 5 and 5.5, or at 5.5. In certain embodiments, to produce an antibody the filamentous fungal cell or *Trichoderma* fungal cell is cultured at a pH range selected from 4.7 to 6.5; pH 4.8 to 6.0; pH 4.9 to 5.9; and pH 5.0 to 5.8.

In some embodiments of the disclosure, the method comprises culturing in a medium comprising one or two protease inhibitors.

In a specific embodiment of the disclosure, the method comprises culturing in a medium comprising one or two protease inhibitors selected from SBTI and chymostatin.

In some embodiments, the glycoprotein is a heterologous glycoprotein, preferably a mammalian glycoprotein. In other embodiments, the heterologous glycoprotein is a non-mammalian glycoprotein.

In certain embodiments, a mammalian glycoprotein is selected from an immunoglobulin, immunoglobulin or antibody heavy or light chain, a monoclonal antibody, a Fab fragment, an F(ab')2 antibody fragment, a single chain antibody, a monomeric or multimeric single domain antibody, a camelid antibody, or their antigen-binding fragments.

A fragment of a protein, as used herein, consists of at least 10, 20, 30, 40, 50, 60, 70, 80, 90, 100 consecutive amino acids of a reference protein.

As used herein, an "immunoglobulin" refers to a multimeric protein containing a heavy chain and a light chain covalently coupled together and capable of specifically combining with antigen. Immunoglobulin molecules are a large family of molecules that include several types of molecules such as IgM, IgD, IgG, IgA, and IgE.

As used herein, an "antibody" refers to intact immunoglobulin molecules, as well as fragments thereof which are capable of binding an antigen. These include hybrid (chimeric) antibody molecules (see, e.g., Winter et al. Nature 349:293-99225, 1991; and U.S. Pat No. 4,816,567 226); F(ab')2 molecules; non-covalent heterodimers; dimeric and trimeric antibody fragment constructs; humanized antibody molecules (see e.g., Riechmann et al. Nature 332, 323-27, 1988; Verhoeyan et al. Science 239, 1534-36, 1988; and GB 2,276,169); and any functional fragments obtained from such molecules, as well as antibodies obtained through non-conventional processes such as phage display or transgenic mice. Preferably, the antibodies are classical antibodies with Fc region. Methods of manufacturing antibodies are well known in the art.

In further embodiments, the yield of the mammalian glycoprotein, for example, the antibody, is at least 0.5, at least 1, at least 2, at least 3, at least 4, or at least 5 grams per liter.

In certain embodiments, the mammalian glycoprotein is an antibody, optionally, IgG1, IgG2, IgG3, or IgG4. In further embodiments, the yield of the antibody is at least 0.5, at least 1, at least 2, at least 3, at least 4, or at least 5 grams per liter. In further embodiments, the mammalian glycoprotein is an antibody, and the antibody contains at least 70 %, at least 80 %, at least 90 %, at least 95 %, or at least 98 % of a natural antibody C-terminus and N-terminus without additional amino acid residues. In other embodiments, the mammalian glycoprotein is an antibody, and the antibody contains at least 70 %, at least 80 %, at least 90 %, at least 95 %, or at least 98 % of a natural antibody C-terminus and N-terminus that do not lack any C-terminal or N-terminal amino acid residues.

In certain embodiments where the mammalian glycoprotein (e.g. the antibody) is purified from cell culture, the culture containing the mammalian glycoprotein contains polypeptide fragments that make up a mass percentage that is less than 50%, less than 40%, less than 30%, less than 20%, or less than 10% of the mass of the produced polypeptides. In certain preferred embodiments, the mammalian glycoprotein is an antibody, and the polypeptide fragments are heavy chain fragments and/or light chain fragments. In other embodiments, where the mammalian glycoprotein is an antibody and the antibody purified from cell culture, the culture containing the antibody contains free heavy chains and/or free light chains that make up a mass percentage that is less than 50%, less than 40%, less than 30%, less than 20%, or less than 10% of the mass of the produced antibody. Methods of determining the mass percentage of polypeptide fragments are well known in the art and include, measuring signal intensity from an SDS-gel.

In other embodiments, the heterologous glycoprotein (e.g. the antibody) with increased N-glycosylation site occupancy, for example, the antibody, comprises the trimannosyl N-glycan structure Manα3[Manα6]Manβ4GlcNAcβ4GlcNAc. In some embodiments, the Manα3[Manα6]Manβ4GlcNAcβ4GlcNAc structure represents at least 20%, 30%; 40%, 50%; 60%, 70%, 80% (mol%) or more, of the total N-glycans of the heterologous glycoprotein (e.g. the antibody) composition obtained by the methods of the disclosure. In other embodiments, the heterologous glycoprotein (e.g. the antibody) comprises the G0 N-glycan structure GlcNAcβ2Manα3[GlcNAcβ2Manα6]Manβ4GlcNAcβ4GlcNAc. In other embodiments, the non-fucosylated G0 glycoform structure represents at least 20%, 30%; 40%, 50%; 60%, 70%, 80% (mol%) or more, of the total N-glycans of the heterologous glycoprotein (e.g. the antibody) composition obtained by the methods of the disclosure. In other embodiments, galactosylated N-glycans represents less (mol%) than 0.5%, 0.1%, 0.05%, 0.01% of total N-glycans of the culture, and/or of the heterologous glycoprotein with increased N-glycosylation site occupancy. In certain embodiments, the culture or the heterologous glycoprotein, for example an antibody, comprises no galactosylated N-glycans.

In certain embodiments of any of the disclosed methods, the method includes the further step of providing one or more, two or more, three or more, four or more, or five or more protease inhibitors. In certain embodiments, the protease inhibitors are peptides that are co-expressed with the mammalian glycoprotein. In other embodiments, the inhibitors inhibit at least two, at least three, or at least four proteases from a protease family selected from aspartic proteases, trypsin-like serine proteases, subtilisin proteases, and glutamic proteases.

In certain embodiments of any of the disclosed methods, the filamentous fungal cell or *Trichoderma* fungal cell also contains a carrier protein. As used herein, a "carrier protein" is portion of a protein that is endogenous to and highly secreted by a filamentous fungal cell or *Trichoderma* fungal cell. Suitable carrier proteins include, without limitation, those of *T. reesei* mannanase I (Man5A, or MANI), *T. reesei* cellobiohydrolase II (Cel6A, or CBHII) (see, e.g., Paloheimo et al Appl. Environ. Microbiol. 2003 December; 69(12): 7073-7082) or *T. reesei* cellobiohydrolase I (CBHI). In some embodiments, the carrier protein is CBH1. In other embodiments, the carrier protein is a truncated *T. reesei* CBH1 protein that includes the CBH1 core region and part of the CBH1 linker region. In some embodiments, a carrier such as a cellobiohydrolase or its fragment is fused to an antibody light chain and/or an antibody heavy chain. In some embodiments, a carrier-antibody fusion polypeptide comprises a Kex2 cleavage site. In certain embodiments, Kex2, or other carrier cleaving enzyme, is endogenous to a filamentous fungal cell. In certain embodiments, carrier cleaving protease is heterologous to the filamentous fungal cell, for example, another Kex2 protein derived from yeast or a TEV protease. In certain embodiments, carrier cleaving enzyme is overexpressed. In certain embodiments, the carrier consists of about 469 to 478 amino acids of N-terminal part of the *T. reesei* CBH1 protein GenBank accession No. EGR44817.1.

In one embodiment, the polynucleotide encoding the heterologous glycoprotein (e.g. the antibody) further comprises a polynucleotide encoding CBH1 catalytic domain and linker as a carrier protein, and/or cbh1 promoter.

In certain embodiments, the filamentous fungal cell of the disclosure overexpress KEX2 protease. In an embodiment the heterologous glycoprotein (e.g. the antibody) is expressed as fusion construct comprising an endogenous fungal polypeptide, a protease site such as a Kex2 cleavage site, and the heterologous protein such as an antibody heavy and/or light chain. Useful 2-7 amino acids combinations preceding Kex2 cleavage site have been described, for example, in Mikosch et al. (1996) J. Biotechnol. 52:97-106; Goller et al. (1998) Appl Environ Microbiol. 64:3202-3208; Spencer et al. (1998) Eur. J. Biochem. 258:107-112; Jalving et al. (2000) Appl. Environ. Microbiol. 66:363-368; Ward et al. (2004) Appl. Environ. Microbiol. 70:2567-2576; Ahn et al. (2004) Appl. Microbiol. Biotechnol. 64:833-839; Paloheimo et al. (2007) Appl Environ Microbiol. 73:3215-3224; Paloheimo et al. (2003) Appl Environ Microbiol. 69:7073-7082; and Margolles-Clark et al. (1996) Eur J Biochem. 237:553-560.

The disclosure further relates to the glycoprotein composition, for example the antibody composition, obtainable or obtained by the method as disclosed above.

In other specific embodiments, such glycoprotein or antibody composition further comprises as 50%, 60 %, 70 % or 80 % (mole% neutral N-glycan), of the following glycoform:
(i) Manα3[Manα6(Manα3)Manα6]Manβ4GlcNAβ4GlcNAc (Man5 glycoform);
(ii) GlcNAcβ2Manα3[Manα6(Manα3)Manα6]Manβ4GlcNAβ4GlcNAc, or β4-galactosylated variant thereof;
(iii) Manα6(Manα3)Manβ4GlcNAβ4GlcNAc;
(iv) Manα6(GlcNAcβ2Manα3)Manβ4GlcNAβ4GlcNAc, or β4-galactosylated variant thereof: or,
(v) complex type N-glycans selected from the G0, G1 or G2 glycoform.

In some embodiments the N-glycan glycoform according to iii-v comprises less than 15 %, 10 %, 7 %, 5 %, 3 %, 1 % or 0.5 % or is devoid of Man5 glycan as defined in i) above.

### EXAMPLES

### FUNCTIONAL ASSAYS

### Assay for measuring total protease activity of cells of the disclosure

The protein concentrations were determined from supernatant samples from day 2-7 of 1x-7x protease deficient strains (described in PCT/EP2013/050126) according to EnzChek protease assay kit (Molecular probes #E6638, green fluorescent casein substrate). Briefly, the supernatants were diluted in sodium citrate buffer to equal total protein concentration and equal amounts of the diluted supernatants were added into a black 96 well plate, using 3 replicate wells per sample. Casein FL diluted stock made in sodium citrate buffer was added to each supernatant containing well and the plates were incubated covered in plastic bag at 37°C. The fluorescence from the wells was measured after 2, 3, and 4 hours. The readings were done on the Varioskan fluorescent plate reader using 485 nm excitation and 530 nm emission. Some protease activity measurements were performed using succinylated casein (QuantiCleave protease assay kit, Pierce #23263) according to the manufacturer's protocol.

The pep1 single deletion reduced the protease activity by 1.7-fold, the pep1/tsp1 double deletion reduced the protease activity by 2-fold, the pep1/tsp1/slp1 triple deletion reduced the protease activity by 3.2-fold, the pep1/tsp1/slp1/gap1 quadruple deletion reduced the protease activity by 7.8-fold compared to the wild type M124 strain, the pep1/tsp1/slp1/gap1/gap2 5-fold deletion reduced the protease activity by 10-fold, the pep1/tsp1/slp1/gap1/gap2/pep4 6-fold deletion reduced the protease activity by 15.9-fold, and the pep1/tsp1/slp1/gap1/gap2/pep4/pep3 7-fold deletion reduced the protease activity by 18.2-fold.

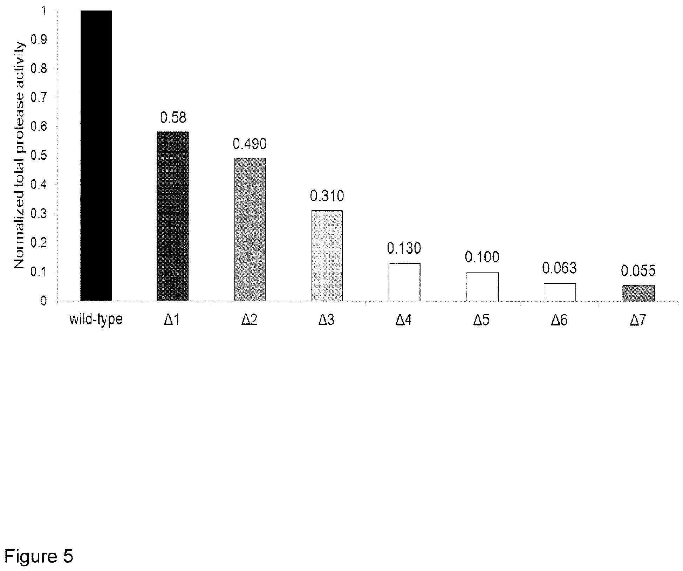

Figure 5 graphically depicts normalized protease activity data from culture supernatants from each of the protease deletion supernatants (from 1-fold to 7-fold deletion mutant) and the parent strain without protease deletions. Protease activity was measured at pH 5.5 in first 5 strains and at pH 4.5 in the last three deletion strains. Protease activity is against green fluorescent casein. The six-fold protease deletion strain has only 6% of the wild type parent strain and the 7-fold protease deletion strain protease activity was about 40% less than the 6-fold protease deletion strain activity.

### Assay for measuring N-glycosylation site occupancy in a glycoprotein composition

10 - 30 µg of antibody is digested with 13.4 - 30 U of FabRICATOR (Genovis), +37°C, 60 min - overnight, producing one F(ab')2 fragment and one Fc fragment per an antibody molecule. Digested samples are purified using Poros R1 filter plate (Glyken corp.) and the Fc fragments are analysed for N-glycan site occupancy using MALDI-TOF MS. The percentage of site occupancy of an Fc is the average of two values: the one obtained from intensity values of the peaks (single and double charged) and the other from area of the peaks (single and double charged); both the values are calculated as glycosylated signal divided by the sum of non-glycosylated and glycosylated signals.

### EXAMPLE 1 - Generation of T. reesei expressing L. major STT3

The *Leishmania major* oligosaccharyl transferase 3D (old GenBank No. XP_843223.1, new XP_003722509.1; SEQ ID NO: 1) coding sequence was codon optimized for *Trichoderma reesei* expression (codon optimized nucleic acid sequence SEQ ID NO: 2). The optimized coding sequence was synthesized along with cDNA1 promoter (SEQ ID NO: 3) and TrpC terminator flanking sequence (SEQ ID NO: 4). The *Leishmania major* STT3 gene was excised from the optimized cloning vector using Pacl restriction enzyme digestion. The expression entry vector was also digested with Pacl and dephosphorylated with calf alkaline phosphatase. The STT3 gene and the digested vector were separated with agarose gel electrophoresis and correct fragments were isolated from the gel with a gel extraction kit (Qiagen) according to manufacturer's protocol. The purified *Leishmania major* STT3 gene was ligated into the expression vector with T4 DNA ligase. The ligation reaction was transformed into chemically competent DH5α *E. coli* and grown on ampicillin (100 µg/ml) selection plates. Miniprep plasmid preparations were made from several colonies. The presence of the *Leishmania major* STT3 gene insert was checked by digesting the prepared plasmids with Pacl digestion and several positive clones were sequenced to verify the gene orientation. One correctly orientated clone was chosen to be the final vector pTTv201.

The expression cassette contained the constitutive *cDNA1* promoter from *Trichoderma reesei* to drive expression of *Leishmania major STT3.* The terminator sequence included in the cassette was the TrpC terminator from *Aspergillus niger.* The expression cassette was targeted into the xylanase 1 locus (*xyn1,* tre74223) using the xylanase 1 sequence from the 5' and 3' flanks of the gene (SEQ ID NO: 5 and SEQ ID NO: 6). These sequences were included in the cassette to allow the cassette to integrate into the *xyn1* locus via homologous recombination. The cassette contained a *pyr4* loopout marker for selection. The *pyr4* gene encodes the orotidine-5'-monophosphate (OMP) decarboxylase of *T. reesei* (Smith, J.L., et al., 1991, Current Genetics 19:27-33) and is needed for uridine synthesis. Strains deficient for OMP decarboxylase activity are unable to grow on minimal medium without uridine supplementation (i.e. are uridine auxotrophs).

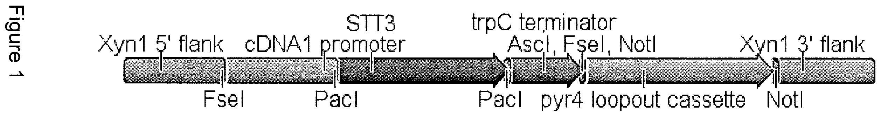

To prepare the vector for transformation, the vector was cut with Pmel to release the expression cassette (Fig. 1). The digest was separated with agarose gel electrophoresis and the correct fragment was isolated from the gel with a gel extraction kit (Qiagen) according to manufacturer's protocol. The purified expression cassette DNA (5 µg) was then transformed into protoplasts of the *Trichoderma reesei* strain M317 (M317 has been described in the International Patent Application No. PCT/EP2013/050126; M317 is pyr4- of M304 and it comprises MAB01 light chain fused to *T*. *reesei* truncated CBH1 carrier with NVISKR Kex2 cleavage sequence, MAB01 heavy chain fused to *T. reesei* truncated CBH1 carrier with AXE1 [DGETVVKR] Kex2 cleavage sequence, Δ*pep1*Δ*tsp1*Δ*slp1,* and overexpression of *T. reesei* KEX2). Preparation of protoplasts and transformation were carried out according to methods in Penttilä et al. (1987, Gene 61:155-164) and Gruber et al (1990, Curr. Genet. 18:71-76) for pyr4 selection. The transformed protoplasts were plated onto *Trichoderma* minimal media (TrMM) plates.

Transformants were then streaked onto TrMM plates with 0.1% TritonX-100. Transformants growing fast as selective streaks were screened by PCR using the primers listed in Table 1. DNA from mycelia was purified and analyzed by PCR to look at the integration of the 5' and 3' flanks of cassette and the existence of the xylanase 1 ORF. The cassette was targeted into the xylanase 1 locus; therefore the open reading frame was not present in the positively integrated transformants. To screen for 5' integration, sequence outside of the 5' integration flank was used to create a forward primer that would amplify genomic DNA flanking *xyn1* and the reverse primer was made from sequence in the cDNA promoter of the cassette. To check for proper integration of the cassette in the 3' flank, a forward primer was made from sequence outside of the 3' integration flank that would amplify genomic DNA flanking *xyn1* and the reverse primer was made from sequence in the *pyr4* marker. Thus, one primer would amply sequence from genomic DNA outside of the cassette and the other would amply sequence from DNA in the cassette. The primer sequences are listed in Table 1. Four final strains showing proper integration and a deletion of *xyn1* orf were called M420-M423.

Shake flask cultures were conducted for four of the STT3 producing strains (M420-M423) to evaluate growth characteristics and to provide samples for glycosylation site occupancy analysis. The shake flask cultures were done in TrMM, 40 g/l lactose, 20 g/l spent grain extract, 9 g/l casamino acids, 100 mM PIPPS, pH 5.5. *L. major* STT3 expression did not affect growth negatively when compared to the parental strain M304 (Tables 2 and 3). The cell dry weight for the STT3 expressing transformants appeared to be slightly higher compared to the parent strain M304.

**Table 1: List of primers used for PCR screening of STT3 transformants.**

| **5' flank screening primers:** | **1205 bp product** |
|---|---|
| T403_Xyn1_5'flank_fwd | CCGCGTTGAACGGCTTCCCA ID NO:48) |
| T140_cDNA1 promoter_rev | TAACTTGTACGCTCTCAGTTCGAG (SEQ ID NO:49) |

| **3' flank screening primers:** | **1697 bp product** |
|---|---|
| T404_Xyn1 3'flank fwd | GCGACGGCGACCCATTAGCA ID NO:50) |
| T028_Pyr4_flank_rev | CATCCTCAAGGCCTCAGAC (SEQ ID NO:51) |

| **xylanase 1 orf primers:** | **589 bp product** |
|---|---|
| T405_Xyn1_orf_screen_fwd | TGCGCTCTCACCAGCATCGC ID NO:52) |
| T406_Xyn1_orf_screen_rev | GTCCTGGGCGAGTTCCGCAC (SEQ ID NO:53) |

**Table 2: Cell dry weight from large shake flask cultures.**

| **Cell dry weight (g/L)** | | | |
|---|---|---|---|
| | **day 3** | **day 5** | **day 7** |
| M304 | 2.3 | 3.3 | 4.3 |
| M420 | 3.7 | 4.3 | 5.4 |
| M421 | 3.7 | 4.6 | 6.3 |
| M422 | 3.8 | 4.5 | 5.4 |
| M423 | 3.7 | 4.6 | 5.7 |

**Table 3: pH values from large shake flask cultures.**

| **pH values** | | | |
|---|---|---|---|
| | **day 3** | **day 5** | **day 7** |
| M304 | 5.6 | 6.1 | 6.2 |
| M420 | 6.1 | 6.1 | 6.1 |
| M421 | 6.0 | 5.9 | 6.0 |
| M422 | 6.1 | 6.1 | 6.2 |
| M423 | 6.1 | 6.1 | 6.1 |

### Site occupancy analysis

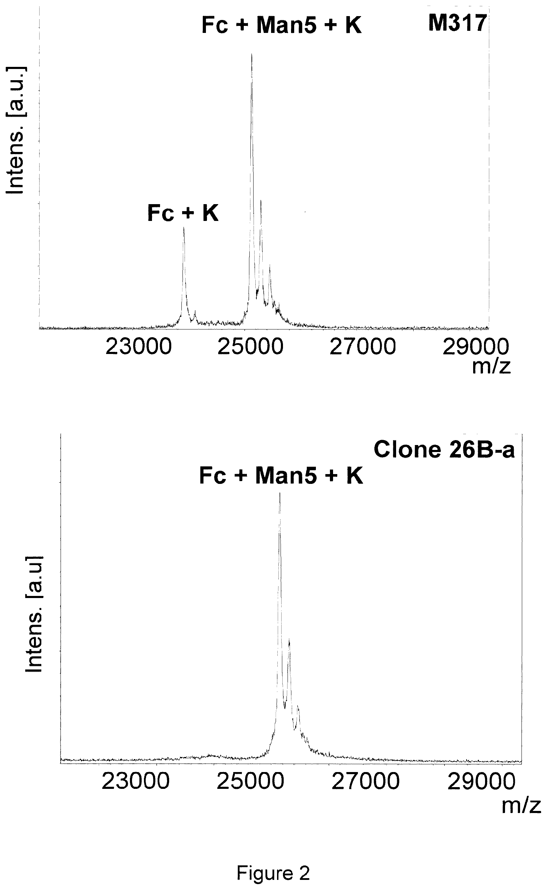

Four transformants [pTTv201; 17A-a (M420), 26B-a (M421), 65B-a (M422) and 97A-a (M423)] and their parental strain (M317) were cultivated in shake flasks and samples at day 5 and 7 time points were collected. MAB01 antibody was purified from culture supernatants using Protein G HP MultiTrap 96-well plate (GE Healthcare) according to manufacturer's instructions. The antibody was eluted with 0.1 M citrate buffer, pH 2.6 and neutralized with 2 M Tris, pH 9. The concentration was determined via UV absorbance in spectrophotometer against MAB01 standard curve. 10 µg of antibody was digested with 13.4 U of FabRICATOR (Genovis), +37°C, 60 min, producing one F(ab')2 fragment and one Fc fragment. Digested samples were purified using Poros R1 filter plate (Glyken corp.) and the Fc fragments were analysed for N-glycan site occupancy using MALDI-TOF MS (Fig. 2).

The overexpression of *STT3* from *Leishmania major* enhanced the site coverage compared to the parental strain. The best clone was re-cultivated in three parallel shake flasks each and the analysis results were comparable to the first analysis. Compared to parental strain the signals Fc and Fc + K are practically absent in STT3 clones.

The difference in site occupancy between parental strain and all clones of STT3 from *L. major* was significant (Fig. 2). Because the signals coming from Fc or Fc + K were practically absent, the N-glycan site occupancy of MAB01 in these shake flask cultivations was 100% (Table 4).

**Table 4: Site occupancy analysis of parental strain M317 and four transformants of STT3 from L. major. The averages have been calculated from area and intensity from single and double charged signals from three parallel samples.**

| | **M317** | **17A-a** | **26B-a** | **65B-a** | **97A-a** |
|---|---|---|---|---|---|
| **Glycosylation state** | **Average %** | **Average %** | **Average %** | **Average %** | **Average %** |
| Non-glycosylated | 13.0 | 0.0 | 0.0 | 0.0 | 0.0 |
| Glycosylated | 87.0 | 100.0 | 100.0 | 100.0 | 100.0 |

### Fermenter cultivations

Three STT3 (L. major) clones (M420, M421 and M422) as well as parental strain M304 were cultivated in fermenter. Samples at day 3, 4, 5, 6 and 7 time points were collected and the site occupancy analysis was performed to purified antibody. STT3 overexpression strains and the respective control strain (M304) were grown in batch fermentations for 7 days, in media containing 2% yeast extract, 4% cellulose, 4% cellobiose, 2% sorbose, 5g/L KH2PO4, and 5g/L (NH4)2SO4. Culture pH was controlled at pH 5.5 (adjusted with NH3OH). The temperature was shifted from 28 °C to 22°C at 48 hours elapsed process time. Fermentations were carried out in 4 parallel 2 L glass vessel reactors with a culture volume of 1 L. Culture supernatant samples were taken during the course of the runs and stored at -20 ºC. MAB01 antibody was purified and digested with FabRICATOR as described above. The antibody titers are shown in Table 5.

### Results

The site occupancy in parental strain M304 was less than 60% but in all analyzed STT3 clones the site occupancy had increased up to 98% (Table 6).

**Table 5: MAB01 antibody titers of the LmSTT3 strains M420, M421 and M422 and their parental strain M304.**

| | **Titer g/l** | | | | |
|---|---|---|---|---|---|
| **Strain** | **d3** | **d4** | **d5** | **d6** | **d7** |
| **M304** | 0.225 | 0.507 | 0.981 | 1.52 | 1.7 |
| **M420** | 0.758 | 1.21 | 1.55 | 1.71 | 1.69 |
| **M421** | 0.76 | 1.24 | 1.54 | 1.67 | 1.6 |
| **M422** | 0.65 | 1.07 | 1.43 | 1.56 | 1.54 |

**Table 6: The N-glycosylation site occupancies of MAB01 antibody of the LmSTT3 strains M420, M421 and M422 and their parental strain M304.**

| | **Site occupancy %** | | | | |
|---|---|---|---|---|---|
| **Strain** | **d3** | **d4** | **d5** | **d6** | **d7** |
| **M304** | 48.0 | 47.7 | 47.7 | 46.3 | 55.4 |
| **M420** | 97.8 | 97.5 | 96.9 | 94.3 | 94.6 |
| **M421** | 96.1 | 90.8 | 91.5 | 89.7 | 95.6 |
| **M422** | 94.4 | 88.5 | 80.9 | 83.6 | 75.2 |

In conclusion, overexpression of the STT3D gene from *L. major* increased the N-glycosylation site occupancy from 46%-87% in the parental strain to 98%-100% in transformants having *Leishmania* STT3 under shake flask or fermentation culture conditions.

The overexpression of the STT3D gene from *L. major* significantly increased the N-glycosylation site occupancy in strains producing an antibody as a heterologous protein. The antibody titers did not vary significantly between transformants having STT3 and parental strain.

### EXAMPLE 2 - Generation of T. reesei strains expressing STT3 from T. vaginalis, L. infantums or E. histolytica

The coding sequences of the *Trichomonas vaginalis, Leishmania infantum* and *Entamoeba histolytica* oligosaccharyl transferase (STT3; amino acid sequences *T. vaginalis* SEQ ID NO: 7, *L*. *infantum* SEQ ID NO: 8, and *E. histolytica* SEQ ID NO: 10) were codon optimized for *Trichoderma reesei* expression (codon optimized *L. infantum* nucleic acid SEQ ID NO: 9). The optimized coding sequences were synthesized along with *T. reesei cbh1* terminator flanking sequence (SEQ ID NO: 11). Plasmids containing the STT3 genes under the constitutive *cDNA1* promoter, with *cbh1* terminator, *pyr4* loopout marker and *alg3* flanking regions (SEQ ID NO: 12 and SEQ ID NO: 13) were cloned by yeast homologous recombination as described in WO2012/069593. *Not*I fragment of plasmid pTTv38 was used as vector backbone. This vector contains *alg3* (tre104121) 5' and 3' flanks of the gene to allow the expression cassette to integrate into the *alg3* locus via homologous recombination in *T. reesei* and the plasmid has been described in WO2012/069593. The *STT3* genes were excised from the cloning vectors using *Sfi*I restriction enzyme digestion. The *cdna1* promoter and *cbh1* terminator fragments were created by PCR, using plasmids pTTv163 and pTTv166 as templates, respectively. The *pyr4* loopout marker was extracted from plasmid pTTv142 by *Not*I digestion (the plasmid pTTv142 having a human GNT2 catalytic domain fused with T. reesei MNT1/KRE2 targeting peptide has been described in WO2012/069593). The *pyr4* gene encodes the orotidine-5'-monophosphate (OMP) decarboxylase of *T. reesei* (Smith, J.L., et al., 1991, Current Genetics 19:27-33) and is needed for uridine synthesis. Strains deficient for OMP decarboxylase activity are unable to grow on minimal medium without uridine supplementation (i.e. are uridine auxotrophs). The primers used for cloning are listed in Table 7. The digested fragments and PCR products were separated with agarose gel electrophoresis and correct fragments were isolated from the gel with a gel extraction kit (Qiagen) according to manufacturer's protocol. The plasmids were constructed using the yeast homologous recombination method, using overlapping oligonucleotides for the recombination of the gap between the *pyr4* marker and *alg3* 3' flank as described in WO2012/069593. The plasmid DNA were rescued from yeast and transformed into electrocompetent TOP10 *E. coli* that were grown on ampicillin (100 µg/ml) selection plates. Miniprep plasmid preparations were made from several colonies. The presence of the *Trichomonas vaginalis* and *Leishmania infantum* STT3 genes was confirmed by digesting the prepared plasmids with *Bgl*II*-Kpn*I whereas the *Entamoeba histolytica* plasmid was digested with *Hind*III-*Kpn*I*.* Positive clones were sequenced to verify the plasmid sequences. One correct *Trichomonas vaginalis* clone was chosen to be the final vector pTTv321, and correct clones of *Leishmania infantum* and *Entamoeba histolytica* were chosen to be the pTTv322 and pTTv323 vectors, respectively. The primers used for sequencing the vectors are listed in Table 8.

**Table 7: List of primers used for cloning vectors pTTv321, pTTv322 and pTTv323.**

| Fragment | Primer | Primer sequence |
|---|---|---|
| *cDNA1* promoter, pTTv321 | T1177_pTTv321_1 | |
| | T1178_pTTv321_2 | |
| | | |
| *cDNA1* promoter, pTTv322 | T1177_pTTv321_1 | |
| | T1183_pTTv322_1 | |
| *cDNA1* promoter, pTTv323 | T1177_pTTv321_1 | |
| | T1184_pTTv323_1 | |
| *cbh1* terminator | T1179_pTTv321_3 | |
| | T1180_pTTv321_4 | |
| *pyr4-alg3* 3' flank overlappin g oligos | T1181_pTTv321_5 | |
| | T1182_pTTv321_6 | |

**Table 8: List of primers used for sequencing vectors pTTv321, pTTv322 and pTTv323.**

| Primer | Sequence |
|---|---|
| T027_Pyr4_orf_start_rev | TGCGTCGCCGTCTCGCTCCT (SEQ ID NO:64) |
| T061_pyr4_orf_screen_2F | TTAGGCGACCTCTTTTTCCA (SEQ ID NO:65) |
| T143_cDNA1 promoter_seqF3 | CGAGGAAGTCTCGTGAGGAT (SEQ ID NO:66) |
| T410_alg3_5-flank_F | CAGCTAAACCGACGGGCCA (SEQ ID NO:67) |
| T1153_cbh1_term_start_rev | GACCGTATATTTGAAAAGGG (SEQ ID NO:68) |

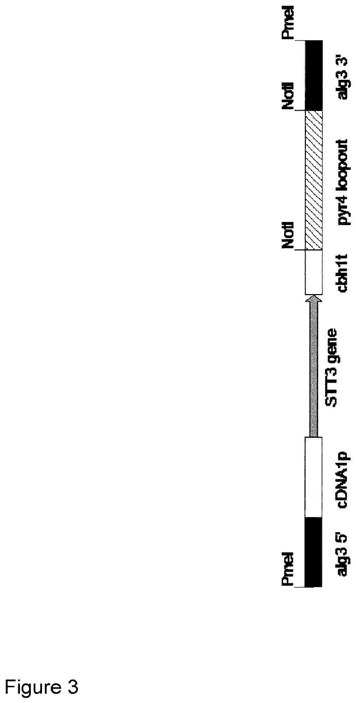

To prepare the vectors for transformation, the vectors were cut with *Pme*I to release the expression cassettes (Fig. 3). The fragments were separated with agarose gel electrophoresis and the correct fragment was isolated from the gel with a gel extraction kit (Qiagen) according to manufacturer's protocol. The purified expression cassette DNA was then transformed into protoplasts of the *Trichoderma reesei* M317. Preparation of protoplasts and transformation were carried out essentially according to methods in Penttilä et al. (1987, Gene 61:155-164) and Gruber et al (1990, Curr. Genet. 18:71-76) for *pyr4* selection. The transformed protoplasts were plated onto *Trichoderma* minimal media (TrMM) plates containing sorbitol.

Transformants were then streaked onto TrMM plates with 0.1% TritonX-100. Transformants growing fast as selective streaks were screened by PCR using the primers listed in Table 9. DNA from mycelia was purified and analyzed by PCR to look at the integration of the 5' and 3' flanks of cassette and the existence of the *alg3* ORF. The cassette was targeted into the *alg3* locus; therefore the open reading frame was not present in the positively integrated transformants, purified to single cell clones. To screen for 5' integration, sequence outside of the 5' integration flank was used to create a forward primer that would amplify genomic DNA flanking *alg3* and the reverse primer was made from sequence in the *cDNA1* promoter of the cassette. To check for proper integration of the cassette in the 3' flank, a reverse primer was made from sequence outside of the 3' integration flank that would amplify genomic DNA flanking *alg3* and the forward primer was made from sequence in the *pyr4* marker. Thus, one primer would amplify sequence from genomic DNA outside of the cassette and the other would amplify sequence from DNA in the cassette.

**Table 9: List of primers used for PCR screening of T. reesei transformants.**

| | |
|---|---|
| 5' flank screening primers: | 1165 bp product |
| T066_104121_5int | GATGTTGCGCCTGGGTTGAC (SEQ ID NO:69) |
| T140_cDNA1 promoter_seqR1 | TAACTTGTACGCTCTCAGTTCGA (SEQ ID NO:70) |
| 3' flank screening primers: | 1469 bp product |
| T026_Pyr4_orf_5rev2 | CCATGAGCTTGAACAGGTAA (SEQ ID NO:71) |
| T068_104121_3int | GATTGTCATGGTGTACGTGA (SEQ ID NO:72) |
| alg3 ORF primers: | 689 bp product |
| T767_alg3_del_F | CAAGATGGAGGGCGGCACAG (SEQ ID NO:73) |
| T768_alg3_del_R | GCCAGTAGCGTGATAGAGAAGC (SEQ ID NO:74) |
| alg3 ORF primers: | 1491 bp product |
| T069_104121 _5orf_pcr | GCGTCACTCATCAAAACTGC (SEQ ID NO:75) |
| T070_104121_3orf_pcr | CTTCGGCTTCGATGTTTCA (SEQ ID NO:76) |

Four final strains each showing proper integration and a deletion of *alg3* ORF were grown in large shake flasks in TrMM medium supplemented with 40 g/l lactose, 20 g/l spent grain extract, 9 g/l casamino acids and 100 mM PIPPS, pH 5.5. Growth for pTTv321 and pTTv323 strains was somewhat slower than parental strain M304 (Table 10). Three out of four *Leishmania infantum* pTTv322 clones grew somewhat better than the parental strain.

**Table 10. Cell dry weight measurements (in g/L) of the parental strains M304 and STT3 expressing strains.**

| Strain | 3 days | 5 days | 7 days |
|---|---|---|---|
| M304 | 3,06 | 3,34 | 4,08 |
| pTTv321 #18-9-2 | 2,54 | 2,89 | 2,52 |
| pTTv321 #18-9-10 | 2,44 | 3,03 | 2,65 |
| pTTv321#18-12-1 | 2,43 | 3,12 | 2,86 |
| pTTv321 #18-12-2 | 2,84 | 3,49 | 3,39 |
| pTTv322#60-2 | 3,02 | 3,42 | 3,63 |
| pTTv322#60-6 | 3,37 | 4,45 | 4,68 |
| pTTv322#60-12 | 3,30 | 4,15 | 4,29 |
| pTTv322#60-14 | 2,92 | 3,90 | 4,39 |
| pTTv323#37-4-1 | 2,29 | 2,27 | 2,59 |
| pTTv323#37-4-14 | 1,88 | 2,08 | 2,69 |
| pTTv323#37-11-3 | 2,15 | 2,27 | 2,62 |
| pTTv323#37-11-8 | 1,92 | 2,25 | 2,62 |

### Site occupancy and glycan analyses

From day 5 supernatant samples, MAB01 was purified using Protein G HP MultiTrap 96-well filter plate (GE Healthcare) according to manufacturer's instructions. Approx. 1.4 ml of culture supernatant was loaded and the elution volume was 230 µl. The antibody concentrations were determined via UV absorbance against MAB01 standard curve.

For site occupancy analysis 16-20 µg of purified MAB01 antibody was taken and antibodies were digested, purified, and analysed as described in example 1. The 100% site occupancy was achieved with *Leishmania infantum* STT3 clones 60-6, 60-12 and 60-14 (Table 11). In *T. vaginalis* and *E. histolytica* STT3 transformants the site occupancy was low and in the latter the antibodies appeared to be degraded resulting that no site occupancy analysis could be performed for one strain.

**Table 11. N-glycosylation site occupancy of antibodies from STT3 variants and parental M304 at day 5.**

| **M304** | | | | |
|---|---|---|---|---|
| **Glycosylation state** | **%** | | | |
| Non-glycosylated | 8 | | | |
| Glycosylated | 92 | | | |
| | | | | |

| ***Trichomonas vaginalis* STT3, Δalg3** | **18-9-2** | **18-9-10** | **18-12-1** | **18-12-2** |
|---|---|---|---|---|
| **Glycosylation state** | **%** | **%** | **%** | **%** |
| Non-glycosylated | 75 | 71 | 69 | 64 |
| Glycosylated | 25 | 29 | 31 | 36 |
| Glycosylated | 18 | n.d. | 27 | 14 |
| | | | | |

| ***Leishmania infantum* STT3, Δalg3** | **60-2** | **60-6** | **60-12** | **60-14** |
|---|---|---|---|---|
| **Glycosylation state** | **%** | **%** | **%** | **%** |
| Non-glycosylated | 38 | 0 | 0 | 0 |
| Glycosylated | 62 | 100 | 100 | 100 |
| | | | | |

| ***Entamoeba histolytica* STT3, Δalg3** | **37-4-1** | **37-4-14** | **37-11-3** | **37-11-8** |
|---|---|---|---|---|
| **Glycosylation state** | **%** | **%** | **%** | **%** |
| Non-glycosylated | 82 | n.d. | 73 | 86 |

These results shows that overexpression of the catalytic subunit of *Leishmania infantum* is capable of increasing the N-glycosylation site occupancy in filamentous fungal cells, up to 100%.

In contrast, the STT3 genes from *Trichomonas vaginalis* or *Entamoeba histolytica* do not result in high N-glycosylation site occupancy.

N-glycans were analysed from three of the *Leishmania infantum* STT3 clones. The PNGase F reactions were carried out to 20 µg of MAB01 antibody as described in examples and the released N-glycans were analysed with MALDI-TOF MS. The three strains produced about 25% of Man3 N-glycan attached to MAB01 whereas Hex6 glycoform represents about 60% of N-glycans attached to MAB01 (Table 12).

**Table 12: Neutral N-glycans and site occupancy analysis of MAB01 from L. infantum STT3 clones at day 5.**

| ***Leishmania infantum* STT3, Δalg3** | | | | |
|---|---|---|---|---|
| **Clones** | | **60-6** | **60-12** | **60-14** |
| **Short** | **m\z** | **%** | **%** | **%** |
| **Man3** | 933.3 | 25.9 | 26.4 | 25.9 |
| **Man4** | 1095.4 | 9.4 | 9.3 | 9.0 |
| **Man5** | 1257.4 | 6.5 | 6.1 | 7.6 |
| **Hex6** | 1419.5 | 58.3 | 58.2 | 57.5 |
| **Fc** | | 0 | 0 | 0 |
| **Fc+Gn** | | 0 | 0 | 0 |
| **Glycosylated** | | 100 | 100 | 100 |

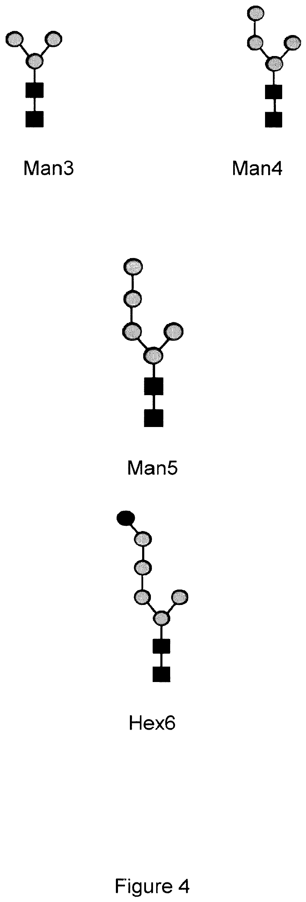

This shows that the Man3, G0, G1 and/or G2 glycoforms represent at least 25% of the total neutral N-glycans of MAB01 in 3 different clones overexpressing STT3 from *L. infantum.* Figure 4 shows the glycan structures of Man3, Man4, Man5, and Hex6 produced in Δalg3 strains. "Fc" means an Fc fragment (without any N-glycans) and "Fc+Gn" means an Fc fragment with one attached N-acetylglucosamine (possible Endo T enzyme activity could cleave N-glycans of an Fc resulting Fc+Gn).

### EXAMPLE 3 - Generation of Δalg3 strains of MAB01 expressing strains

The acetamide marker of the pTTv38 *alg3* deletion plasmid was changed to *pyr4* marker. The pTTv38 and pTTv142 vectors were digested with *Not*I and fragments separated with agarose gel electrophoresis. Correct fragments were isolated from the gel with a gel extraction kit (Qiagen) according to manufacturer's protocol. The purified *pyr4* loopout marker from pTTv142 was ligated into the pTTv38 plasmid with T4 DNA ligase. The ligation reaction was transformed into electrocompetent TOP10 *E. coli* and grown on ampicillin (100 µg/ml) selection plates. Miniprep plasmid preparations were made from four colonies. The orientation of the marker was confirmed by sequencing the clones with primers listed in Table 13. A clone with the marker in inverted direction was chosen to be the final vector pTTv324.

**Table 13. List of primers used for sequencing vectors pTTv324.**

| Primer | Sequence |
|---|---|
| T027_Pyr4_orf_start_rev | TGCGTCGCCGTCTCGCTCCT (SEQ ID NO:77) |
| T060_pyr4_orf_screen_1F | TGACGTACCAGTTGGGATGA (SEQ ID NO:78) |

A *pyr4-* strain of the *Leishmania major* STT3 expressing strain M420 was generated by looping out the *pyr4* marker by 5-FOA selection as described in the International Patent Application No. PCT/EP2013/050126. One *pyr4-* strains was designated with number M602.

To prepare the vectors for transformation, the pTTv324 vector was cut with *Pme*I to release the deletion cassette. The fragments were separated with agarose gel electrophoresis and the correct fragment was isolated from the gel with a gel extraction kit (Qiagen) according to manufacturer's protocol. The purified deletion cassette DNA was then transformed into protoplasts of the *Trichoderma reesei* M317 and M602. Preparation of protoplasts, transformation, and protoplast plating were carried out as described above.

Transformants were then streaked onto TrMM plates with 0.1% TritonX-100. Transformants growing fast as selective streaks were screened by PCR using the primers listed in Table 14. DNA from mycelia was purified and analyzed by PCR to look at the integration of the 5' and 3' flanks of cassette and the existence of the *alg3* ORF. The cassette was targeted into the *alg3* locus; therefore the open reading frame was not present in the positively integrated transformants, purified to single cell clones. To screen for 5' integration, sequence outside of the 5' integration flank was used to create a forward primer that would amplify genomic DNA flanking *alg3* and the reverse primer was made from sequence in the *pyr4* marker of the cassette. To check for proper integration of the cassette in the 3' flank, a reverse primer was made from sequence outside of the 3' integration flank that would amplify genomic DNA flanking *alg3* and the forward primer was made from sequence in the *pyr4* marker. Thus, one primer would amplify sequence from genomic DNA outside of the cassette and the other would amplify sequence from DNA in the cassette.

**Table 14: List of primers used for PCR screening of T. reesei transformants.**

| 5' flank screening primers: | 1455 bp product |
|---|---|
| T066_104121_5int | GATGTTGCGCCTGGGTTGAC (SEQ ID NO:79) |
| T060_pyr4_orf_screen_1F | TGACGTACCAGTTGGGATGA (SEQ ID NO:80) |

| 3' flank screening primers: | 1433 bp product |
|---|---|
| T027_Pyr4_orf_start_rev | TGCGTCGCCGTCTCGCTCCT (SEQ ID NO:81) |
| T068_104121_3int | GATTGTCATGGTGTACGTGA (SEQ ID NO:82) |
| alg3 ORF primers: | 689 bp product |
| T767_alg3_del_F | CAAGATGGAGGGCGGCACAG (SEQ ID NO:83) |
| T768_alg3_del_R | GCCAGTAGCGTGATAGAGAAGC (SEQ ID NO:84) |

Two M602 strains and seven M317 strains showing proper integration and a deletion of *alg3* ORF were grown in large shake flasks in TrMM medium supplemented with 40 g/l lactose, 20 g/l spent grain extract, 9 g/l casamino acids and 100 mM PIPPS, pH 5.5 (Table 15). The M317 strain 19.13 and 19.20 were designated the numbers M697 and M698, respectively, and the M602 strains 1.22 and 11.18 were designated the numbers M699 and M700, respectively.

**Table 15 Cell dry weight measurements (in g/l) of the parental strains M304 and STT3 expressing strain M420 and alg3 deletion transformants.**

| Strain | 3 days | 5 days | 7 days |
|---|---|---|---|
| M602 1.22 | 3,63 | 3,23 | 3,79 |
| M602 11.18 | 3,52 | 3,74 | 4,12 |
| M317 19.1 | 3,64 | 3,84 | 4,22 |
| M317 19.5 | 3,54 | 3,87 | 4,31 |
| M317 19.6 | 3,72 | 3,66 | 4,78 |
| M317 19.13 | 3,63 | 3,21 | 4,06 |
| M317 19.20 | 3,97 | 4,28 | 5,09 |
| M317 19.43 | 3,77 | 4,02 | 4,18 |
| M317 19.44 | 3,58 | 3,78 | 4,17 |
| M420 | 3,31 | 3,69 | 5,57 |
| M304 | 2,55 | 2,99 | 4,09 |

### Site occupancy and glycan analyses

Two transformants from overexpression of STT3 from *Leishmania major* in *alg3* deletion strain [pTTv324; 1.22 (M699) and 11.18 (M700)] and seven transformants with *alg3* deletion [M317, *pyr4-*of M304; clones 19.1, 19.5, 19.6, 19.13 (M697), 19.20 (M698), 19.43 and 19.44], and their parental strains M420 and M304 were cultivated in shake flasks in TrMM, 4% lactose, 2% spent grain extract, 0.9% casamino acids, 100 mM PIPPS, pH 5.5. MAB01 antibody was purified and analysed from culture supernatants from day 5 as described in Example 1 except that 30 µg of antibody was digested with 80.4 U of FabRICATOR (Genovis), +37°C, overnight, to produce F(ab')2 and Fc fragments.

In both clones with *alg3* deletion and overexpression of LmSTT3 the site occupancy was 100% (Table 16). Without LmSTT3 the site coverage varied between 56 - 71% in *alg3* deletion clones. The improved site occupancy was shown also in parental strain M420 compared to M304, both with wild type glycosylation.

**Table 16: The site occupancy of the shake flask samples. The analysis failed in M317 clones 19.5 and 19.6.**

| **Strain** | **Clone** | **Explanation** | **Site occupancy %** |
|---|---|---|---|
| M602 | 1.22 | M304 LmSTT3 Δalg3 | 100 |
| M602 | 11.18 | M304 LmSTT3 Δalg3 | 100 |
| M317 | 19.1 | M304 Δalg3 | 71 |
| M317 | 19.13 | M304 Δalg3 | 62 |
| M317 | 19.2 | M304 Δalg3 | 56 |
| M317 | 19.43 | M304 Δalg3 | 63 |
| M317 | 19.44 | M304 Δalg3 | 60 |
| M420 | | Parental strain M304 LmSTT3 | 100 |
| M304 | | Parental strain | 89 |

For N-glycan analysis MAB01 was purified from day 7 culture supernatants as described above and N-glycans were released from EtOH precipitated and SDS denatured antibody using PNGase F (Prozyme) in 20 mM sodium phosphate buffer, pH 7.3, in overnight reaction at +37ºC. The released N-glycans were purified with Hypersep C18 and Hypersep Hypercarb (Thermo Scientific) and analysed with MALDI-TOF MS.

Man3 levels were in range of 21 to 49% whereas the main glycoform in clones of M602 and M317 was Hex6 (Table 17). Man5 levels were about 73% in the strains expressing wild type glycosylation (M304) and LmSTT3 (M420).

**Table 17. Relative proportions of neutral N-glycans from purified antibody from M602 and M317 clones and parental strains M420 and M304.**

| | | | **M602** | | **M317** | | | | | **Parental strains** | |
|---|---|---|---|---|---|---|---|---|---|---|---|
| | | | **1.22** | **11.18** | **19.1** | **19.13** | **19.2** | **19.43** | **19.44** | **M420** | **M304** |
| **Composition** | **Short** | **m\z** | **%** | **%** | **%** | **%** | **%** | **%** | **%** | **%** | **%** |
| Hex3HexNAc2 | Man3 | 933.3 | 21.1 | 27.3 | 45.4 | 37.5 | 34.9 | 24.6 | 48.6 | 0.0 | 0.0 |
| Hex4HexNAc2 | Man4 | 1095.4 | 9.5 | 8.7 | 6.2 | 7.6 | 7.1 | 7.5 | 9.4 | 0.8 | 0.0 |
| Hex5HexNAc2 | Man5 | 1257.4 | 5.8 | 7.0 | 8.1 | 7.6 | 6.7 | 5.6 | 6.6 | 72.5 | 72.8 |
| Hex6HexNAc2 | Man6/Hex6 | 1419.5 | 63.1 | 56.6 | 39.7 | 45.8 | 51.4 | 61.8 | 34.6 | 15.6 | 16.4 |
| Hex7HexNAc2 | Man7/Hex7 | 1581.5 | 0.5 | 0.5 | 0.6 | 0.8 | 0.0 | 0.5 | 0.7 | 7.2 | 7.9 |
| Hex8HexNAc2 | Man8/Hex8 | 1743.6 | 0.0 | 0.0 | 0.0 | 0.6 | 0.0 | 0.0 | 0.0 | 3.2 | 2.4 |
| Hex9HexNAc2 | Man9/Hex9 | 1905.6 | 0.0 | 0.0 | 0.0 | 0.0 | 0.0 | 0.0 | 0.0 | 0.7 | 0.5 |

### Fermentation and site occupancy

*L. major* STT3 *alg3* deletion strain M699 (pTTv324; clone 1.22) and strain M698 with *alg3* deletion [M317, *pyr4-* of M304; clone 19.20], and the parental strain M304 were fermented in 2% YE, 4% cellulose, 8% cellobiose, 4% sorbose. The samples were harvested on day 3, 4, 5 and 6. MAB01 antibody was purified and analysed from culture supernatants from day 5 as described in Example 1 except that 30 µg of antibody was digested with 80.4 U of FabRICATOR (Genovis), +37°C, overnight, to produce F(ab')2 and Fc fragments.

### Results

In the strain M699 site occupancy was more than 90% in all time points (Table 18). Without LmSTT3 the site coverage varied between 29-37% in the strain M698. In the parental strain M304 the site coverage varied between 45-57%. At day 6 MAB01 titers were 1.2 and 1.3 g/L for strains M699 and M698, respectively, and 1.8 g/L in the parental strain M304.

**Table 18. MAB01 antibody titers and site occupancy analysis results of fermented strains M699 and M698 and the parental strain M304.**

| **M699** | **d3** | **d4** | **d5** | **d6** |
|---|---|---|---|---|
| **Titer g/l** | 0.206 | 0.361 | 0.685 | 1.22 |
| **Glycosylation state** | **%** | **%** | **%** | **%** |
| Non-glycosylated | 2.4 | 6.8 | 8.0 | 8.5 |
| Glycosylated | 97.6 | 93.2 | 92.0 | 91.5 |
| Fc + Gn | 0.0 | 0.0 | 0.0 | 0.0 |
| | | | | |

| **M698** | **d3** | **d4** | **d5** | **d6** |
|---|---|---|---|---|
| **Titer g/l** | 0.252 | 0.423 | 0.8 | 1.317 |
| **Glycosylation state** | **%** | **%** | **%** | **%** |
| Non-glycosylated | 63.0 | 70.8 | 64.3 | 65.8 |
| Glycosylated | 37.0 | 29.2 | 35.7 | 34.2 |
| Fc + Gn | 0.0 | 0.0 | 0.0 | 0.0 |
| | | | | |

| **M304** | **d3** | **d4** | **d5** | **d6** |
|---|---|---|---|---|
| **Titer g/l** | 0.589 | 0.964 | 1.41 | 1.79 |
| **Glycosylation state** | **%** | **%** | **%** | **%** |
| Non-glycosylated | 45.9 | 43.3 | n.d. | 54.9 |
| Glycosylated | 54.1 | 56.7 | n.d. | 45.1 |
| Fc + Gn | 0.0 | 0.0 | n.d. | 0.0 |

In conclusion, overexpression of the catalytic subunit of *Leishmania* STT3 is capable of increasing the N-glycosylation site occupancy in Δalg3 filamentous fungal cells up to 91.5- 100%.

Table 19 below recapitulates the different strains used in the Examples:

| **Database** | **Vector** | **Clone** | **Strain transformed** | **Locus, random or K/o** | **Proteases k/o** | **Description** | **Selection of tr.** | **Markers in strain** |
|---|---|---|---|---|---|---|---|---|
| M44 | | | | | None | Base strain | | None |
| M124 | | | | K/o mus53 | None | mus53 deletion of M44 | | pyr4 |
| M127 | | | | pyr4-of M124 | None | pyr4 negative strain of M124 | | pyr4- |
| M181 | pTTv71 | 9-20A-1 | M127 | K/o pep1 | pep1 | pep1 deletion | pyr4 | pyr4 |
| M194 | pTTv42 | 13-172D | M181 | K/o tsp1 | pep1 tsp1 | pep1 tsp1 deletion | bar | bar/pyr4 |
| M252 | pTTv99/67 | 6.14A | M194 | cbh1 egl1 loci | pep1 tsp1 | MAB01 LC NVISKR/HC AXE1 | AmdS/HvqR | AmdS/HygR/bar/pyr4 |
| M284 | 5-FOA of M252 | 3A | pyr4-of M252 | Spontaneous mutation | pep1 tsp1 | pyr4 negative strain of M252 | none | AmdS/Hyg R/bar/pyr4- |
| M304 | pTTv128 | 12A | M284 | K/o slp1, Kex2 o/e | pep1 tsp1 slp1 | Overexpression of native Kex2, slp1 del | pyr4 | AmdS/HyaR/bar/pyr4 |
| M317 | 5-FOA of M304 | 1A | pyr4-of M304 | pyr4 loopout | pep1 tsp1 slp1 | pyr4 negative strain of M304 | None | AmdS/Hyg R/bar/pyr4- |
| M420 | pTTv201 | 17A-a | M317 | xylanase 1 | pep1 tsp1 slp1 | *Leishmania major* stt3 Oligosaccharyl transferase | pyr4 | AmdS/HygR/bar/pyr4 |
| M421 | pTTv201 | 26B-a | M317 | xylanase 1 | pep1 tsp1 slp1 | *Leishmania major* stt3 Oligosaccharyl transferase | pyr4 | AmdS/Hyg R/bar/pyr4 |
| M422 | pTTv201 | 65B-a | M317 | xylanase 1 | pep1 tsp1 slp1 | *Leishmania major* stt3 Oligosaccharyl transferase | pyr4 | AmdS/Hyg R/bar/pyr4 |
| M423 | pTTv201 | 97A-a | M317 | xylanase 1 | pep1 tsp1 slp1 | *Leishmania major* stt3 Oligosaccharyl transferase | pyr4 | AmdS/HygR/bar/pyr4 |
| M602 | 5-FOA of M420 | 2A | pyr4-of M420 | pyr4 loopout | pep1 tsp1 slp1 | pyr4 negative strain of M420 | none | AmdS/HygR/bar/pyr4- |
| M698 | pTTv324 | 19.20 | M317 | alg3 | pep1 tsp1 slp1 | Deletion of alg3 | pyr4 | AmdS/Hyg R/bar/pyr4 |
| M699 | pTTv324 | 1.22 | M602 | alg3 | pep1 tsp1 slp1 | Deletion of alg3 | pyr4 | AmdS/HygR/bar/pyr4 |
| M800 | pTTv322 | 60-6 | M317 | alg3 | pep1 tsp1 slp1 | Leishmania infantum STT3, cDNA1p cbh1t | pyr4 | AmdS/Hyg R/bar/pyr4 |
| M801 | pTTv322 | 60-12 | M317 | alg3 | pep1 tsp1 slp1 | Leishmania infantum STT3, cDNA1p cbh1t | pyr4 | AmdS/HygR/bar/pyr4 |
| M802 | pTTv322 | 60-14 | M317 | alg3 | pep1 tsp1 slp1 | Leishmania infantum STT3, cDNA1p cbh1t | pyr4 | AmdS/Hyg R/bar/pyr4 |

| | | | | | | | | |
|---|---|---|---|---|---|---|---|---|
| *Trichoderma* strains having STT3 (M420-M423) are triple protease deficient (pep1, tsp1, slp1) as well as deficient of xylanase1, cbh1, and egl1. Embodiments include also higher order protease deficient strains. | | | | | | | | |

## Claims

1. A Trichoderma or Myceliophthora cell comprising
i. one or more mutations that reduces or eliminates one or more endogenous protease activity compared to a parental Trichoderma or Myceliophthora cell which does not have said mutation(s),
ii. a polynucleotide encoding a heterologous catalytic subunit of oligosaccharyl transferase, and
iii. a polynucleotide encoding a heterologous glycoprotein,
wherein said catalytic subunit of oligosaccharyl transferase is selected from *Leishmania* oligosaccharyl transferase catalytic subunits, wherein N-glycosylation site occupancy of the heterologous glycoprotein expressed in the Trichoderma or Myceliophthora cell is at least 90%, and wherein said Trichoderma or Myceliophthora cell has at least a two-fold reduction in total protease activity compared to a parental Trichoderma or Myceliophthora cell which does not have the protease activity reducing or eliminating mutation(s) and wherein the heterologous glycoprotein is a mammalian glycoprotein and wherein the yield of the mammalian glycoprotein is at least 1 gram per liter.

2. The Trichoderma or Myceliophthora cell of claim 1, wherein said polynucleotide encoding the heterologous catalytic subunit of oligosaccharyltransferase comprises a nucleic acid selected from the group consisting of SEQ ID NO: 2, SEQ ID NO: 9, SEQ ID NO: 88 and SEQ ID NO: 90 or a polynucleotide encoding a functional variant polypeptide having at least 50%, at least 60%, at least 70% identity, at least 80% identity, at least 90% identity, or at least 95% identity with SEQ ID NO: 1, SEQ ID NO: 8, SEQ ID NO: 89 or SEQ ID NO:91, said functional variant polypeptide having oligosaccharyltransferase activity.

3. The Trichoderma or Myceliophthora cell of anyone of the preceding claims, wherein the N-glycosylation site occupancy of the heterologous glycoprotein is at least 95% and Man3, Man5, G0, G1 and/or G2 glycoforms represent at least 50% of total neutral N-glycans of the heterologous glycoprotein.

4. The Trichoderma or Myceliophthora cell of any one of the preceding claims, wherein said cell is, for example, *Trichoderma reesei,* and said cell comprises mutations that reduce or eliminate the activity of
• the three endogenous proteases pep1, tsp1, and slp1;
• the three endogenous proteases gap1, slp1, and pep1;
• the three endogenous proteases selected from the group consisting of pep1, pep2, pep3, pep4, pep5, pep8, pep9, pep11, pep12, tsp1, slp1, slp2, slp3, slp7, gap1 and gap2;
• three to six proteases selected from the group consisting of pep1, pep2, pep3, pep4, pep5, tsp1, slp1, slp2, slp3, gap1 and gap2; or,
• seven to ten proteases selected from the group consisting of pep1, pep2, pep3, pep4, pep5, pep7, pep8, tsp1, slp1, slp2, slp3, slp5, slp6, slp7, slp8, tpp1, gap1 and gap2.

5. The Trichoderma or Myceliophtora cell of anyone of the preceding claims, wherein the Trichoderma cell further comprises a mutation in the gene encoding ALG3 that reduces or eliminates the corresponding ALG3 expression compared to the level of expression of ALG3 gene in a parental cell which does not have such mutation.

6. The Trichoderma or Myceliophthora cell of any one of the preceding claims, further comprising a polynucleotide encoding an N-acetylglucosaminyltransferase I catalytic domain and an N-acetylglucosaminyltransferase II catalytic domain.

7. The Trichoderma or Myceliophthora cell any one of the preceding claims, further comprising one or more polynucleotides encoding a polypeptide selected from the group consisting of:
i. α1, 2 mannosidase;
ii. N-acetylglucosaminyltransferase I catalytic domain;
iii. α-mannosidase II;
iv. N-acetylglucosaminyltransferase II catalytic domain;
v. β1,4 galactosyltransferase; and,
vi. fucosyltransferase.

8. A method of producing a heterologous glycoprotein with increased N-glycosylation site occupancy, comprising
a) providing a Trichoderma or Myceliophthora cell, having a *Leishmania* STT3D gene encoding a catalytic subunit of oligosaccharyl transferase, and a polynucleotide encoding said heterologous glycoprotein,
b) culturing the cell under appropriate conditions for expression of the STT3D gene, and the production of the heterologous glycoprotein; and,
c) recovering and, optionally, purifying the heterologous glycoprotein,
wherein said Trichoderma or Myceliophthora cell comprises one or more mutations that reduces or eliminates one or more endogenous protease activity compared to a parental Trichoderma or Myceliophthora cell which does not have said mutation(s), and wherein N-glycosylation site occupancy of the heterologous glycoprotein expressed in the Trichoderma or Myceliophthora cell is at least 90% and wherein the heterologous glycoprotein is a mammalian glycoprotein and wherein the yield of the mammalian glycoprotein is at least 1 gram per liter.

9. The method of claim 8, wherein said glycoprotein is an antibody.

10. The method of any one of claims 8 to 9, wherein said Trichoderma or Myceliophthora host cell is the cell as defined in any of claims 1-7.

11. The method of any one of claims 8 to 10, wherein said *Leishmania* STT3D gene encoding a catalytic subunit of oligosaccharyl transferase comprises a nucleic acid sequence selected from the group consisting of SEQ ID NO:2, SEQ ID NO:9, SEQ ID NO: 88 and SEQ ID NO: 90, or a polynucleotide encoding a functional variant polypeptide having at least 50%, at least 60%, at least 70% identity, at least 80% identity, at least 90% identity, or at least 95% identity with SEQ ID NO:1, SEQ ID NO:8, SEQ ID NO: 89 or SEQ ID NO: 91, said functional variant polypeptide having oligosaccharyltransferase activity.

## Patentansprüche

1. Trichoderma- oder Myceliophtora-Zelle, umfassend
i. eine oder mehrere Mutationen, durch die eine oder mehrere endogene Protease-Aktivität verringert oder beseitigt wird, verglichen mit einer Trichoderma- oder Myceliophtora-Elternzelle, die die Mutation(en) nicht aufweist,
ii. ein Polynukleotid, das eine heterologe katalytische Untereinheit von Oligosaccharyltransferase codiert, und
iii. ein Polynukleotid, das ein heterologes Glykoprotein codiert,
wobei die katalytische Untereinheit von Oligosaccharyltransferase aus katalytischen Untereinheiten von *Leishmania*-Oligosaccharyltransferase ausgewählt ist, wobei die N-Glykosylierungsstellenbelegung des in der Trichoderma- oder Myceliophtora-Zelle exprimierten heterologen Glykoproteins bei wenigstens 90% liegt und wobei die Trichoderma- oder Myceliophtora-Zelle wenigstens eine zweifache Verringerung der Protease-Gesamtaktivität verglichen mit einer Trichoderma- oder Myceliophtora-Elternzelle, die die Protease-Aktivität verringernde(n) oder beseitigend(e) Mutation(en) nicht aufweist, aufweist und wobei es sich bei dem heterologen Glykoprotein um ein Säuger-Glykoprotein handelt und wobei die Ausbeute des Säuger-Glykoprotein wenigstens 1 Gramm pro Liter beträgt.

2. Trichoderma- oder Myceliophtora-Zelle nach Anspruch 1, wobei das Polynukleotid, das die heterologe katalytische Untereinheit von Oligosaccharyltransferase codiert, eine Nukleinsäure, die aus der Gruppe bestehend aus SEQ ID NO: 2, SEQ ID NO: 9, SEQ ID NO: 88 und SEQ ID NO: 90 ausgewählt ist, oder ein Polynukleotid, das eine funktionelle Polypeptidvariante mit wenigstens 50%, wenigstens 60%, wenigstens 70% Identität, wenigstens 80% Identität, wenigstens 90% Identität oder wenigstens 95% Identität mit SEQ ID NO: 1, SEQ ID NO: 8, SEQ ID NO: 89 oder SEQ ID NO: 91 codiert, wobei die funktionelle Polypeptidvariante Oligosaccharyltransferase-Aktivität aufweist, umfasst.

3. Trichoderma- oder Myceliophtora-Zelle nach einem der vorhergehenden Ansprüche, wobei die N-Glykosylierungsstellenbelegung des heterologen Glykoproteins bei wenigstens 95% liegt und Man3-, Man5-, G0-, G1- und/oder G2-Glykoformen wenigstens 50% der gesamten neutralen N-Glykane des heterologen Glykoproteins repräsentieren.

4. Trichoderma- oder Myceliophtora-Zelle nach einem der vorhergehenden Ansprüche, wobei es sich bei der Zelle z. B. um *Trichoderma reesei* handelt und die Zelle Mutationen umfasst, durch die die Aktivität von
• den drei endogenen Proteasen pep1, tsp1 und slpl;
• den drei endogenen Proteasen gap1, slp1 und pep1;
• den drei endogenen Proteasen, die aus der Gruppe bestehend aus pep1, pep2, pep3, pep4, pep5, pep8, pep9, pep11, pep12, tspl, slp1, slp2, slp3, slp7, gap1 und gap2 ausgewählt sind;
• drei bis sechs Proteasen, die aus der Gruppe bestehend aus pep1, pep2, pep3, pep4, pep5, tspl, slp1, slp2, slp3, gap1 und gap2 ausgewählt sind; oder
• sieben bis zehn Proteasen, die aus der Gruppe bestehend aus pep1, pep2, pep3, pep4, pep5, pep7, pep8, tspl, slp1, slp2, slp3, slp5, slp6, slp7, slp8, tpp1, gap1 und gap2 ausgewählt sind,
verringert oder beseitigt wird.

5. Trichoderma- oder Myceliophtora-Zelle nach einem der vorhergehenden Ansprüche, wobei die Trichoderma-Zelle ferner eine Mutation im ALG3 codierenden Gen umfasst, durch die die entsprechende ALG3-Expression verglichen mit dem Expressionsniveau des ALG3-Gens in einer Elternzelle, die keine solche Mutation aufweist, verringert oder beseitigt wird.

6. Trichoderma- oder Myceliophtora-Zelle nach einem der vorhergehenden Ansprüche, ferner umfassend ein Polynukleotid, das eine katalytische Domäne von N-Acetylglucosaminyltransferase I und eine katalytische Domäne von N-Acetylglucosaminyltransferase II codiert.

7. Trichoderma- oder Myceliophtora-Zelle nach einem der vorhergehenden Ansprüche, ferner umfassend ein oder mehrere Polynukleotide, die ein Polypeptid codieren, das ausgewählt ist aus der Gruppe bestehend aus:
i. α1,2-Mannosidase;
ii. N-Acetylglucosaminyltransferase I, katalytische Domäne;
iii. α-Mannosidase II;
iv. N-Acetylglucosaminyltransferase II, katalytische Domäne;
v. β1,4-Galactosyltransferase; und
vi. Fucosyltransferase.

8. Verfahren zur Herstellung eines heterologen Glykoproteins mit erhöhter N-Glykosylierungsstellenbelegung, umfassend
a) Bereitstellen einer Trichoderma- oder Myceliophtora-Zelle mit einem *Leishmania-*STT3D-Gen, das eine katalytische Untereinheit von Oligosaccharyltransferase codiert, und einem Polynukleotid, das das heterologe Glykoprotein codiert,
b) Kultivieren der Zelle unter geeigneten Bedingungen für Expression des STT3D-Gens und die Herstellung des heterologen Glykoproteins; und
c) Gewinnen und gegebenenfalls Aufreinigen des heterologen Glykoproteins,
wobei die Trichoderma- oder Myceliophtora-Zelle eine oder mehrere Mutationen umfasst, durch die eine oder mehrere endogene Protease-Aktivität verringert oder beseitigt wird, verglichen mit einer Trichoderma- oder Myceliophtora-Elternzelle, die die Mutation(en) nicht aufweist, und wobei die N-Glykosylierungsstellenbelegung des in der Trichoderma- oder Myceliophtora-Zelle exprimierten heterologen Glykoproteins bei wenigstens 90% liegt und wobei es sich bei dem heterologen Glykoprotein um ein Säuger-Glykoprotein handelt und wobei die Ausbeute des Säuger-Glykoprotein wenigstens 1 Gramm pro Liter beträgt.

9. Verfahren nach Anspruch 8, wobei es sich bei dem Glykoprotein um einen Antikörper handelt.

10. Verfahren nach einem der Ansprüche 8 bis 9, wobei es sich bei der Trichoderma- oder Myceliophtora-Wirtszelle um die Zelle mit der in einem der Ansprüche 1-7 angegebenen Bedeutung handelt.

11. Verfahren nach einem der Ansprüche 8 bis 10, wobei das *Leishmania*-STT3D-Gen, das eine katalytische Untereinheit von Oligosaccharyltransferase codiert, eine Nukleinsäuresequenz, die aus der Gruppe bestehend aus SEQ ID NO: 2, SEQ ID NO: 9, SEQ ID NO: 88 und SEQ ID NO: 90 ausgewählt ist, oder ein Polynukleotid, das eine funktionelle Polypeptidvariante mit wenigstens 50%, wenigstens 60%, wenigstens 70% Identität, wenigstens 80% Identität, wenigstens 90% Identität oder wenigstens 95% Identität mit SEQ ID NO: 1, SEQ ID NO: 8, SEQ ID NO: 89 oder SEQ ID NO: 91 codiert, wobei die funktionelle Polypeptidvariante Oligosaccharyltransferase-Aktivität aufweist, umfasst.

## Revendications

1. Cellule de Trichoderma ou de Myceliophthora comprenant
i. une ou plusieurs mutations qui réduisent ou éliminent une ou plusieurs activités protéases endogènes en comparaison avec une cellule parentale de Trichoderma ou de Myceliophthora qui ne présente pas ladite ou lesdites mutations,
ii. un polynucléotide codant pour une sous-unité catalytique hétérologue d'oligosaccharyltransférase, et
iii. un polynucléotide codant pour une glycoprotéine hétérologue,
dans laquelle ladite sous-unité catalytique d'oligosaccharyltransférase est sélectionnée parmi des sous-unités catalytiques d'oligosaccharyltransférase de *Leishmania,* dans laquelle un taux d'occupation des sites de N-glycosylation de la glycoprotéine hétérologue exprimée dans la cellule de Trichoderma ou de Myceliophthora est d'au moins 90 %, et dans laquelle ladite cellule de Trichoderma ou de Myceliophthora présente au moins une réduction par deux de l'activité protéase totale en comparaison avec une cellule parentale de Trichoderma ou de Myceliophthora qui ne présente pas la ou les mutations réduisant ou éliminant une activité protéase et dans laquelle la glycoprotéine hétérologue est une glycoprotéine mammifère et dans laquelle le rendement de la glycoprotéine mammifère est d'au moins 1 gramme par litre.

2. Cellule de Trichoderma ou de Myceliophthora selon la revendication 1, dans laquelle ledit polynucléotide codant pour la sous-unité catalytique hétérologue d'oligosaccharyltransférase comprend un acide nucléique sélectionné parmi le groupe constitué de SEQ ID N° : 2, SEQ ID N° : 9, SEQ ID N° : 88 et SEQ ID N° : 90 ou un polynucléotide codant pour un variant polypeptidique fonctionnel présentant une identité d'au moins 50 %, d'au moins 60 %, d'au moins 70 %, une identité d'au moins 80 %, une identité d'au moins 90 %, ou une identité d'au moins 95 % avec SEQ ID N° : 1, SEQ ID N° : 8, SEQ ID N° : 89 ou SEQ ID N° : 91, ledit variant polypeptidique fonctionnel présentant une activité oligosaccharyltransférase.

3. Cellule de Trichoderma ou de Myceliophthora selon l'une quelconque des revendications précédentes, dans laquelle le taux d'occupation des sites de N-glycosylation de la glycoprotéine hétérologue est d'au moins 95 % et les glycoformes Man3, Man5, G0, G1 et/ou G2 représentent au moins 50 % de la totalité des N-glycanes neutres de la glycoprotéine hétérologue.

4. Cellule de Trichoderma ou de Myceliophthora selon l'une quelconque des revendications précédentes, dans laquelle ladite cellule est, par exemple, *Trichoderma reesei,* et ladite cellule comprend des mutations qui réduisent ou éliminent l'activité
- des trois protéases endogènes pep1, tsp1, et slp1 ;
- des trois protéases endogènes gap1, slp1, et pep1 ;
- des trois protéases endogènes sélectionnées parmi le groupe constitué de pep1, pep2, pep3, pep4, pep5, pep8, pep9, pep11, pep12, tsp1, slp1, slp2, slp3, slp7, gap1 et gap2 ;
- de trois à six protéases sélectionnées parmi le groupe constitué de pep1, pep2, pep3, pep4, pep5, tsp1, slp1, slp2, slp3, gap1 et gap2 ; ou,
- de sept à dix protéases sélectionnées parmi le groupe constitué de pep1, pep2, pep3, pep4, pep5, pep7, pep8, tsp1, slp1, slp2, slp3, slp5, slp6, slp7, slp8, tpp1, gap1 et gap2.

5. Cellule de Trichoderma ou de Myceliophthora selon l'une quelconque des revendications précédentes, dans laquelle la cellule de Trichoderma comprend en outre une mutation dans le gène codant pour ALG3 qui réduit ou élimine l'expression de ALG3 correspondante en comparaison avec le niveau d'expression d'un gène ALG3 dans une cellule parente qui ne présente pas une telle mutation.

6. Cellule de Trichoderma ou de Myceliophthora selon l'une quelconque des revendications précédentes, comprenant en outre un polynucléotide codant pour un domaine catalytique de N-acétylglucosaminyltransférase I et un domaine catalytique de N-acétylglucosaminyltransférase II.

7. Cellule de Trichoderma ou de Myceliophthora selon l'une quelconque des revendications précédentes, comprenant en outre un ou plusieurs polynucléotides codant pour un polypeptide sélectionné parmi le groupe constitué de :
i. l'α1,2 mannosidase ;
ii. du domaine catalytique de N-acétylglucosaminyltransférase I ;
iii. de l'α-mannosidase II ;
iv. du domaine catalytique de N-acétylglucosaminyltransférase II ;
v. de la β1,4 galactosyltransférase ; et
vi. de la fucosyltransférase.

8. Procédé de production d'une protéine hétérologue avec un taux d'occupation des sites de N-glycosylation accru, comprenant
a) la fourniture d'une cellule de Trichoderma ou de Myceliophthora, présentant un gène STT3D de *Leishmania* codant pour une sous-unité catalytique d'oligosaccharyltransférase, et un polynucléotide codant pour ladite glycoprotéine hétérologue,
b) la mise en culture de la cellule dans des conditions appropriées pour une expression du gène STT3D, et la production de la glycoprotéine hétérologue ; et
c) la récupération et, éventuellement, une purification de la glycoprotéine hétérologue,
dans lequel ladite cellule de Trichoderma ou de Myceliophthora comprend une ou plusieurs mutations qui réduisent ou éliminent une ou plusieurs activités de protéase endogène en comparaison avec une cellule parentale de Trichoderma ou de Myceliophthora qui ne présente pas ladite ou lesdites mutations, et dans lequel un taux d'occupation des sites de N-glycosylation de la glycoprotéine hétérologue exprimée dans la cellule de Trichoderma ou de Myceliophthora est d'au moins 90 % et dans lequel la glycoprotéine hétérologue est une glycoprotéine mammifère et dans lequel le rendement de la glycoprotéine mammifère est d'au moins 1 gramme par litre.

9. Procédé selon la revendication 8, dans lequel ladite glycoprotéine est un anticorps.

10. Procédé selon l'une quelconque des revendications 8 et 9, dans lequel ladite cellule hôte de Trichoderma ou de Myceliophthora est la cellule telle que définie selon l'une quelconque des revendications 1 à 7.

11. Procédé selon l'une quelconque des revendications 8 à 10, dans lequel ledit gène STT3D de *Leishmania* codant pour une sous-unité catalytique d'oligosaccharyltransférase comprend un acide nucléique sélectionné parmi le groupe constitué de SEQ ID N° : 2, SEQ ID N° : 9, SEQ ID N° : 88 et SEQ ID N° : 90, ou un polynucléotide codant pour un variant polypeptidique fonctionnel présentant une identité d'au moins 50 %, d'au moins 60 %, d'au moins 70 %, une identité d'au moins 80 %, une identité d'au moins 90 %, ou une identité d'au moins 95 % avec SEQ ID N° : 1, SEQ ID N° : 8, SEQ ID N° : 89 ou SEQ ID N° : 91, ledit variant polypeptidique fonctionnel présentant une activité oligosaccharyltransférase.


### SEQUENCE LISTING

<110> Novartis AG
<120> Production of glycoproteins having increased N-glycosylation site occupancy
<130> NOVA 0005 WO
<160> 91
<170> PatentIn version 3.5
<210> 1
   <211> 857
   <212> PRT
   <213> Leishmania major
<400> 1
   Met Gly Lys Arg Lys Gly Asn Ser Leu Gly Asp Ser Gly Ser Ala Ala
   1               5                   10                  15
   Thr Ala Ser Arg Glu Ala Ser Ala Gln Ala Glu Asp Ala Ala Ser Gln
               20                  25                  30
   Thr Lys Thr Ala Ser Pro Pro Ala Lys Val Ile Leu Leu Pro Lys Thr
           35                  40                  45
   Leu Thr Asp Glu Lys Asp Phe Ile Gly Ile Phe Pro Phe Pro Phe Trp
       50                  55                  60
   Pro Val His Phe Val Leu Thr Val Val Ala Leu Phe Val Leu Ala Ala
   65                  70                  75                  80
   Ser Cys Phe Gln Ala Phe Thr Val Arg Met Ile Ser Val Gln Ile Tyr
                   85                  90                  95
   Gly Tyr Leu Ile His Glu Phe Asp Pro Trp Phe Asn Tyr Arg Ala Ala
               100                 105                 110
   Glu Tyr Met Ser Thr His Gly Trp Ser Ala Phe Phe Ser Trp Phe Asp
           115                 120                 125
   Tyr Met Ser Trp Tyr Pro Leu Gly Arg Pro Val Gly Ser Thr Thr Tyr
       130                 135                 140
   Pro Gly Leu Gln Leu Thr Ala Val Ala Ile His Arg Ala Leu Ala Ala
   145                 150                 155                 160
   Ala Gly Met Pro Met Ser Leu Asn Asn Val Cys Val Leu Met Pro Ala
                   165                 170                 175
   Trp Phe Gly Ala Ile Ala Thr Ala Thr Leu Ala Phe Cys Thr Tyr Glu
   180 185 190
   Ala Ser Gly Ser Thr Val Ala Ala Ala Ala Ala Ala Leu Ser Phe Ser
   195 200 205
   Ile Ile Pro Ala His Leu Met Arg Ser Met Ala Gly Glu Phe Asp Asn
   210 215 220
   Glu Cys Ile Ala Val Ala Ala Met Leu Leu Thr Phe Tyr Cys Trp Val
   225 230 235 240
   Arg Ser Leu Arg Thr Arg Ser Ser Trp Pro Ile Gly Val Leu Thr Gly
   245 250 255
   Val Ala Tyr Gly Tyr Met Ala Ala Ala Trp Gly Gly Tyr Ile Phe Val
   260 265 270
   Leu Asn Met Val Ala Met His Ala Gly Ile Ser Ser Met Val Asp Trp
   275 280 285
   Ala Arg Asn Thr Tyr Asn Pro Ser Leu Leu Arg Ala Tyr Thr Leu Phe
   290 295 300
   Tyr Val Val Gly Thr Ala Ile Ala Val Cys Val Pro Pro Val Gly Met
   305 310 315 320
   Ser Pro Phe Lys Ser Leu Glu Gln Leu Gly Ala Leu Leu Val Leu Val
   325 330 335
   Phe Leu Cys Gly Leu Gln Val Cys Glu Val Leu Arg Ala Arg Ala Gly
   340 345 350
   Val Glu Val Arg Ser Arg Ala Asn Phe Lys Ile Arg Val Arg Val Phe
   355 360 365
   Ser Val Met Ala Gly Val Ala Ala Leu Ala Ile Ser Val Leu Ala Pro
   370 375 380
   Thr Gly Tyr Phe Gly Pro Leu Ser Val Arg Val Arg Ala Leu Phe Val
   385 390 395 400
   Glu His Thr Arg Thr Gly Asn Pro Leu Val Asp Ser Val Ala Glu His
   405 410 415
   Gln Pro Ala Ser Pro Glu Ala Met Trp Ala Phe Leu His Val Cys Gly
   420 425 430
   Val Thr Trp Gly Leu Gly Ser Ile Val Leu Ala Val Ser Thr Phe Val
   435 440 445
   His Tyr Ser Pro Ser Lys Val Phe Trp Leu Leu Asn Ser Gly Ala Val
   450 455 460
   Tyr Tyr Phe Ser Thr Arg Met Ala Arg Leu Leu Leu Leu Ser Gly Pro
   465 470 475 480
   Ala Ala Cys Leu Ser Thr Gly Ile Phe Val Gly Thr Ile Leu Glu Ala
   485 490 495
   Ala Val Gln Leu Ser Phe Trp Asp Ser Asp Ala Thr Lys Ala Lys Lys
   500 505 510
   Gln Gln Lys Gln Ala Gln Arg His Gln Arg Gly Ala Gly Lys Gly Ser
   515 520 525
   Gly Arg Asp Asp Ala Lys Asn Ala Thr Thr Ala Arg Ala Phe Cys Asp
   530 535 540
   Val Phe Ala Gly Ser Ser Leu Ala Trp Gly His Arg Met Val Leu Ser
   545 550 555 560
   Ile Ala Met Trp Ala Leu Val Thr Thr Thr Ala Val Ser Phe Phe Ser
   565 570 575
   Ser Glu Phe Ala Ser His Ser Thr Lys Phe Ala Glu Gln Ser Ser Asn
   580 585 590
   Pro Met Ile Val Phe Ala Ala Val Val Gln Asn Arg Ala Thr Gly Lys
   595 600 605
   Pro Met Asn Leu Leu Val Asp Asp Tyr Leu Lys Ala Tyr Glu Trp Leu
   610 615 620
   Arg Asp Ser Thr Pro Glu Asp Ala Arg Val Leu Ala Trp Trp Asp Tyr
   625 630 635 640
   Gly Tyr Gln Ile Thr Gly Ile Gly Asn Arg Thr Ser Leu Ala Asp Gly
   645 650 655
   Asn Thr Trp Asn His Glu His Ile Ala Thr Ile Gly Lys Met Leu Thr
   660 665 670
   Ser Pro Val Val Glu Ala His Ser Leu Val Arg His Met Ala Asp Tyr
               675                 680                 685
   Val Leu Ile Trp Ala Gly Gln Ser Gly Asp Leu Met Lys Ser Pro His
       690                 695                 700
   Met Ala Arg Ile Gly Asn Ser Val Tyr His Asp Ile Cys Pro Asp Asp
   705                 710                 715                 720
   Pro Leu Cys Gln Gln Phe Gly Phe His Arg Asn Asp Tyr Ser Arg Pro
                   725                 730                 735
   Thr Pro Met Met Arg Ala Ser Leu Leu Tyr Asn Leu His Glu Ala Gly
               740                 745                 750
   Lys Arg Lys Gly Val Lys Val Asn Pro Ser Leu Phe Gln Glu Val Tyr
           755                 760                 765
   Ser Ser Lys Tyr Gly Leu Val Arg Ile Phe Lys Val Met Asn Val Ser
       770                 775                 780
   Ala Glu Ser Lys Lys Trp Val Ala Asp Pro Ala Asn Arg Val Cys His
   785                 790                 795                 800
   Pro Pro Gly Ser Trp Ile Cys Pro Gly Gln Tyr Pro Pro Ala Lys Glu
                   805                 810                 815
   Ile Gln Glu Met Leu Ala His Arg Val Pro Phe Asp Gln Val Thr Asn
               820                 825                 830
   Ala Asp Arg Lys Asn Asn Val Gly Ser Tyr Gln Glu Glu Tyr Met Arg
           835                 840                 845
   Arg Met Arg Glu Ser Glu Asn Arg Arg
       850                 855
<210> 2
   <211> 2575
   <212> DNA
   <213> Leishmania major
<400> 2
   aatgggcaag cgcaagggca acagcctcgg cgacagcggc agcgccgcca ccgcctcacg     60
   agaggcctct gcccaggccg aggacgccgc cagccagacc aagaccgcca gcccccctgc    120
   caaggtcatc ctcctgccca agaccctcac cgacgagaag gacttcatcg gcatcttccc    180
   gttcccgttc tggcccgtcc acttcgtcct caccgtcgtc gccctcttcg tcctcgccgc    240
   cagctgcttc caggccttca ccgtccgcat gatcagcgtc cagatctacg gctacctcat    300
   ccacgagttc gacccctggt tcaactaccg agccgccgag tacatgagca cccacggctg 360
   gtccgccttt ttcagctggt tcgactacat gagctggtat ccgctcggcc gacccgtcgg 420
   cagcaccacc taccccggcc tccagctcac cgccgtggcc atccatcgag ccctcgccgc 480
   tgccggcatg cctatgagcc tcaacaacgt ctgcgtcctc atgcccgcct ggttcggcgc 540
   cattgcgacc gccaccctcg cgttctgcac ctacgaggcc agcggctcta cagtggccgc 600
   tgccgcggct gccctcagct tcagcatcat ccccgcccac ctcatgcgct ccatggccgg 660
   cgagttcgac aacgagtgca ttgccgtcgc cgccatgctc ctcaccttct actgctgggt 720
   ccgcagcctc cgcacgcgca gcagctggcc catcggcgtc ctgaccggcg tcgcctacgg 780
   ctacatggct gccgcctggg gcggctacat cttcgtcctc aacatggtgg ccatgcacgc 840
   cggcatcagc agcatggtcg actgggcccg caacacctac aaccccagcc tgctccgcgc 900
   ctacaccctc ttctacgtcg tcggcaccgc cattgccgtc tgcgtccccc ccgtcggcat 960
   gagccccttc aagagcctcg agcagctcgg cgccctcctc gtcctggtct ttctgtgcgg 1020
   cctccaggtc tgcgaggtcc tccgagcccg agccggcgtc gaggtccgct ctcgcgccaa 1080
   cttcaagatc cgcgtccgcg tctttagcgt catggccggc gtggccgccc tcgccatctc 1140
   tgtcctcgcc cccaccggct acttcggccc cctcagcgtc cgagtgcgcg ccctgttcgt 1200
   cgagcacacc cgcaccggca accccctcgt cgacagcgtc gccgagcacc agcccgccag 1260
   ccccgaggcc atgtgggcct ttctccacgt ctgcggcgtc acctggggcc tcggcagcat 1320
   cgtcctggcc gtcagcacct tcgtccacta cagccccagc aaggtctttt ggctcctcaa 1380
   ctctggcgcc gtctactact tctcgacccg aatggcccgc ctcctcctcc tgtccggccc 1440
   tgccgcctgc ctgagcaccg gcatcttcgt cggcacgatc ctcgaggccg ccgtccagct 1500
   cagcttctgg gacagcgacg ccaccaaggc caagaagcag cagaagcagg cccagcgcca 1560
   ccagcgaggc gctggcaagg gctctggccg cgacgacgcc aagaacgcga cgaccgcccg 1620
   agccttctgc gacgtctttg ccggcagcag cctcgcctgg ggccaccgca tggtcctctc 1680
   gatcgccatg tgggcgctcg tcacgacaac ggccgtcagc ttcttcagca gcgagttcgc 1740
   cagccacagc accaagttcg ccgagcagag cagcaacccc atgatcgtct ttgccgccgt 1800
   cgtccagaac cgcgccaccg gcaagccgat gaacctcctc gtcgacgact acctcaaggc 1860
   ctacgagtgg ctccgcgaca gcacccctga ggacgcccgc gtcctggcct ggtgggacta 1920
   cggctaccag atcaccggca tcggcaaccg caccagcctc gccgacggca acacctggaa 1980
   ccacgagcac attgccacca tcggcaagat gctcaccagc ccggtcgtcg aggcccacag 2040
   cctcgtccgc cacatggccg actacgtcct catctgggct ggccagagcg gcgacctcat 2100
   gaagtccccc cacatggccc gcatcggcaa cagcgtctac cacgacatct gccccgacga 2160
   ccccctctgc cagcagttcg gcttccaccg caacgactac agccgcccca ccccgatgat 2220
   gcgcgccagc ctcctctaca acctccacga ggccggcaag cgaaagggcg tcaaggtcaa 2280
   cccctcgctg ttccaggagg tctacagcag caagtacggc ctggtccgca tcttcaaggt 2340
   catgaacgtc agcgccgaga gcaagaagtg ggtcgccgat cccgccaacc gagtctgcca 2400
   cccccctggc agctggatct gccctggcca gtaccctccc gccaaggaaa tccaggagat 2460
   gctcgcccac cgcgtcccgt tcgaccaggt caccaacgcc gaccgcaaga acaacgtcgg 2520
   cagctaccaa gaggagtaca tgcgccgcat gcgcgagagc gagaaccgcc gctag 2575
<210> 3
   <211> 50
   <212> DNA
   <213> Trichoderma reesei
<400> 3
   accaaagact ttttgatcaa tccaacaact tctctcaact taattaaatc 50
<210> 4
   <211> 48
   <212> DNA
   <213> Aspergillus niger
<400> 4
   ttaattaaga tccacttaac gttactgaaa tcatcaaaca gcttgacg 48
<210> 5
   <211> 1000
   <212> DNA
   <213> Trichoderma reesei
<400> 5
   caagtcttcg tactctatcg aagtctcgcc ttacgtactt gatctgctgt ctttcgtgtc      60
   cggtcaacat atactcgcac acattagccc cagcagaaca tgtcgtcggc ataaaaggcc     120
   aattcagatc gcagataaca aaatgctacc agcatctgtc tagttgtgga gatatgaagg     180
   ggtatttcag gctttctttg tgggaataaa gagagaaaga gagacttaca ggagctctag     240
   gcttcgtagc ccctgcgttc ttagttcgca atgccgtgaa agcagctaca tctaccaaga     300
   cactcgtgca tcgtctattt tatttgttac atgctgggaa tttccgggac attgtttaag     360
   gatgactagg ttcagccgtt aaagaatgga aggccatggc ttgtccctct gtggcaagtc     420
   attgcactcc aaggccttct cctgtactag tcctacaatt ctgcagcaaa tggcctcaag     480
   caactacgta aaactccatg agattgcaga tgcggcccac tggaatacaa catcctccgc     540
   aagtccgaca tgaagcccct tgacttgatt ggcaggctaa atgcgacatc ttagccggat     600
   gcaccccaga tctggggaac gcgccgcttg aggcccgaag cgccgggttc gatgcattac     660
   tgccatattt cagcagttaa ctaggaccgg cttgtgtcga tattgcgggt ggcgttcaat     720
   ctattccggc actcctatgc cgtttgatcc gatacctgga gggcgtgctt taggcaaaat     780
   gccaagcttc gaggatactg tacgagccgc tttcaacctc acttgatgat gtctgagttt     840
   catcaagaga attgaagtca aagctcaaat catgatgtga agaggttttg aatgtggaag     900
   aattctgcat atataaagcc atggaagaag acgtaaaact gagacagcaa gctcaactgc     960
   atagtatcga cttcaaggaa aacacgcaca aataatcatc                          1000
<210> 6
   <211> 1000
   <212> DNA
   <213> Trichoderma reesei
<400> 6
   aggggtttga gctggtatgt agtattgggg tggttagtga gttaacttga cagactgcac        60
   tttggcaaca gagccgacga ttaagagatt gctgtcatgt aactaaagta gcctgccttt       120
   gacgctgtat gctcatgata catgcgtgac atcgaaatat atcagccaaa gtatccgtcc       180
   ggcgacatgc ccatcaacta tattgaagtc agaaacacac tgtccctctt ccctcctatg       240
   cttttacaag ctgctcctct atccgccccc acagtccctt gttcatatac cccgaaagcc       300
   aaaagtttcc atccttgtcc ttgcccatga tcgggaagcc gtttggtagc acgatacccc       360
   actgattatt ctgtatatag atcggtgaac ccgatttccc accctcccta ctgggctgaa       420
   gcacagctgc agaaaagtcc aagtcgaaca gctttgcctt gccccaattt gacaacgtaa       480
   tcatgtgcat gttgccgttg ccgaagaaag gcggaatcct cccgctagat cctcgccaca       540
   tagcgaaaaa ggcttctacc tgagaccgag ttcccagttc ttgaatcgcg gttcgagtag       600
   cagcagcaat ataactcagc ggcttctcaa atatgtggtg caccggcagt agcacgttga       660
   tgaagccggt accgttggag acatatggca cccctttcgg cagcagatcc gtctctagac       720
   actttcgtag agagtatgcg ttgttgatga caaccgtcct ctggctattc gctggcagat       780
   gtgaagtggc aactttgatc caccaggcgc agagaacatc gccttcagtc aagaaagtgt       840
   tttctgcgcc ctcggactca agctcactga ttgcctcttt gcgaaggttc tcaatgaaag       900
   atccaggaac acaaagcatg cgattctctt gcgctcggaa gagatcgagg acattgttga       960
   tcccatactg ggccagccca aacattgaca agcgccgaga                           1000
<210> 7
   <211> 688
   <212> PRT
   <213> Trichomonas vaginalis
<400> 7
   Met Gly Asn Thr Val Lys Val Ile Gln Leu Ile Thr Leu Leu Leu Ser
   1               5                   10                  15
   Cys Leu Leu Ala Phe Leu Ile Arg Gln Phe Ala Asn Val Val Asn Glu
               20                  25                  30
   Pro Ile Ile His Glu Phe Asp Pro His Phe Asn Trp Arg Cys Thr Gln
           35                  40                  45
   Tyr Ile Asp Thr His Gly Leu Tyr Glu Phe Leu Gly Trp Phe Asp Asn
       50                  55                  60
   Ile Ser Trp Tyr Pro Gln Gly Arg Pro Val Gly Glu Thr Ala Tyr Pro
   65                  70                  75                  80
   Gly Leu Met Tyr Thr Ser Ala Ile Val Lys Trp Ala Leu Gln Lys Ile
                   85                  90                  95
   His Ile Ile Val Asp Leu Arg Asn Ile Cys Val Phe Met Gly Pro Ser
               100                 105                 110
   Val Ser Ile Leu Ser Val Leu Val Ala Phe Leu Phe Gly Glu Leu Val
           115                 120                 125
   Gly Ser Ala Gln Leu Gly Thr Leu Phe Gly Ala Ile Thr Ser Phe Ile
       130                 135                 140
   Pro Gly Met Ile Ser Arg Ser Val Gly Gly Ala Tyr Asp Tyr Glu Cys
   145                 150                 155                 160
   Ile Gly Leu Phe Ile Ile Val Leu Ser Leu Tyr Thr Phe Ala Leu Ala
                   165                 170                 175
   Leu Lys Ser Gly Ser Ile Leu Leu Ser Val Ile Ala Ala Phe Ala Tyr
               180                 185                 190
   Ser Tyr Leu Ala Leu Thr Trp Gly Gly Tyr Val Phe Val Ser Asn Cys
           195                 200                 205
   Ile Pro Leu Phe Ala Ala Gly Leu Val Ala Ile Gly Arg Tyr Ser Trp
       210                 215                 220
   Arg Leu His Ile Thr Tyr Ser Ile Trp Phe Ile Val Ala Ser Ile Leu
   225                 230                 235                 240
   Thr Ala Gln Ile Pro Phe Ile Gly Asp Lys Ile Leu Lys Lys Pro Glu
                   245                 250                 255
   His Phe Ala Met Leu Gly Thr Phe Leu Val Met Gln Ile Trp Gly Phe
               260                 265                 270
   Phe Thr Phe Ile Lys Ser Arg Phe Ser Pro Thr Thr Tyr Asn Ser Val
           275                 280                 285
   Ala Ile Thr Ser Ile Leu Ile Leu Pro Ser Phe Leu Leu Leu Met Ile
       290                 295                 300
   Thr Val Gly Met Ser Thr Gly Leu Leu Gly Gly Phe Ser Gly Arg Leu
   305                 310                 315                 320
   Leu Gln Met Phe Asp Pro Thr Tyr Ala Ala Lys Asn Val Pro Ile Ile
                   325                 330                 335
   Asn Ser Val Ala Glu His Gln Pro Thr Ala Trp Val Lys Tyr Tyr Ser
               340                 345                 350
   Asp Cys Glu Leu Phe Ile Phe Phe Phe Pro Leu Gly Ala Tyr Ile Val
           355                 360                 365
   Ile Ser Ser Leu Ile Arg Thr Gln Lys Thr Lys Asp Gln Thr Glu Leu
       370                 375                 380
   Lys Arg Ala Glu Thr Leu Leu Leu Leu Phe Ile Tyr Gly Phe Ser Thr
   385                 390                 395                 400
   Leu Tyr Phe Ala Ser Ile Met Val Arg Leu Val Leu Val Phe Thr Pro
                   405                 410                 415
   Ala Leu Val Phe Val Ala Gly Ile Ala Ile His Gln Leu Leu Arg Glu
               420                 425                 430
   Ser Phe Lys Gln Lys Ser Phe Leu His Pro Val Ser Leu Thr Met Ile
           435                 440                 445
   Ile Leu Thr Phe Ile Ile Cys Leu His Gly Val Leu His Ala Thr His
       450                 455                 460
   Phe Ala Cys Tyr Ser Tyr Ser Gly Asp His Leu His Phe Asn Ile Met
   465                 470                 475                 480
   Thr Pro Arg Gly Val Glu Thr Ser Asp Asp Tyr Arg Glu Gly Tyr Arg
                   485                 490                 495
   Trp Leu Thr Glu Asn Thr Tyr Arg Asp Asp Ile Val Met Ser Trp Trp
               500                 505                 510
   Asp Tyr Gly Tyr Gln Ile Thr Ser Met Gly Asn Arg Gly Cys Ile Ala
           515                 520                 525
   Asp Gly Asn Thr Asn Asn Phe Thr His Ile Gly Ile Ile Gly Met Ala
       530                 535                 540
   Met Ser Ser Pro Glu Pro Ile Ser Trp Arg Ile Ala Arg Leu Met Asn
   545                 550                 555                 560
   Val Lys Tyr Met Leu Val Ile Phe Gly Gly Ala Ala Gln Tyr Ser Gly
                   565                 570                 575
   Asp Asp Ile Asn Lys Phe Leu Trp Met Pro Arg Ile Ala His Gln Thr
               580                 585                 590
   Phe Asp Asn Ile Thr Gly Glu Met Tyr Gln Ile Pro Tyr Arg His Ile
           595                 600                 605
   Val Gly Glu Ser Met Thr Lys Asn Met Thr Leu Ser Met Met Phe Lys
       610                 615                 620
   Phe Cys Tyr Asn Asn Tyr Lys Tyr Tyr Gln Pro His Pro Gln Phe Pro
   625                 630                 635                 640
   Thr Gly Tyr Asp Leu Thr Arg Arg Thr Ser Ile Pro Asn Ile Lys Asp
                   645                 650                 655
   Ile Ser Met Ser Gln Phe Thr Glu Ala Phe Thr Thr Lys Asn Trp Ile
               660                 665                 670
   Val Arg Ile Tyr Lys Val Gly Asp Asp Pro Gln Trp Asn Arg Val Tyr
           675                 680                 685
<210> 8
   <211> 836
   <212> PRT
   <213> Leishmania infantum
<400> 8
   Met Gly Lys Lys Gly Asn Leu Leu Gly Asp Ser Gly Ser Ala Ala Thr
   1               5                   10                  15
   Ala Ser Pro Pro Ala Asn Met Ile Leu Leu Pro Lys Thr Pro Ile Asp
               20                  25                  30
   Thr Lys Asp Phe Ile Gly Ile Phe Ser Phe Pro Phe Trp Pro Val Arg
           35                  40                  45
   Phe Val Val Thr Val Val Ala Leu Phe Val Val Gly Ala Ser Cys Phe
       50                  55                  60
   Gln Ala Phe Thr Val Arg Met Thr Ser Val Gln Ile Tyr Gly Tyr Leu
   65                  70                  75                  80
   Ile His Glu Phe Asp Pro Trp Phe Asn Tyr Arg Ala Ala Glu Tyr Met
                   85                  90                  95
   Ser Thr His Gly Trp Ser Ala Phe Phe Ser Trp Phe Asp Tyr Met Ser
               100                 105                 110
   Trp Tyr Pro Leu Gly Arg Pro Val Gly Ser Thr Thr Tyr Pro Gly Leu
           115                 120                 125
   Gln Leu Thr Ala Val Ala Ile His Arg Ala Leu Ala Ala Ala Gly Met
       130                 135                 140
   Pro Met Ser Leu Asn Asn Val Cys Val Leu Met Pro Ala Trp Phe Gly
   145                 150                 155                 160
   Ala Ile Ala Thr Ala Thr Leu Ala Phe Cys Thr Tyr Glu Ala Ser Gly
                   165                 170                 175
   Ser Thr Val Ala Ala Ala Ala Ala Ala Leu Ser Phe Ser Ile Ile Pro
               180                 185                 190
   Ala His Leu Met Arg Ser Met Ala Gly Glu Phe Asp Asn Glu Cys Ile
           195                 200                 205
   Ala Val Ala Ala Met Leu Leu Thr Phe Tyr Cys Trp Val Arg Ser Leu
       210                 215                 220
   Arg Thr Arg Ser Ser Trp Pro Ile Gly Val Leu Thr Gly Val Ala Tyr
   225                 230                 235                 240
   Gly Tyr Met Val Ala Ala Trp Gly Gly Tyr Ile Phe Val Leu Asn Met
                   245                 250                 255
   Val Ala Met His Ala Gly Ile Ser Ser Met Val Asp Trp Ala Arg Asn
               260                 265                 270
   Thr Tyr Asn Pro Ser Leu Leu Arg Ala Tyr Thr Leu Phe Tyr Val Val
           275                 280                 285
   Gly Thr Ala Ile Ala Val Cys Val Pro Pro Val Gly Met Ser Pro Phe
       290                 295                 300
   Lys Ser Leu Glu Gln Leu Gly Ala Leu Leu Val Leu Val Phe Leu Cys
   305                 310                 315                 320
   Gly Leu Gln Ala Cys Glu Val Phe Arg Ala Arg Ala Gly Val Glu Val
                   325                 330                 335
   Arg Ser Arg Ala Asn Phe Lys Ile Arg Val Arg Val Phe Ser Val Met
               340                 345                 350
   Ala Gly Val Ala Ala Leu Ala Ile Ala Val Leu Ala Pro Thr Gly Tyr
           355                 360                 365
   Phe Gly Pro Leu Ser Val Arg Val Arg Ala Leu Phe Val Glu His Thr
       370                 375                 380
   Arg Thr Gly Asn Pro Leu Val Asp Ser Val Ala Glu His Gln Pro Ala
   385                 390                 395                 400
   Gly Pro Glu Ala Met Trp Ser Phe Leu His Val Cys Gly Val Thr Trp
                   405                 410                 415
   Gly Leu Gly Ser Ile Val Leu Ala Leu Ser Thr Phe Val His Tyr Ala
               420                 425                 430
   Pro Ser Lys Leu Phe Trp Leu Leu Asn Ser Gly Ala Val Tyr Tyr Phe
           435                 440                 445
   Ser Thr Arg Met Ala Arg Leu Leu Leu Leu Ser Gly Pro Ala Ala Cys
       450                 455                 460
   Leu Ser Thr Gly Ile Phe Val Gly Thr Ile Leu Glu Ala Ala Val Gln
   465                 470                 475                 480
   Leu Ser Phe Trp Asp Ser Asp Ala Thr Lys Ala Arg Lys Gln Gln Lys
                   485                 490                 495
   Pro Ala Gln Arg His Arg Arg Gly Ala Gly Lys Asp Ser Asp Arg Asp
               500                 505                 510
   Asp Ala Glu Ser Ala Thr Thr Ala Arg Thr Leu Cys Asp Val Phe Ala
           515                 520                 525
   Gly Ser Pro Leu Ala Trp Gly His Arg Met Val Leu Phe Ile Ala Val
       530                 535                 540
   Trp Ala Leu Val Thr Thr Thr Ala Val Ser Phe Phe Ser Ser Asp Phe
   545                 550                 555                 560
   Ala Ser His Ser Thr Thr Phe Ala Glu Gln Ser Ser Asn Pro Met Ile
   565 570 575
   Val Phe Ala Ala Val Val Gln Asn Arg Ala Thr Gly Lys Pro Met Asn
   580 585 590
   Ile Leu Val Asp Asp Tyr Leu Arg Ser Tyr Ile Trp Leu Arg Asp Asn
   595 600 605
   Thr Pro Glu Asp Ala Arg Ile Leu Ala Trp Trp Asp Tyr Gly Tyr Gln
   610 615 620
   Ile Thr Gly Ile Gly Asn Arg Thr Ser Leu Ala Asp Gly Asn Thr Trp
   625 630 635 640
   Asn His Glu His Ile Ala Thr Ile Gly Lys Met Leu Thr Ser Pro Val
   645 650 655
   Ala Glu Ala His Ser Leu Val Arg His Met Ala Asp Tyr Val Leu Ile
   660 665 670
   Trp Ala Gly Gln Ser Gly Asp Leu Met Lys Ser Pro His Met Ala Arg
   675 680 685
   Ile Gly Asn Ser Val Tyr His Asp Ile Cys Pro His Asp Pro Leu Cys
   690 695 700
   Gln Gln Phe Gly Phe Tyr Arg Asn Asp Tyr Ser Arg Pro Thr Pro Met
   705 710 715 720
   Met Arg Ala Ser Leu Leu Tyr Asn Leu His Glu Val Gly Lys Thr Lys
   725 730 735
   Gly Val Lys Val Asp Pro Ser Leu Phe Gln Glu Val Tyr Ser Ser Lys
   740 745 750
   Tyr Gly Leu Val Arg Val Phe Lys Val Met Asn Val Ser Glu Glu Ser
   755 760 765
   Lys Lys Trp Val Ala Asp Pro Ala Asn Arg Val Cys His Pro Pro Gly
   770 775 780
   Ser Trp Ile Cys Pro Gly Gln Tyr Pro Pro Ala Lys Glu Ile Gln Glu
   785 790 795 800
   Met Leu Ala His Arg Val Pro Phe Asp Gln Val Glu Lys Val Asp Arg
   805 810 815
   Lys Asn His Val Gly Ser Tyr His Glu Glu Tyr Met Arg Arg Met Arg
               820                 825                 830
   Glu Ser Glu Ser
           835
<210> 9
   <211> 2511
   <212> DNA
   <213> Leishmania infantum
<400> 9
   atgggcaaga agggcaacct cctcggcgat agcggctctg ctgccaccgc cagcccccct      60
   gccaacatga tcctgctccc caagaccccc atcgacacca aggacttcat cggcatcttc     120
   agcttcccgt tctggcccgt ccgcttcgtc gtcaccgtcg tcgccctctt cgtcgtcggc     180
   gccagctgct tccaggcctt caccgtccgc atgaccagcg tccagatcta cggctacctc     240
   atccacgagt tcgacccctg gttcaactac cgagccgccg agtacatgag cacccacggc     300
   tggtccgcct ttttcagctg gttcgactat atgagctggt atcccctcgg ccgacccgtc     360
   ggcagcacca cctaccccgg cctccagctc accgctgtcg ccatccaccg agccctcgct     420
   gcggctggca tgcccatgag cctcaacaac gtctgcgtcc tcatgcccgc ctggttcggc     480
   gccattgcga ccgccaccct cgcgttctgc acctacgagg ccagcggcag cacagtggct     540
   gctgccgctg cggccctcag cttcagcatc atccccgccc acctcatgcg cagcatggcc     600
   ggcgagttcg acaacgagtg cattgccgtc gccgccatgc tcctcacctt ctactgctgg     660
   gtccgctccc tccgcacccg cagcagctgg cccatcggcg tcctcaccgg ggtcgcctac     720
   ggctacatgg tggccgcctg gggcggctac atcttcgtcc tcaacatggt cgccatgcac     780
   gccggcatca gcagcatggt cgactgggcc cgcaacacct acaaccccag cctgctccgc     840
   gcctacaccc tcttctacgt cgtcggcacc gccattgccg tctgcgtccc ccccgtcggc     900
   atgagcccct tcaagagcct cgagcagctc ggagcgctgc tcgtcctggt ctttctgtgc     960
   ggcctccagg cctgcgaggt ctttcgcgcc cgagccggcg tcgaggtccg cagccgcgcc    1020
   aacttcaaga tccgcgtccg cgtgttcagc gtcatggccg gcgtcgccgc cttggctatc    1080
   gccgtcctcg cccccaccgg ctacttcggc cccctcagcg tccgcgtgcg cgccctgttc    1140
   gtcgagcaca cccgcaccgg caatcccctg gtcgacagcg tcgccgagca ccagcctgcc    1200
   ggccctgagg ccatgtggtc gttcctccac gtctgcggcg tcacctgggg cctcggatcc    1260
   atcgtcctgg ccctcagcac cttcgtccac tacgccccca gcaagctgtt ctggctcctc    1320
   aactctggcg ccgtctacta cttctcgacc cgaatggccc gcctcctgct cctcagcggc    1380
   cctgccgcct gcctcagcac cggcatcttc gtgggcacca tcctcgaggc cgccgtccag    1440
   ctcagcttct gggacagcga cgccaccaag gcccgcaagc agcagaagcc tgcccagcgc    1500
   caccgacggg gagccggcaa ggatagcgac cgcgacgacg ccgagtctgc caccaccgcc     1560
   cgcaccctct gcgacgtctt tgccggcagc cccctcgcct ggggccaccg catggtcctc     1620
   ttcattgccg tgtgggccct cgtcacgacg accgccgtca gcttcttcag cagcgacttc     1680
   gccagccaca gcaccacctt cgccgagcag agcagcaacc ccatgatcgt ctttgccgcc     1740
   gtcgtccaga accgcgccac cggcaagccg atgaacatcc tcgtcgacga ctacctccgc     1800
   agctacatct ggctccgcga caacaccccc gaggacgccc gcatcctcgc ctggtgggac     1860
   tacggctacc agatcaccgg catcggcaac cgcaccagcc tcgccgacgg caacacctgg     1920
   aaccacgagc acattgccac catcggcaag atgctcacca gccccgtcgc cgaggcccac     1980
   agcctcgtcc gccacatggc cgactacgtc ctcatctggg ctggccagag cggcgacctc     2040
   atgaagtccc cccacatggc ccgcatcggc aacagcgtct accacgacat ctgcccccac     2100
   gaccccctct gccagcagtt cggcttctac cgcaacgact acagccgccc caccccgatg     2160
   atgcgcgcca gcctcctcta caacctccac gaggtcggca agaccaaggg cgtcaaggtc     2220
   gaccccagcc tcttccaaga ggtctacagc agcaagtacg gcctcgtgcg cgtgttcaag     2280
   gtcatgaacg tcagcgaaga gtccaagaag tgggtcgcgg accccgccaa cagggtctgc     2340
   cacccccctg gcagctggat ctgccctggc cagtaccctc ccgccaaaga gatccaagag     2400
   atgctcgccc accgcgtccc gttcgaccag gtcgagaagg tcgaccgcaa gaaccacgtc     2460
   ggctcctacc acgaagagta catgcgccgc atgcgcgaga gcgagagctg a              2511
<210> 10
   <211> 721
   <212> PRT
   <213> Entamoeba histolytica
<400> 10
   Met Gly Phe Phe Lys Thr Leu Val Gln Leu Ile Leu Lys Asn Ile Gly
   1               5                   10                  15
   Ile Thr Leu Ile Cys Ile Ile Ala Phe Ser Ser Arg Leu Tyr Ser Ile
               20                  25                  30
   Ile Met Tyr Glu Ala Ile Ile His Glu Phe Asp Pro Tyr Phe Asn Phe
           35                  40                  45
   Arg Ala Thr Lys Tyr Leu Val Glu His Gly Pro Thr Ala Phe Met Asn
       50                  55                  60
   Trp Phe Asp Pro Asp Ser Trp Tyr Pro Leu Gly Arg Asn Ile Gly Thr
   65                  70                  75                  80
   Thr Val Phe Pro Gly Leu Met Phe Thr Ser Ala Phe Ile Phe Lys Phe
                   85                  90                  95
   Leu Ala Tyr Phe Asn Leu Ile Ile Asp Val Arg Leu Ile Cys Val Cys
               100                 105                 110
   Met Gly Pro Ile Tyr Ser Val Ile Thr Cys Ile Val Ala Tyr Leu Phe
           115                 120                 125
   Gly Ser Arg Val His Ser Asp Arg Ala Gly Leu Phe Ala Ala Ala Leu
       130                 135                 140
   Ile Ser Val Val Pro Gly Tyr Met Ser Arg Ser Val Ala Gly Ser Tyr
   145                 150                 155                 160
   Asp Tyr Glu Cys Ile Ser Ile Thr Ile Leu Ile Leu Thr Phe Tyr Leu
                   165                 170                 175
   Trp Ile Glu Ala Val His Asn Asn Ser Pro Ile Leu Ser Ala Val Thr
               180                 185                 190
   Ala Leu Ser Tyr Phe Tyr Met Ala Ser Thr Trp Gly Ala Tyr Val Phe
           195                 200                 205
   Ile Asn Asn Ile Ile Pro Leu His Val Leu Ile Ser Ile Phe Cys Gly
       210                 215                 220
   Phe Tyr Asn Lys Lys Leu Tyr Ser Cys Tyr Ser Ile Tyr Tyr Ile Phe
   225                 230                 235                 240
   Ala Thr Ile Leu Ser Met Gln Val Pro Phe Ile Asn Tyr Val Pro Ile
                   245                 250                 255
   Arg Ser Ser Glu His Ile Gly Ala Met Gly Val Phe Gly Ile Cys Gln
               260                 265                 270
   Leu Ile Glu Leu Tyr Ser Leu Ile His Lys Leu Leu Gly Gln Lys Lys
           275                 280                 285
   Thr Val Glu Leu Ile Lys Lys Val Leu Met Gly Ser Val Ile Ile Gly
       290                 295                 300
   Ile Ile Met Val Leu Ile Leu Ile Lys Lys Gly Tyr Ile Ser Ala Trp
   305                 310                 315                 320
   Ser Gly Arg Phe Tyr Ala Leu Phe Asp Pro Thr Phe Ala Lys Lys Asn
                   325                 330                 335
   Ile Pro Leu Ile Val Ser Val Ser Glu His Gln Pro Ala Asn Trp Ala
               340                 345                 350
   Ser Tyr Phe Phe Asp Leu His Cys Leu Ile Val Ile Ala Pro Ala Gly
           355                 360                 365
   Leu Tyr Tyr Cys Phe Lys Lys Phe Asp Phe Asn Met Leu Phe Leu Ile
       370                 375                 380
   Ile Tyr Ser Val Ser Val Phe Tyr Phe Ser Cys Val Met Ser Arg Leu
   385                 390                 395                 400
   Val Leu Ile Leu Ala Pro Ala Ile Cys Leu Leu Ser Gly Ile Ala Leu
                   405                 410                 415
   Ala Glu Phe Phe Thr Gln Ile Gln Lys Gln Leu Glu Ser Thr Leu Lys
               420                 425                 430
   Met Val Phe Lys Ser Asn Lys Lys Gln Gln Gln Gln Gln Ser Asn Glu
           435                 440                 445
   Pro Thr Thr Lys Ile Glu Lys Glu Lys Arg Lys Ile His Pro Pro Lys
       450                 455                 460
   Lys Glu Gln Asn Asn Glu Lys Ser Phe Ile Ser Glu Phe Ile Ile Phe
   465                 470                 475                 480
   Ile Ile Met Thr Ile Val Gly Ile Leu Leu Ile Ile Phe Leu Phe Lys
                   485                 490                 495
   Phe Phe Glu Tyr Ser Ile Gln Met Ser Lys Asn Tyr Ser Ser Pro Ser
               500                 505                 510
   Val Val Leu Tyr Gly Asn His Gly Gly Lys Gln Ile Ala Phe Asp Asp
           515                 520                 525
   Tyr Arg Glu Ala Tyr Arg Trp Leu Ala His Asn Thr Pro Glu Gly Ser
       530                 535                 540
   Arg Val Met Ser Trp Trp Asp Tyr Gly Tyr Gln Ile Ser His Leu Ala
   545                 550                 555                 560
   Asn Arg Thr Val Ile Val Asp Asn Asn Thr Trp Asn Asn Ser His Ile
                   565                 570                 575
   Ala Leu Thr Gly Asn Val Met Ala Ser Arg Glu Glu Asp Ala Met Lys
               580                 585                 590
   Thr Ile Arg Asp Leu Asp Val Asp Tyr Leu Leu Val Val Phe Gly Gly
   595 600 605
   Tyr Leu Gly Tyr Ser Ser Asp Asp Ile Asn Lys Phe Leu Trp Met Ile
   610 615 620
   Arg Ile Gly Ala Gly Val Asn Pro Ser Leu Asn Glu Asn Asn Tyr Tyr
   625 630 635 640
   Asn His Asn Ala Tyr Thr Val Ala Asp Pro Ser Asp Thr Phe Lys Tyr
   645 650 655
   Ser Met Met Tyr Lys Met Cys Tyr His Asn Phe Tyr Lys Ala Ser Asn
   660 665 670
   Gly Tyr Arg Ala Gly Met Asp Ala Val Arg Arg Glu Val Ile Glu Glu
   675 680 685
   Gln Thr Tyr Phe Lys Asn Ile Gln Glu Ala Phe Thr Ser Gln His Trp
   690 695 700
   Val Val Arg Ile Tyr Lys Val Asn Lys Pro Asn Pro Ile Asp Ser Leu
   705 710 715 720
   Leu
<210> 11
   <211> 40
   <212> DNA
   <213> Trichoderma reesei
<400> 11
   agctccgtgg cgaaagcctg acgcaccggt agattcttgg 40
<210> 12
   <211> 1000
   <212> DNA
   <213> Artificial
<220>
   <223> Primer
<400> 12
   gttgggctga ggccgtatcg gagggacggg gtgaggattg aggcggagga gatgaagggg 60
   gatgatgggg agacggtggt tgttgtgcat aattatgggc atgcgggatg ggggtatcag 120
   gggtcgtatg ggtgtgcgga gagggttgtc gagttggtgg aggggattgt gaggggatga 180
   gcggatgttt ttgatgtttt gactgctcgc ctttgactcg attctgatac ggacactttt 240
   cgacctttgt ttctccaaga tggccctgta cagtcagatt gatagaggag catgtataat 300
   tcattgccgg ttgccgtccc gtttccaagc agaaagccac tgttgagaag caacgtgctt 360
   tgacgaaagt cgtggctcac tactcaaatc tctccacact catacattgt gtttcagtca 420
   aaacactttg gcaaccaaga cgtgggaggg agtatctgca tcttttctca tcggcaagct 480
   atctgactcg attgagaaga tgcgtggttc atatcacctg gccgttggag gtttcttcct 540
   aggcagtcgc tctgttctcc ttctataaag aactccatcg ttcttgaata cctctttggc 600
   cttcaagctc gatagtattg aacccattct tcactcatgc tgctcatcat tccacctccc 660
   tcaagttggg tgtcgttgag tacctagtgt acataagcgg gtctatgcat ttaaaggggt 720
   atcttcacca ccagcaatat ccacacttct aggctccacg ttgcacataa cgaaaccaaa 780
   acagctaaac cgacgggcca atttcacgcg catcttcatc gacgaagcga gcgacagcga 840
   agccgatacg caaatcctct tcagacaagc tcaactcggc caagcctcat gttttgccaa 900
   cggaaccctg cacaagtcgg ctggcattaa agaggaaagg agaacagaaa gagagtgagc 960
   agatttcagt ctctcaccac tcacctgagt tgcctctctc 1000
<210> 13
   <211> 1001
   <212> DNA
   <213> Artificial
<220>
   <223> Primer
<400> 13
   gggcagtatg ccggatggct ggcttataca ggcaaaaacc accttcttca ttcttcattc      60
   ttcgtcttct tcttcttctt cctcctcatc gtcggtaggc ggcagctttc ccacattgga     120
   gtcgctctcc tcgtcgctga gttcctcgac cgtcttttcg aattcctttg gcctggagtc     180
   atcataatag tttaatacac gtttagagta tagagagaaa aaataagggg gaaaaagacg     240
   caaatcatac cagtacggct gcttccgcca gagcttctcg tcgcgcacga ccttgataat     300
   ctcgccaaag gccctgctgt cctgcgtgcc gacgggatat ccctcggcca ggacgtagcc     360
   gccgcgcttg atccagacgg tgttgcgcag gcgctgggcg agctcgagga cgacgtgctt     420
   cttgctgggc atctcgcagg tgtagaggtt gttgccctcg ggcttcagga cgcgcacaat     480
   ggcctgcgtc ggctcgaggg catcgggagg cgtgagggcc tcttgtgtgg cggcaaggac     540
   atttcgcttc ggcttaccca tggctgcgag tctttggggt cgattcggtg atactatctg     600
   atcccaagaa aaaagagaca aaatttcatt gttgttgatt ggaaaataaa ctggggccgt     660
   gatggagggg cagctttatc gataggacgg ggatttctcg aataggaaaa taaaacccct     720
   ccgcccgtcc cgctctccgg cacggtgttg ccccattcgg cgaaaccgct tcagggacca     780
   aactagaagt aaggtaccta tccataagct atcacgatga tatagaaggc atggatgtat     840
   tgcaaaagcg aattgttaga cgccccaatg ggaggcttgg tggggttatc ggtttacgaa     900
   atacttgaat caatgcatta ttaatctatc cattaggcat tttggcgttc accagaccgt     960
   ttgactcacc gatatcgttc gtggtggtac tcggccagat g                        1001
<210> 14
   <211> 19
   <212> DNA
   <213> Artificial
<220>
   <223> Primer
<400> 14
   gcacactttc aagattggc 19
<210> 15
   <211> 19
   <212> DNA
   <213> Artificial
<220>
   <223> Primer
<400> 15
   gcacactttc aagattggc 19
<210> 16
   <211> 19
   <212> DNA
   <213> Artificial
<220>
   <223> Primer
<400> 16
   gtacggtgtt gccaagaag 19
<210> 17
   <211> 448
   <212> DNA
   <213> Artificial
<220>
   <223> Primer
<400> 17
   gttgagtaca tcgagcgcga cagcattgtg cacaccatgc ttcccctcga gtccaaggac        60
   agcatcatcg ttgaggactc gtgcaacggc gagacggaga agcaggctcc ctggggtctt       120
   gcccgtatct ctcaccgaga gacgctcaac tttggctcct tcaacaagta cctctacacc       180
   gctgatggtg gtgagggtgt tgatgcctat gtcattgaca ccggcaccaa catcgagcac       240
   gtcgactttg agggtcgtgc caagtggggc aagaccatcc ctgccggcga tgaggacgag       300
   gacggcaacg gccacggcac tcactgctct ggtaccgttg ctggtaagaa gtacggtgtt       360
   gccaagaagg cccacgtcta cgccgtcaag gtgctccgat ccaacggatc cggcaccatg       420
   tctgacgtcg tcaagggcgt cgagtacg                                          448
<210> 18
   <211> 399
   <212> PRT
   <213> Trichoderma reesei
<400> 18
   Met Gln Pro Ser Phe Gly Ser Phe Leu Val Thr Val Leu Ser Ala Ser
   1               5                   10                  15
   Met Ala Ala Gly Ser Val Ile Pro Ser Thr Asn Ala Asn Pro Gly Ser
               20                  25                  30
   Phe Glu Ile Lys Arg Ser Ala Asn Lys Ala Phe Thr Gly Arg Asn Gly
           35                  40                  45
   Pro Leu Ala Leu Ala Arg Thr Tyr Ala Lys Tyr Gly Val Glu Val Pro
       50                  55                  60
   Lys Thr Leu Val Asp Ala Ile Gln Leu Val Lys Ser Ile Gln Leu Ala
   65                  70                  75                  80
   Lys Arg Asp Ser Ala Thr Val Thr Ala Thr Pro Asp His Asp Asp Ile
                   85                  90                  95
   Glu Tyr Leu Val Pro Val Lys Ile Gly Thr Pro Pro Gln Thr Leu Asn
               100                 105                 110
   Leu Asp Phe Asp Thr Gly Ser Ser Asp Leu Trp Val Phe Ser Ser Asp
           115                 120                 125
   Val Asp Pro Thr Ser Ser Gln Gly His Asp Ile Tyr Thr Pro Ser Lys
       130                 135                 140
   Ser Thr Ser Ser Lys Lys Leu Glu Gly Ala Ser Trp Asn Ile Thr Tyr
   145                 150                 155                 160
   Gly Asp Arg Ser Ser Ser Ser Gly Asp Val Tyr His Asp Ile Val Ser
                   165                 170                 175
   Val Gly Asn Leu Thr Val Lys Ser Gln Ala Val Glu Ser Ala Arg Asn
               180                 185                 190
   Val Ser Ala Gln Phe Thr Gln Gly Asn Asn Asp Gly Leu Val Gly Leu
   195 200 205
   Ala Phe Ser Ser Ile Asn Thr Val Lys Pro Thr Pro Gln Lys Thr Trp
   210 215 220
   Tyr Asp Asn Ile Val Gly Ser Leu Asp Ser Pro Val Phe Val Ala Asp
   225 230 235 240
   Leu Arg His Asp Thr Pro Gly Ser Tyr His Phe Gly Ser Ile Pro Ser
   245 250 255
   Glu Ala Ser Lys Ala Phe Tyr Ala Pro Ile Asp Asn Ser Lys Gly Phe
   260 265 270
   Trp Gln Phe Ser Thr Ser Ser Asn Ile Ser Gly Gln Phe Asn Ala Val
   275 280 285
   Ala Asp Thr Gly Thr Thr Leu Leu Leu Ala Ser Asp Asp Leu Val Lys
   290 295 300
   Ala Tyr Tyr Ala Lys Val Gln Gly Ala Arg Val Asn Val Phe Leu Gly
   305 310 315 320
   Gly Tyr Val Phe Asn Cys Thr Thr Gln Leu Pro Asp Phe Thr Phe Thr
   325 330 335
   Val Gly Glu Gly Asn Ile Thr Val Pro Gly Thr Leu Ile Asn Tyr Ser
   340 345 350
   Glu Ala Gly Asn Gly Gln Cys Phe Gly Gly Ile Gln Pro Ser Gly Gly
   355 360 365
   Leu Pro Phe Ala Ile Phe Gly Asp Ile Ala Leu Lys Ala Ala Tyr Val
   370 375 380
   Ile Phe Asp Ser Gly Asn Lys Gln Val Gly Trp Ala Gln Lys Lys
   385 390 395
<210> 19
   <211> 452
   <212> PRT
   <213> Trichoderma reesei
<400> 19
   Met Glu Ala Ile Leu Gln Ala Gln Ala Lys Phe Arg Leu Asp Arg Gly
   1 5 10 15
   Leu Gln Lys Ile Thr Ala Val Arg Asn Lys Asn Tyr Lys Arg His Gly
               20                  25                  30
   Pro Lys Ser Tyr Val Tyr Leu Leu Asn Arg Phe Gly Phe Glu Pro Thr
           35                  40                  45
   Lys Pro Gly Pro Tyr Phe Gln Gln His Arg Ile His Gln Arg Gly Leu
       50                  55                  60
   Ala His Pro Asp Phe Lys Ala Ala Val Gly Gly Arg Val Thr Arg Gln
   65                  70                  75                  80
   Lys Val Leu Ala Lys Lys Val Lys Glu Asp Gly Thr Val Asp Ala Gly
                   85                  90                  95
   Gly Ser Lys Thr Gly Glu Val Asp Ala Glu Asp Gln Gln Asn Asp Ser
               100                 105                 110
   Glu Tyr Leu Cys Glu Val Thr Ile Gly Thr Pro Gly Gln Lys Leu Met
           115                 120                 125
   Leu Asp Phe Asp Thr Gly Ser Ser Asp Leu Trp Val Phe Ser Thr Glu
       130                 135                 140
   Leu Ser Lys His Leu Gln Glu Asn His Ala Ile Phe Asp Pro Lys Lys
   145                 150                 155                 160
   Ser Ser Thr Phe Lys Pro Leu Lys Asp Gln Thr Trp Gln Ile Ser Tyr
                   165                 170                 175
   Gly Asp Gly Ser Ser Ala Ser Gly Thr Cys Gly Ser Asp Thr Val Thr
               180                 185                 190
   Leu Gly Gly Leu Ser Ile Lys Asn Gln Thr Ile Glu Leu Ala Ser Lys
           195                 200                 205
   Leu Ala Pro Gln Phe Ala Gln Gly Thr Gly Asp Gly Leu Leu Gly Leu
       210                 215                 220
   Ala Trp Pro Gln Ile Asn Thr Val Gln Thr Asp Gly Arg Pro Thr Pro
   225                 230                 235                 240
   Ala Asn Thr Pro Val Ala Asn Met Ile Gln Gln Asp Asp Ile Pro Ser
                   245                 250                 255
   Asp Ala Gln Leu Phe Thr Ala Ala Phe Tyr Ser Glu Arg Asp Glu Asn
               260                 265                 270
   Ala Glu Ser Phe Tyr Thr Phe Gly Tyr Ile Asp Gln Asp Leu Val Ser
           275                 280                 285
   Ala Ser Gly Gln Glu Ile Ala Trp Thr Asp Val Asp Asn Ser Gln Gly
       290                 295                 300
   Phe Trp Met Phe Pro Ser Thr Lys Thr Thr Ile Asn Gly Lys Asp Ile
   305                 310                 315                 320
   Ser Gln Glu Gly Asn Thr Ala Ile Ala Asp Thr Gly Thr Thr Leu Ala
                   325                 330                 335
   Leu Val Ser Asp Glu Val Cys Glu Ala Leu Tyr Lys Ala Ile Pro Gly
               340                 345                 350
   Ala Lys Tyr Asp Asp Asn Gln Gln Gly Tyr Val Phe Pro Ile Asn Thr
           355                 360                 365
   Asp Ala Ser Ser Leu Pro Glu Leu Lys Val Ser Val Gly Asn Thr Gln
       370                 375                 380
   Phe Val Ile Gln Pro Glu Asp Leu Ala Phe Ala Pro Ala Asp Asp Ser
   385                 390                 395                 400
   Asn Trp Tyr Gly Gly Val Gln Ser Arg Gly Ser Asn Pro Phe Asp Ile
                   405                 410                 415
   Leu Gly Asp Val Phe Leu Lys Ser Val Tyr Ala Ile Phe Asp Gln Gly
               420                 425                 430
   Asn Gln Arg Phe Gly Ala Val Pro Lys Ile Gln Ala Lys Gln Asn Leu
           435                 440                 445
   Gln Pro Pro Gln
       450
<210> 20
   <211> 395
   <212> PRT
   <213> Trichoderma reesei
<400> 20
   Met Lys Ser Ala Leu Leu Ala Ala Ala Ala Leu Val Gly Ser Ala Gln
   1               5                   10                  15
   Ala Gly Ile His Lys Met Lys Leu Gln Lys Val Ser Leu Glu Gln Gln
               20                  25                  30
   Leu Glu Gly Ser Ser Ile Glu Ala His Val Gln Gln Leu Gly Gln Lys
           35                  40                  45
   Tyr Met Gly Val Arg Pro Thr Ser Arg Ala Glu Val Met Phe Asn Asp
       50                  55                  60
   Lys Pro Pro Lys Val Gln Gly Gly His Pro Val Pro Val Thr Asn Phe
   65                  70                  75                  80
   Met Asn Ala Gln Tyr Phe Ser Glu Ile Thr Ile Gly Thr Pro Pro Gln
                   85                  90                  95
   Ser Phe Lys Val Val Leu Asp Thr Gly Ser Ser Asn Leu Trp Val Pro
               100                 105                 110
   Ser Gln Ser Cys Asn Ser Ile Ala Cys Phe Leu His Ser Thr Tyr Asp
           115                 120                 125
   Ser Ser Ser Ser Ser Thr Tyr Lys Pro Asn Gly Ser Asp Phe Glu Ile
       130                 135                 140
   His Tyr Gly Ser Gly Ser Leu Thr Gly Phe Ile Ser Asn Asp Val Val
   145                 150                 155                 160
   Thr Ile Gly Asp Leu Lys Ile Lys Gly Gln Asp Phe Ala Glu Ala Thr
                   165                 170                 175
   Ser Glu Pro Gly Leu Ala Phe Ala Phe Gly Arg Phe Asp Gly Ile Leu
               180                 185                 190
   Gly Leu Gly Tyr Asp Thr Ile Ser Val Asn Gly Ile Val Pro Pro Phe
           195                 200                 205
   Tyr Gln Met Val Asn Gln Lys Leu Ile Asp Glu Pro Val Phe Ala Phe
       210                 215                 220
   Tyr Leu Gly Ser Ser Asp Glu Gly Ser Glu Ala Val Phe Gly Gly Val
   225                 230                 235                 240
   Asp Asp Ala His Tyr Glu Gly Lys Ile Glu Tyr Ile Pro Leu Arg Arg
                   245                 250                 255
   Lys Ala Tyr Trp Glu Val Asp Leu Asp Ser Ile Ala Phe Gly Asp Glu
               260                 265                 270
   Val Ala Glu Leu Glu Asn Thr Gly Ala Ile Leu Asp Thr Gly Thr Ser
           275                 280                 285
   Leu Asn Val Leu Pro Ser Gly Leu Ala Glu Leu Leu Asn Ala Glu Ile
   290 295 300
   Gly Ala Lys Lys Gly Phe Gly Gly Gln Tyr Thr Val Asp Cys Ser Lys
   305 310 315 320
   Arg Asp Ser Leu Pro Asp Ile Thr Phe Ser Leu Ala Gly Ser Lys Tyr
   325 330 335
   Ser Leu Pro Ala Ser Asp Tyr Ile Ile Glu Met Ser Gly Asn Cys Ile
   340 345 350
   Ser Ser Phe Gln Gly Met Asp Phe Pro Glu Pro Val Gly Pro Leu Val
   355 360 365
   Ile Leu Gly Asp Ala Phe Leu Arg Arg Tyr Tyr Ser Val Tyr Asp Leu
   370 375 380
   Gly Arg Asp Ala Val Gly Leu Ala Lys Ala Lys
   385 390 395
<210> 21
   <211> 426
   <212> PRT
   <213> Trichoderma reesei
<400> 21
   Met Lys Phe His Ala Ala Ala Leu Thr Leu Ala Cys Leu Ala Ser Ser
   1 5 10 15
   Ala Ser Ala Gly Val Ala Gln Pro Arg Ala Asp Glu Val Glu Ser Ala
   20 25 30
   Glu Gln Gly Lys Thr Phe Ser Leu Glu Gln Ile Pro Asn Glu Arg Tyr
   35 40 45
   Lys Gly Asn Ile Pro Ala Ala Tyr Ile Ser Ala Leu Ala Lys Tyr Ser
   50 55 60
   Pro Thr Ile Pro Asp Lys Ile Lys His Ala Ile Glu Ile Asn Pro Asp
   65 70 75 80
   Leu His Arg Lys Phe Ser Lys Leu Ile Asn Ala Gly Asn Met Thr Gly
   85 90 95
   Thr Ala Val Ala Ser Pro Pro Pro Gly Ala Asp Ala Glu Tyr Val Leu
   100 105 110
   Pro Val Lys Ile Gly Thr Pro Pro Gln Thr Leu Pro Leu Asn Leu Asp
           115                 120                 125
   Thr Gly Ser Ser Asp Leu Trp Val Ile Ser Thr Asp Thr Tyr Pro Pro
       130                 135                 140
   Gln Val Gln Gly Gln Thr Arg Tyr Asn Val Ser Ala Ser Thr Thr Ala
   145                 150                 155                 160
   Gln Arg Leu Ile Gly Glu Ser Trp Val Ile Arg Tyr Gly Asp Gly Ser
                   165                 170                 175
   Ser Ala Asn Gly Ile Val Tyr Lys Asp Arg Val Gln Ile Gly Asn Thr
               180                 185                 190
   Phe Phe Asn Gln Gln Ala Val Glu Ser Ala Val Asn Ile Ser Asn Glu
           195                 200                 205
   Ile Ser Asp Asp Ser Phe Ser Ser Gly Leu Leu Gly Ala Ala Ser Ser
       210                 215                 220
   Ala Ala Asn Thr Val Arg Pro Asp Arg Gln Thr Thr Tyr Leu Glu Asn
   225                 230                 235                 240
   Ile Lys Ser Gln Leu Ala Arg Pro Val Phe Thr Ala Asn Leu Lys Lys
                   245                 250                 255
   Gly Lys Pro Gly Asn Tyr Asn Phe Gly Tyr Ile Asn Gly Ser Glu Tyr
               260                 265                 270
   Ile Gly Pro Ile Gln Tyr Ala Ala Ile Asn Pro Ser Ser Pro Leu Trp
           275                 280                 285
   Glu Val Ser Val Ser Gly Tyr Arg Val Gly Ser Asn Asp Thr Lys Tyr
       290                 295                 300
   Val Pro Arg Val Trp Asn Ala Ile Ala Asp Thr Gly Thr Thr Leu Leu
   305                 310                 315                 320
   Leu Val Pro Asn Asp Ile Val Ser Ala Tyr Tyr Ala Gln Val Lys Gly
                   325                 330                 335
   Ser Thr Phe Ser Asn Asp Val Gly Met Met Leu Val Pro Cys Ala Ala
               340                 345                 350
   Thr Leu Pro Asp Phe Ala Phe Gly Leu Gly Asn Tyr Arg Gly Val Ile
   355 360 365
   Pro Gly Ser Tyr Ile Asn Tyr Gly Arg Met Asn Lys Thr Tyr Cys Tyr
   370 375 380
   Gly Gly Ile Gln Ser Ser Glu Asp Ala Pro Phe Ala Val Leu Gly Asp
   385 390 395 400
   Ile Ala Leu Lys Ala Gln Phe Val Val Phe Asp Met Gly Asn Lys Val
   405 410 415
   Val Gly Phe Ala Asn Lys Asn Thr Asn Val
   420 425
<210> 22
   <211> 407
   <212> PRT
   <213> Trichoderma reesei
<400> 22
   Met Gln Thr Phe Gly Ala Phe Leu Val Ser Phe Leu Ala Ala Ser Gly
   1               5                   10                  15
   Leu Ala Ala Ala Leu Pro Thr Glu Gly Gln Lys Thr Ala Ser Val Glu
               20                  25                  30
   Val Gln Tyr Asn Lys Asn Tyr Val Pro His Gly Pro Thr Ala Leu Phe
           35                  40                  45
   Lys Ala Lys Arg Lys Tyr Gly Ala Pro Ile Ser Asp Asn Leu Lys Ser
       50                  55                  60
   Leu Val Ala Ala Arg Gln Ala Lys Gln Ala Leu Ala Lys Arg Gln Thr
   65                  70                  75                  80
   Gly Ser Ala Pro Asn His Pro Ser Asp Ser Ala Asp Ser Glu Tyr Ile
                   85                  90                  95
   Thr Ser Val Ser Ile Gly Thr Pro Ala Gln Val Leu Pro Leu Asp Phe
               100                 105                 110
   Asp Thr Gly Ser Ser Asp Leu Trp Val Phe Ser Ser Glu Thr Pro Lys
           115                 120                 125
   Ser Ser Ala Thr Gly His Ala Ile Tyr Thr Pro Ser Lys Ser Ser Thr
       130                 135                 140
   Ser Lys Lys Val Ser Gly Ala Ser Trp Ser Ile Ser Tyr Gly Asp Gly
   145 150 155 160
   Ser Ser Ser Ser Gly Asp Val Tyr Thr Asp Lys Val Thr Ile Gly Gly
   165 170 175
   Phe Ser Val Asn Thr Gln Gly Val Glu Ser Ala Thr Arg Val Ser Thr
   180 185 190
   Glu Phe Val Gln Asp Thr Val Ile Ser Gly Leu Val Gly Leu Ala Phe
   195 200 205
   Asp Ser Gly Asn Gln Val Arg Pro His Pro Gln Lys Thr Trp Phe Ser
   210 215 220
   Asn Ala Ala Ser Ser Leu Ala Glu Pro Leu Phe Thr Ala Asp Leu Arg
   225 230 235 240
   His Gly Gln Asn Gly Ser Tyr Asn Phe Gly Tyr Ile Asp Thr Ser Val
   245 250 255
   Ala Lys Gly Pro Val Ala Tyr Thr Pro Val Asp Asn Ser Gln Gly Phe
   260 265 270
   Trp Glu Phe Thr Ala Ser Gly Tyr Ser Val Gly Gly Gly Lys Leu Asn
   275 280 285
   Arg Asn Ser Ile Asp Gly Ile Ala Asp Thr Gly Thr Thr Leu Leu Leu
   290 295 300
   Leu Asp Asp Asn Val Val Asp Ala Tyr Tyr Ala Asn Val Gln Ser Ala
   305 310 315 320
   Gln Tyr Asp Asn Gln Gln Glu Gly Val Val Phe Asp Cys Asp Glu Asp
   325 330 335
   Leu Pro Ser Phe Ser Phe Gly Val Gly Ser Ser Thr Ile Thr Ile Pro
   340 345 350
   Gly Asp Leu Leu Asn Leu Thr Pro Leu Glu Glu Gly Ser Ser Thr Cys
   355 360 365
   Phe Gly Gly Leu Gln Ser Ser Ser Gly Ile Gly Ile Asn Ile Phe Gly
   370 375 380
   Asp Val Ala Leu Lys Ala Ala Leu Val Val Phe Asp Leu Gly Asn Glu
   385 390 395 400
   Arg Leu Gly Trp Ala Gln Lys
   405
<210> 23
   <211> 446
   <212> PRT
   <213> Trichoderma reesei
<400> 23
   Met Thr Leu Pro Val Pro Leu Arg Glu His Asp Leu Pro Phe Leu Lys
   1 5 10 15
   Glu Lys Arg Lys Leu Pro Ala Asp Asp Ile Pro Ser Gly Thr Tyr Thr
   20 25 30
   Leu Pro Ile Ile His Ala Arg Arg Pro Lys Leu Ala Ser Arg Ala Ile
   35 40 45
   Glu Val Gln Val Glu Asn Arg Ser Asp Val Ser Tyr Tyr Ala Gln Leu
   50 55 60
   Asn Ile Gly Thr Pro Pro Gln Thr Val Tyr Ala Gln Ile Asp Thr Gly
   65 70 75 80
   Ser Phe Glu Leu Trp Val Asn Pro Asn Cys Ser Asn Val Gln Ser Ala
   85 90 95
   Asp Gln Arg Phe Cys Arg Ala Ile Gly Phe Tyr Asp Pro Ser Ser Ser
   100 105 110
   Ser Thr Ala Asp Val Thr Ser Gln Ser Ala Arg Leu Arg Tyr Gly Ile
   115 120 125
   Gly Ser Ala Asp Val Thr Tyr Val His Asp Thr Ile Ser Leu Pro Gly
   130 135 140
   Ser Gly Ser Gly Ser Lys Ala Met Lys Ala Val Gln Phe Gly Val Ala
   145 150 155 160
   Asp Thr Ser Val Asp Glu Phe Ser Gly Ile Leu Gly Leu Gly Ala Gly
   165 170 175
   Asn Gly Ile Asn Thr Glu Tyr Pro Asn Phe Val Asp Glu Leu Ala Ala
   180 185 190
   Gln Gly Val Thr Ala Thr Lys Ala Phe Ser Leu Ala Leu Gly Ser Lys
   195 200 205
   Ala Glu Glu Glu Gly Val Ile Ile Phe Gly Gly Val Asp Thr Ala Lys
       210                 215                 220
   Phe His Gly Glu Leu Ala His Leu Pro Ile Val Pro Ala Asp Asp Ser
   225                 230                 235                 240
   Pro Asp Gly Val Ala Arg Tyr Trp Val Lys Met Lys Ser Ile Ser Leu
                   245                 250                 255
   Thr Pro Pro Pro Pro Ser Ser Ser Gly Ser Thr Asp Asp Asn Asn Asn
               260                 265                 270
   Lys Pro Val Ala Phe Pro Gln Thr Ser Met Thr Val Phe Leu Asp Ser
           275                 280                 285
   Gly Ser Thr Leu Thr Leu Leu Pro Pro Ala Leu Val Arg Gln Ile Ala
       290                 295                 300
   Ser Ala Leu Gly Ser Thr Gln Thr Asp Glu Ser Gly Phe Phe Val Val
   305                 310                 315                 320
   Asp Cys Ala Leu Ala Ser Gln Asp Gly Thr Ile Asp Phe Glu Phe Asp
                   325                 330                 335
   Gly Val Thr Ile Arg Val Pro Tyr Ala Glu Met Ile Arg Gln Val Ser
               340                 345                 350
   Thr Leu Pro Pro His Cys Tyr Leu Gly Met Met Gly Ser Thr Gln Phe
           355                 360                 365
   Ala Leu Leu Gly Asp Thr Phe Leu Arg Ser Ala Tyr Ala Val Phe Asp
       370                 375                 380
   Leu Thr Ser Asn Val Val His Leu Ala Pro Tyr Ala Asn Cys Gly Thr
   385                 390                 395                 400
   Asn Val Lys Ser Ile Thr Ser Thr Ser Ser Leu Ser Asn Leu Val Gly
                   405                 410                 415
   Thr Cys Asn Asp Pro Ser Lys Pro Ser Ser Ser Pro Ser Pro Ser Gln
               420                 425                 430
   Thr Pro Ser Ala Ser Pro Ser Ser Thr Ala Thr Gln Lys Ala
           435                 440                 445
<210> 24
   <211> 259
   <212> PRT
   <213> Trichoderma reesei
<400> 24
   Met Ala Pro Ala Ser Gln Val Val Ser Ala Leu Met Leu Pro Ala Leu
   1               5                   10                  15
   Ala Leu Gly Ala Ala Ile Gln Pro Arg Gly Ala Asp Ile Val Gly Gly
               20                  25                  30
   Thr Ala Ala Ser Leu Gly Glu Phe Pro Tyr Ile Val Ser Leu Gln Asn
           35                  40                  45
   Pro Asn Gln Gly Gly His Phe Cys Gly Gly Val Leu Val Asn Ala Asn
       50                  55                  60
   Thr Val Val Thr Ala Ala His Cys Ser Val Val Tyr Pro Ala Ser Gln
   65                  70                  75                  80
   Ile Arg Val Arg Ala Gly Thr Leu Thr Trp Asn Ser Gly Gly Thr Leu
                   85                  90                  95
   Val Gly Val Ser Gln Ile Ile Val Asn Pro Ser Tyr Asn Asp Arg Thr
               100                 105                 110
   Thr Asp Phe Asp Val Ala Val Trp His Leu Ser Ser Pro Ile Arg Glu
           115                 120                 125
   Ser Ser Thr Ile Gly Tyr Ala Thr Leu Pro Ala Gln Gly Ser Asp Pro
       130                 135                 140
   Val Ala Gly Ser Thr Val Thr Thr Ala Gly Trp Gly Thr Thr Ser Glu
   145                 150                 155                 160
   Asn Ser Asn Ser Ile Pro Ser Arg Leu Asn Lys Val Ser Val Pro Val
                   165                 170                 175
   Val Ala Arg Ser Thr Cys Gln Ala Asp Tyr Arg Ser Gln Gly Leu Ser
               180                 185                 190
   Val Thr Asn Asn Met Phe Cys Ala Gly Leu Thr Gln Gly Gly Lys Asp
           195                 200                 205
   Ser Cys Ser Gly Asp Ser Gly Gly Pro Ile Val Asp Ala Asn Gly Val
       210                 215                 220
   Leu Gln Gly Val Val Ser Trp Gly Ile Gly Cys Ala Glu Ala Gly Phe
   225                 230                 235                 240
   Pro Gly Val Tyr Thr Arg Ile Gly Asn Phe Val Asn Tyr Ile Asn Gln
   245 250 255
   Asn Leu Ala
<210> 25
   <211> 882
   <212> PRT
   <213> Trichoderma reesei
<400> 25
   Met Val Arg Ser Ala Leu Phe Val Ser Leu Leu Ala Thr Phe Ser Gly
   1 5 10 15
   Val Ile Ala Arg Val Ser Gly His Gly Ser Lys Ile Val Pro Gly Ala
   20 25 30
   Tyr Ile Phe Glu Phe Glu Asp Ser Gln Asp Thr Ala Asp Phe Tyr Lys
   35 40 45
   Lys Leu Asn Gly Glu Gly Ser Thr Arg Leu Lys Phe Asp Tyr Lys Leu
   50 55 60
   Phe Lys Gly Val Ser Val Gln Leu Lys Asp Leu Asp Asn His Glu Ala
   65 70 75 80
   Lys Ala Gln Gln Met Ala Gln Leu Pro Ala Val Lys Asn Val Trp Pro
   85 90 95
   Val Thr Leu Ile Asp Ala Pro Asn Pro Lys Val Glu Trp Val Ala Gly
   100 105 110
   Ser Thr Ala Pro Thr Leu Glu Ser Arg Ala Ile Lys Lys Pro Pro Ile
   115 120 125
   Pro Asn Asp Ser Ser Asp Phe Pro Thr His Gln Met Thr Gln Ile Asp
   130 135 140
   Lys Leu Arg Ala Lys Gly Tyr Thr Gly Lys Gly Val Arg Val Ala Val
   145 150 155 160
   Ile Asp Thr Gly Ile Asp Tyr Thr His Pro Ala Leu Gly Gly Cys Phe
   165 170 175
   Gly Arg Gly Cys Leu Val Ser Phe Gly Thr Asp Leu Val Gly Asp Asp
   180 185 190
   Tyr Thr Gly Phe Asn Thr Pro Val Pro Asp Asp Asp Pro Val Asp Cys
   195 200 205
   Ala Gly His Gly Ser His Val Ala Gly Ile Ile Ala Ala Gln Glu Asn
   210 215 220
   Pro Tyr Gly Phe Thr Gly Gly Ala Pro Asp Val Thr Leu Gly Ala Tyr
   225 230 235 240
   Arg Val Phe Gly Cys Asp Gly Gln Ala Gly Asn Asp Val Leu Ile Ser
   245 250 255
   Ala Tyr Asn Gln Ala Phe Glu Asp Gly Ala Gln Ile Ile Thr Ala Ser
   260 265 270
   Ile Gly Gly Pro Ser Gly Trp Ala Glu Glu Pro Trp Ala Val Ala Val
   275 280 285
   Thr Arg Ile Val Glu Ala Gly Val Pro Cys Thr Val Ser Ala Gly Asn
   290 295 300
   Glu Gly Asp Ser Gly Leu Phe Phe Ala Ser Thr Ala Ala Asn Gly Lys
   305 310 315 320
   Lys Val Ile Ala Val Ala Ser Val Asp Asn Glu Asn Ile Pro Ser Val
   325 330 335
   Leu Ser Val Ala Ser Tyr Lys Ile Asp Ser Gly Ala Ala Gln Asp Phe
   340 345 350
   Gly Tyr Val Ser Ser Ser Lys Ala Trp Asp Gly Val Ser Lys Pro Leu
   355 360 365
   Tyr Ala Val Ser Phe Asp Thr Thr Ile Pro Asp Asp Gly Cys Ser Pro
   370 375 380
   Leu Pro Asp Ser Thr Pro Asp Leu Ser Asp Tyr Ile Val Leu Val Arg
   385 390 395 400
   Arg Gly Thr Cys Thr Phe Val Gln Lys Ala Gln Asn Val Ala Ala Lys
   405 410 415
   Gly Ala Lys Tyr Leu Leu Tyr Tyr Asn Asn Ile Pro Gly Ala Leu Ala
   420 425 430
   Val Asp Val Ser Ala Val Pro Glu Ile Glu Ala Val Gly Met Val Asp
   435 440 445
   Asp Lys Thr Gly Ala Thr Trp Ile Ala Ala Leu Lys Asp Gly Lys Thr
       450                 455                 460
   Val Thr Leu Thr Leu Thr Asp Pro Ile Glu Ser Glu Lys Gln Ile Gln
   465                 470                 475                 480
   Phe Ser Asp Asn Pro Thr Thr Gly Gly Ala Leu Ser Gly Tyr Thr Thr
                   485                 490                 495
   Trp Gly Pro Thr Trp Glu Leu Asp Val Lys Pro Gln Ile Ser Ser Pro
               500                 505                 510
   Gly Gly Asn Ile Leu Ser Thr Tyr Pro Val Ala Leu Gly Gly Tyr Ala
           515                 520                 525
   Thr Leu Ser Gly Thr Ser Met Ala Cys Pro Leu Thr Ala Ala Ala Val
       530                 535                 540
   Ala Leu Ile Gly Gln Ala Arg Gly Thr Phe Asp Pro Ala Leu Ile Asp
   545                 550                 555                 560
   Asn Leu Leu Ala Thr Thr Ala Asn Pro Gln Leu Phe Asn Asp Gly Glu
                   565                 570                 575
   Lys Phe Tyr Asp Phe Leu Ala Pro Val Pro Gln Gln Gly Gly Gly Leu
               580                 585                 590
   Ile Gln Ala Tyr Asp Ala Ala Phe Ala Thr Thr Leu Leu Ser Pro Ser
           595                 600                 605
   Ser Leu Ser Phe Asn Asp Thr Asp His Phe Ile Lys Lys Lys Gln Ile
       610                 615                 620
   Thr Leu Lys Asn Thr Ser Lys Gln Arg Val Thr Tyr Lys Leu Asn His
   625                 630                 635                 640
   Val Pro Thr Asn Thr Phe Tyr Thr Leu Ala Pro Gly Asn Gly Tyr Pro
                   645                 650                 655
   Ala Pro Phe Pro Asn Asp Ala Val Ala Ala His Ala Asn Leu Lys Phe
               660                 665                 670
   Asn Leu Gln Gln Val Thr Leu Pro Ala Gly Arg Ser Ile Thr Val Asp
           675                 680                 685
   Val Phe Pro Thr Pro Pro Arg Asp Val Asp Ala Lys Arg Leu Ala Leu
       690                 695                 700
   Trp Ser Gly Tyr Ile Thr Val Asn Gly Thr Asp Gly Thr Ser Leu Ser
   705                 710                 715                 720
   Val Pro Tyr Gln Gly Leu Thr Gly Ser Leu His Lys Gln Lys Val Leu
                   725                 730                 735
   Tyr Pro Glu Asp Ser Trp Ile Ala Asp Ser Thr Asp Glu Ser Leu Ala
               740                 745                 750
   Pro Val Glu Asn Gly Thr Val Phe Thr Ile Pro Ala Pro Gly Asn Ala
           755                 760                 765
   Gly Pro Asp Asp Lys Leu Pro Ser Leu Val Val Ser Pro Ala Leu Gly
       770                 775                 780
   Ser Arg Tyr Val Arg Val Asp Leu Val Leu Leu Ser Ala Pro Pro His
   785                 790                 795                 800
   Gly Thr Lys Leu Lys Thr Val Lys Phe Leu Asp Thr Thr Ser Ile Gly
                   805                 810                 815
   Gln Pro Ala Gly Ser Pro Leu Leu Trp Ile Ser Arg Gly Ala Asn Pro
               820                 825                 830
   Ile Ala Trp Thr Gly Glu Leu Ser Asp Asn Lys Phe Ala Pro Pro Gly
           835                 840                 845
   Thr Tyr Lys Ala Val Phe His Ala Leu Arg Ile Phe Gly Asn Glu Lys
       850                 855                 860
   Lys Lys Glu Asp Trp Asp Val Ser Glu Ser Pro Ala Phe Thr Ile Lys
   865                 870                 875                 880
   Tyr Ala
<210> 26
   <211> 541
   <212> PRT
   <213> Trichoderma reesei
<400> 26
   Met Arg Ser Val Val Ala Leu Ser Met Ala Ala Val Ala Gln Ala Ser
   1               5                   10                  15
   Thr Phe Gln Ile Gly Thr Ile His Glu Lys Ser Ala Pro Val Leu Ser
               20                  25                  30
   Asn Val Glu Ala Asn Ala Ile Pro Asp Ala Tyr Ile Ile Lys Phe Lys
           35                  40                  45
   Asp His Val Gly Glu Asp Asp Ala Ser Lys His His Asp Trp Ile Gln
       50                  55                  60
   Ser Ile His Thr Asn Val Glu Gln Glu Arg Leu Glu Leu Arg Lys Arg
   65                  70                  75                  80
   Ser Asn Val Phe Gly Ala Asp Asp Val Phe Asp Gly Leu Lys His Thr
                   85                  90                  95
   Phe Lys Ile Gly Asp Gly Phe Lys Gly Tyr Ala Gly His Phe His Glu
               100                 105                 110
   Ser Val Ile Glu Gln Val Arg Asn His Pro Asp Val Glu Tyr Ile Glu
           115                 120                 125
   Arg Asp Ser Ile Val His Thr Met Leu Pro Leu Glu Ser Lys Asp Ser
       130                 135                 140
   Ile Ile Val Glu Asp Ser Cys Asn Gly Glu Thr Glu Lys Gln Ala Pro
   145                 150                 155                 160
   Trp Gly Leu Ala Arg Ile Ser His Arg Glu Thr Leu Asn Phe Gly Ser
                   165                 170                 175
   Phe Asn Lys Tyr Leu Tyr Thr Ala Asp Gly Gly Glu Gly Val Asp Ala
               180                 185                 190
   Tyr Val Ile Asp Thr Gly Thr Asn Ile Glu His Val Asp Phe Glu Gly
           195                 200                 205
   Arg Ala Lys Trp Gly Lys Thr Ile Pro Ala Gly Asp Glu Asp Glu Asp
       210                 215                 220
   Gly Asn Gly His Gly Thr His Cys Ser Gly Thr Val Ala Gly Lys Lys
   225                 230                 235                 240
   Tyr Gly Val Ala Lys Lys Ala His Val Tyr Ala Val Lys Val Leu Arg
                   245                 250                 255
   Ser Asn Gly Ser Gly Thr Met Ser Asp Val Val Lys Gly Val Glu Tyr
               260                 265                 270
   Ala Ala Leu Ser His Ile Glu Gln Val Lys Lys Ala Lys Lys Gly Lys
           275                 280                 285
   Arg Lys Gly Phe Lys Gly Ser Val Ala Asn Met Ser Leu Gly Gly Gly
       290                 295                 300
   Lys Thr Gln Ala Leu Asp Ala Ala Val Asn Ala Ala Val Arg Ala Gly
   305                 310                 315                 320
   Val His Phe Ala Val Ala Ala Gly Asn Asp Asn Ala Asp Ala Cys Asn
                   325                 330                 335
   Tyr Ser Pro Ala Ala Ala Thr Glu Pro Leu Thr Val Gly Ala Ser Ala
               340                 345                 350
   Leu Asp Asp Ser Arg Ala Tyr Phe Ser Asn Tyr Gly Lys Cys Thr Asp
           355                 360                 365
   Ile Phe Ala Pro Gly Leu Ser Ile Gln Ser Thr Trp Ile Gly Ser Lys
       370                 375                 380
   Tyr Ala Val Asn Thr Ile Ser Gly Thr Ser Met Ala Ser Pro His Ile
   385                 390                 395                 400
   Cys Gly Leu Leu Ala Tyr Tyr Leu Ser Leu Gln Pro Ala Gly Asp Ser
                   405                 410                 415
   Glu Phe Ala Val Ala Pro Ile Thr Pro Lys Lys Leu Lys Glu Ser Val
               420                 425                 430
   Ile Ser Val Ala Thr Lys Asn Ala Leu Ser Asp Leu Pro Asp Ser Asp
           435                 440                 445
   Thr Pro Asn Leu Leu Ala Trp Asn Gly Gly Gly Cys Ser Asn Phe Ser
       450                 455                 460
   Gln Ile Val Glu Ala Gly Ser Tyr Thr Val Lys Pro Lys Gln Asn Lys
   465                 470                 475                 480
   Gln Ala Lys Leu Pro Ser Thr Ile Glu Glu Leu Glu Glu Ala Ile Glu
                   485                 490                 495
   Gly Asp Phe Glu Val Val Ser Gly Glu Ile Val Lys Gly Ala Lys Ser
               500                 505                 510
   Phe Gly Ser Lys Ala Glu Lys Phe Ala Lys Lys Ile His Asp Leu Val
           515                 520                 525
   Glu Glu Glu Ile Glu Glu Phe Ile Ser Glu Leu Ser Glu
   530 535 540
<210> 27
   <211> 391
   <212> PRT
   <213> Trichoderma reesei
<400> 27
   Met Arg Leu Ser Val Leu Leu Ser Val Leu Pro Leu Val Leu Ala Ala
   1 5 10 15
   Pro Ala Ile Glu Lys Arg Ala Glu Pro Ala Pro Leu Leu Val Pro Thr
   20 25 30
   Thr Lys His Gly Leu Val Ala Asp Lys Tyr Ile Val Lys Phe Lys Asp
   35 40 45
   Gly Ser Ser Leu Gln Ala Val Asp Glu Ala Ile Ser Gly Leu Val Ser
   50 55 60
   Asn Ala Asp His Val Tyr Gln His Val Phe Arg Gly Phe Ala Ala Thr
   65 70 75 80
   Leu Asp Lys Glu Thr Leu Glu Ala Leu Arg Asn His Pro Glu Val Asp
   85 90 95
   Tyr Ile Glu Gln Asp Ala Val Val Lys Ile Asn Ala Tyr Val Ser Gln
   100 105 110
   Thr Gly Ala Pro Trp Gly Leu Gly Arg Ile Ser His Lys Ala Arg Gly
   115 120 125
   Ser Thr Thr Tyr Val Tyr Asp Asp Ser Ala Gly Ala Gly Thr Cys Ser
   130 135 140
   Tyr Val Ile Asp Thr Gly Val Asp Ala Thr His Pro Asp Phe Glu Gly
   145 150 155 160
   Arg Ala Thr Leu Leu Arg Ser Phe Val Ser Gly Gln Asn Thr Asp Gly
   165 170 175
   Asn Gly His Gly Thr His Val Ser Gly Thr Ile Gly Ser Arg Thr Tyr
   180 185 190
   Gly Val Ala Lys Lys Thr Gln Ile Tyr Gly Val Lys Val Leu Asp Asn
   195 200 205
   Ser Gly Ser Gly Ser Phe Ser Thr Val Ile Ala Gly Met Asp Tyr Val
       210                 215                 220
   Ala Ser Asp Ser Gln Thr Arg Asn Cys Pro Asn Gly Ser Val Ala Asn
   225                 230                 235                 240
   Met Ser Leu Gly Gly Gly Tyr Thr Ala Ser Val Asn Gln Ala Ala Ala
                   245                 250                 255
   Arg Leu Ile Gln Ala Gly Val Phe Leu Ala Val Ala Ala Gly Asn Asp
               260                 265                 270
   Gly Val Asp Ala Arg Asn Thr Ser Pro Ala Ser Glu Pro Thr Val Cys
           275                 280                 285
   Thr Val Gly Ala Ser Thr Ser Ser Asp Ala Arg Ala Ser Phe Ser Asn
       290                 295                 300
   Tyr Gly Ser Val Val Asp Ile Phe Ala Pro Gly Gln Asp Ile Leu Ser
   305                 310                 315                 320
   Thr Trp Pro Asn Arg Gln Thr Asn Thr Ile Ser Gly Thr Ser Met Ala
                   325                 330                 335
   Thr Pro His Ile Val Gly Leu Gly Ala Tyr Leu Ala Gly Leu Glu Gly
               340                 345                 350
   Phe Ser Asp Pro Gln Ala Leu Cys Ala Arg Ile Gln Ser Leu Ala Asn
           355                 360                 365
   Arg Asn Leu Leu Ser Gly Ile Pro Ser Gly Thr Ile Asn Ala Ile Ala
       370                 375                 380
   Phe Asn Gly Asn Pro Ser Gly
   385                 390
<210> 28
   <211> 387
   <212> PRT
   <213> Trichoderma reesei
<400> 28
   Met Gly Leu Val Thr Asn Pro Phe Ala Lys Asn Ile Ile Pro Asn Arg
   1               5                   10                  15
   Tyr Ile Val Val Tyr Asn Asn Ser Phe Gly Glu Glu Ala Ile Ser Ala
               20                  25                  30
   Lys Gln Ala Gln Phe Ala Ala Lys Ile Ala Lys Arg Asn Leu Gly Lys
           35                  40                  45
   Arg Gly Leu Phe Gly Asn Glu Leu Ser Thr Ala Ile His Ser Phe Ser
       50                  55                  60
   Met His Thr Trp Arg Ala Met Ala Leu Asp Ala Asp Asp Ile Met Ile
   65                  70                  75                  80
   Lys Asp Ile Phe Asp Ala Glu Glu Val Ala Tyr Ile Glu Ala Asp Thr
                   85                  90                  95
   Lys Val Gln His Ala Ala Leu Val Ala Gln Thr Asn Ala Ala Pro Gly
               100                 105                 110
   Leu Ile Arg Leu Ser Asn Lys Ala Val Gly Gly Gln Asn Tyr Ile Phe
           115                 120                 125
   Asp Asn Ser Ala Gly Ser Asn Ile Thr Ala Tyr Val Val Asp Thr Gly
       130                 135                 140
   Ile Arg Ile Thr His Ser Glu Phe Glu Gly Arg Ala Thr Phe Gly Ala
   145                 150                 155                 160
   Asn Phe Val Asn Asp Asp Thr Asp Glu Asn Gly His Gly Ser His Val
                   165                 170                 175
   Ala Gly Thr Ile Gly Gly Ala Thr Phe Gly Val Ala Lys Asn Val Glu
               180                 185                 190
   Leu Val Ala Val Lys Val Leu Asp Ala Asp Gly Ser Gly Ser Asn Ser
           195                 200                 205
   Gly Val Leu Asn Gly Met Gln Phe Val Val Asn Asp Val Gln Ala Lys
       210                 215                 220
   Lys Arg Ser Gly Lys Ala Val Met Asn Met Ser Leu Gly Gly Ser Phe
   225                 230                 235                 240
   Ser Thr Ala Val Asn Asn Ala Ile Thr Ala Leu Thr Asn Ala Gly Ile
                   245                 250                 255
   Val Pro Val Val Ala Ala Gly Asn Glu Asn Gln Asp Thr Ala Asn Thr
               260                 265                 270
   Ser Pro Gly Ser Ala Pro Gln Ala Ile Thr Val Gly Ala Ile Asp Ala
           275                 280                 285
   Thr Thr Asp Ile Arg Ala Gly Phe Ser Asn Phe Gly Thr Gly Val Asp
       290                 295                 300
   Ile Tyr Ala Pro Gly Val Asp Val Leu Ser Val Gly Ile Lys Ser Asp
   305                 310                 315                 320
   Ile Asp Thr Ala Val Leu Ser Gly Thr Ser Met Ala Ser Pro His Val
                   325                 330                 335
   Ala Gly Leu Ala Ala Tyr Leu Met Ala Leu Glu Gly Val Ser Asn Val
               340                 345                 350
   Asp Asp Val Ser Asn Leu Ile Lys Asn Leu Ala Ala Lys Thr Gly Ala
           355                 360                 365
   Ala Val Lys Gln Asn Ile Ala Gly Thr Thr Ser Leu Ile Ala Asn Asn
       370                 375                 380
   Gly Asn Phe
   385
<210> 29
   <211> 409
   <212> PRT
   <213> Trichoderma reesei
<400> 29
   Met Ala Ser Leu Arg Arg Leu Ala Leu Tyr Leu Gly Ala Leu Leu Pro
   1               5                   10                  15
   Ala Val Leu Ala Ala Pro Ala Val Asn Tyr Lys Leu Pro Glu Ala Val
               20                  25                  30
   Pro Asn Lys Phe Ile Val Thr Leu Lys Asp Gly Ala Ser Val Asp Thr
           35                  40                  45
   Asp Ser His Leu Thr Trp Val Lys Asp Leu His Arg Arg Ser Leu Gly
       50                  55                  60
   Lys Arg Ser Thr Ala Gly Val Glu Lys Thr Tyr Asn Ile Asp Ser Trp
   65                  70                  75                  80
   Asn Ala Tyr Ala Gly Glu Phe Asp Glu Glu Thr Val Lys Gln Ile Lys
                   85                  90                  95
   Ala Asn Pro Asp Val Ala Ser Val Glu Pro Asp Tyr Ile Met Trp Leu
               100                 105                 110
   Ser Asp Ile Val Glu Asp Lys Arg Ala Leu Thr Thr Gln Thr Gly Ala
           115                 120                 125
   Pro Trp Gly Leu Gly Thr Val Ser His Arg Thr Pro Gly Ser Thr Ser
       130                 135                 140
   Tyr Ile Tyr Asp Thr Ser Ala Gly Ser Gly Thr Phe Ala Tyr Val Val
   145                 150                 155                 160
   Asp Ser Gly Ile Asn Ile Ala His Gln Gln Phe Gly Gly Arg Ala Ser
                   165                 170                 175
   Leu Gly Tyr Asn Ala Ala Gly Gly Asp His Val Asp Thr Leu Gly His
               180                 185                 190
   Gly Thr His Val Ser Gly Thr Ile Gly Gly Ser Thr Tyr Gly Val Ala
           195                 200                 205
   Lys Gln Ala Ser Leu Ile Ser Val Lys Val Phe Gln Gly Asn Ser Ala
       210                 215                 220
   Ser Thr Ser Val Ile Leu Asp Gly Tyr Asn Trp Ala Val Asn Asp Ile
   225                 230                 235                 240
   Val Ser Arg Asn Arg Ala Ser Lys Ser Ala Ile Asn Met Ser Leu Gly
                   245                 250                 255
   Gly Pro Ala Ser Ser Thr Trp Ala Thr Ala Ile Asn Ala Ala Phe Asn
               260                 265                 270
   Lys Gly Val Leu Thr Ile Val Ala Ala Gly Asn Gly Asp Ala Leu Gly
           275                 280                 285
   Asn Pro Gln Pro Val Ser Ser Thr Ser Pro Ala Asn Val Pro Asn Ala
       290                 295                 300
   Ile Thr Val Ala Ala Leu Asp Ile Asn Trp Arg Thr Ala Ser Phe Thr
   305                 310                 315                 320
   Asn Tyr Gly Ala Gly Val Asp Val Phe Ala Pro Gly Val Asn Ile Leu
                   325                 330                 335
   Ser Ser Trp Ile Gly Ser Asn Thr Ala Thr Asn Thr Ile Ser Gly Thr
               340                 345                 350
   Ser Met Ala Thr Pro His Val Val Gly Leu Ala Leu Tyr Leu Gln Ala
   355 360 365
   Leu Glu Gly Leu Ser Thr Pro Thr Ala Val Thr Asn Arg Ile Lys Ala
   370 375 380
   Leu Ala Thr Thr Gly Arg Val Thr Gly Ser Leu Asn Gly Ser Pro Asn
   385 390 395 400
   Thr Leu Ile Phe Asn Gly Asn Ser Ala
   405
<210> 30
   <211> 555
   <212> PRT
   <213> Trichoderma reesei
<400> 30
   Met Arg Ala Cys Leu Leu Phe Leu Gly Ile Thr Ala Leu Ala Thr Ala
   1               5                   10                  15
   Ile Pro Ala Leu Lys Pro Pro His Gly Ser Pro Asp Arg Ala His Thr
               20                  25                  30
   Thr Gln Leu Ala Lys Val Ser Ile Ala Leu Gln Pro Glu Cys Arg Glu
           35                  40                  45
   Leu Leu Glu Gln Ala Leu His His Leu Ser Asp Pro Ser Ser Pro Arg
       50                  55                  60
   Tyr Gly Arg Tyr Leu Gly Arg Glu Glu Ala Lys Ala Leu Leu Arg Pro
   65                  70                  75                  80
   Arg Arg Glu Ala Thr Ala Ala Val Lys Arg Trp Leu Ala Arg Ala Gly
                   85                  90                  95
   Val Pro Ala His Asp Val Leu Thr Asp Gly Gln Phe Ile His Val Arg
               100                 105                 110
   Thr Leu Ala Glu Lys Ala Gln Ala Leu Leu Gly Phe Glu Tyr Asn Ser
           115                 120                 125
   Thr Leu Gly Ser Gln Thr Ile Ala Ile Ser Thr Leu Pro Gly Lys Ile
       130                 135                 140
   Arg Lys His Val Met Thr Val Gln Tyr Val Pro Leu Trp Thr Glu Ala
   145                 150                 155                 160
   Asp Trp Glu Glu Cys Lys Thr Ile Ile Thr Pro Ser Cys Leu Lys Arg
   165 170 175
   Leu Tyr His Val Asp Ser Tyr Arg Ala Lys Tyr Glu Ser Ser Ser Leu
   180 185 190
   Phe Gly Ile Val Gly Phe Ser Gly Gln Ala Ala Gln His Asp Glu Leu
   195 200 205
   Asp Lys Phe Leu His Asp Phe Ala Pro Tyr Ser Thr Asn Ala Asn Phe
   210 215 220
   Ser Ile Glu Ser Val Asn Gly Gly Gln Ser Pro Gln Gly Met Asn Glu
   225 230 235 240
   Pro Ala Ser Glu Ala Asn Gly Asp Val Gln Tyr Ala Val Ala Met Gly
   245 250 255
   Tyr His Val Pro Val Arg Tyr Tyr Ala Val Gly Gly Glu Asn His Asp
   260 265 270
   Ile Ile Pro Asp Leu Asp Leu Val Asp Thr Thr Glu Glu Tyr Leu Glu
   275 280 285
   Pro Phe Leu Glu Phe Ala Ser His Leu Leu Asp Leu Asp Asp Asp Glu
   290 295 300
   Leu Pro Arg Val Val Ser Ile Ser Tyr Gly Ala Asn Glu Gln Leu Phe
   305 310 315 320
   Pro Arg Ser Tyr Ala His Gln Val Cys Asp Met Phe Gly Gln Leu Gly
   325 330 335
   Ala Arg Gly Val Ser Ile Val Val Ala Ala Gly Asp Leu Gly Pro Gly
   340 345 350
   Val Ser Cys Gln Ser Asn Asp Gly Ser Ala Arg Pro Lys Phe Ile Pro
   355 360 365
   Ser Phe Pro Ala Thr Cys Pro Tyr Val Thr Ser Val Gly Ser Thr Arg
   370 375 380
   Gly Ile Met Pro Glu Val Ala Ala Ser Phe Ser Ser Gly Gly Phe Ser
   385 390 395 400
   Asp Tyr Phe Ala Arg Pro Ala Trp Gln Asp Arg Ala Val Gly Ala Tyr
   405 410 415
   Leu Gly Ala His Gly Glu Glu Trp Glu Gly Phe Tyr Asn Pro Ala Gly
   420 425 430
   Arg Gly Phe Pro Asp Val Ala Ala Gln Gly Val Asn Phe Arg Phe Arg
   435 440 445
   Ala His Gly Asn Glu Ser Leu Ser Ser Gly Thr Ser Leu Ser Ser Pro
   450 455 460
   Val Phe Ala Ala Leu Ile Ala Leu Leu Asn Asp His Arg Ser Lys Ser
   465 470 475 480
   Gly Met Pro Pro Met Gly Phe Leu Asn Pro Trp Ile Tyr Thr Val Gly
   485 490 495
   Ser His Ala Phe Thr Asp Ile Ile Glu Ala Arg Ser Glu Gly Cys Pro
   500 505 510
   Gly Gln Ser Val Glu Tyr Leu Ala Ser Pro Tyr Ile Pro Asn Ala Gly
   515 520 525
   Trp Ser Ala Val Pro Gly Trp Asp Pro Val Thr Gly Trp Gly Thr Pro
   530 535 540
   Leu Phe Asp Arg Met Leu Asn Leu Ser Leu Val
   545 550 555
<210> 31
   <211> 388
   <212> PRT
   <213> Trichoderma reesei
<400> 31
   Met Ala Trp Leu Lys Lys Leu Ala Leu Val Leu Leu Ala Ile Val Pro
   1 5 10 15
   Tyr Ala Thr Ala Ser Pro Ala Leu Ser Pro Arg Ser Arg Glu Ile Leu
   20 25 30
   Ser Leu Glu Asp Leu Glu Ser Glu Asp Lys Tyr Val Ile Gly Leu Lys
   35 40 45
   Gln Gly Leu Ser Pro Thr Asp Leu Lys Lys His Leu Leu Arg Val Ser
   50 55 60
   Ala Val Gln Tyr Arg Asn Lys Asn Ser Thr Phe Glu Gly Gly Thr Gly
   65 70 75 80
   Val Lys Arg Thr Tyr Ala Ile Gly Asp Tyr Arg Ala Tyr Thr Ala Val
                   85                  90                  95
   Leu Asp Arg Asp Thr Val Arg Glu Ile Trp Asn Asp Thr Leu Glu Lys
               100                 105                 110
   Pro Pro Trp Gly Leu Ala Thr Leu Ser Asn Lys Lys Pro His Gly Phe
           115                 120                 125
   Leu Tyr Arg Tyr Asp Lys Ser Ala Gly Glu Gly Thr Phe Ala Tyr Val
       130                 135                 140
   Leu Asp Thr Gly Ile Asn Ser Lys His Val Asp Phe Glu Gly Arg Ala
   145                 150                 155                 160
   Tyr Met Gly Phe Ser Pro Pro Lys Thr Glu Pro Thr Asp Ile Asn Gly
                   165                 170                 175
   His Gly Thr His Val Ala Gly Ile Ile Gly Gly Lys Thr Phe Gly Val
               180                 185                 190
   Ala Lys Lys Thr Gln Leu Ile Gly Val Lys Val Phe Leu Asp Asp Glu
           195                 200                 205
   Ala Thr Thr Ser Thr Leu Met Glu Gly Leu Glu Trp Ala Val Asn Asp
       210                 215                 220
   Ile Thr Thr Lys Gly Arg Gln Gly Arg Ser Val Ile Asn Met Ser Leu
   225                 230                 235                 240
   Gly Gly Pro Tyr Ser Gln Ala Leu Asn Asp Ala Ile Asp His Ile Ala
                   245                 250                 255
   Asp Met Gly Ile Leu Pro Val Ala Ala Ala Gly Asn Lys Gly Ile Pro
               260                 265                 270
   Ala Thr Phe Ile Ser Pro Ala Ser Ala Asp Lys Ala Met Thr Val Gly
           275                 280                 285
   Ala Ile Asn Ser Asp Trp Gln Glu Thr Asn Phe Ser Asn Phe Gly Pro
       290                 295                 300
   Gln Val Asn Ile Leu Ala Pro Gly Glu Asp Val Leu Ser Ala Tyr Val
   305                 310                 315                 320
   Ser Thr Asn Thr Ala Thr Arg Val Leu Ser Gly Thr Ser Met Ala Ala
                   325                 330                 335
   Pro His Val Ala Gly Leu Ala Leu Tyr Leu Met Ala Leu Glu Glu Phe
   340 345 350
   Asp Ser Thr Gln Lys Leu Thr Asp Arg Ile Leu Gln Leu Gly Met Lys
   355 360 365
   Asn Lys Val Val Asn Leu Met Thr Asp Ser Pro Asn Leu Ile Ile His
   370 375 380
   Asn Asn Val Lys
   385
<210> 32
   <211> 256
   <212> PRT
   <213> Trichoderma reesei
<400> 32
   Met Phe Ile Ala Gly Val Ala Leu Ser Ala Leu Leu Cys Ala Asp Thr
   1 5 10 15
   Val Leu Ala Gly Val Ala Gln Asp Arg Gly Leu Ala Ala Arg Leu Ala
   20 25 30
   Arg Arg Ala Gly Arg Arg Ser Ala Pro Phe Arg Asn Asp Thr Ser His
   35 40 45
   Ala Thr Val Gln Ser Asn Trp Gly Gly Ala Ile Leu Glu Gly Ser Gly
   50 55 60
   Phe Thr Ala Ala Ser Ala Thr Val Asn Val Pro Arg Gly Gly Gly Gly
   65 70 75 80
   Ser Asn Ala Ala Gly Ser Ala Trp Val Gly Ile Asp Gly Ala Ser Cys
   85 90 95
   Gln Thr Ala Ile Leu Gln Thr Gly Phe Asp Trp Tyr Gly Asp Gly Thr
   100 105 110
   Tyr Asp Ala Trp Tyr Glu Trp Tyr Pro Glu Phe Ala Ala Asp Phe Ser
   115 120 125
   Gly Ile Asp Ile Arg Gln Gly Asp Gln Ile Ala Met Ser Val Val Ala
   130 135 140
   Thr Ser Leu Thr Gly Gly Ser Ala Thr Leu Glu Asn Leu Ser Thr Gly
   145 150 155 160
   Gln Lys Val Thr Gln Asn Phe Asn Arg Val Thr Ala Gly Ser Leu Cys
                   165                 170                 175
   Glu Thr Ser Ala Glu Phe Ile Ile Glu Asp Phe Glu Glu Cys Asn Ser
               180                 185                 190
   Asn Gly Ser Asn Cys Gln Pro Val Pro Phe Ala Ser Phe Ser Pro Ala
           195                 200                 205
   Ile Thr Phe Ser Ser Ala Thr Ala Thr Arg Ser Gly Arg Ser Val Ser
       210                 215                 220
   Leu Ser Gly Ala Glu Ile Thr Glu Val Ile Val Asn Asn Gln Asp Leu
   225                 230                 235                 240
   Thr Arg Cys Ser Val Ser Gly Ser Ser Thr Leu Thr Cys Ser Tyr Val
                   245                 250                 255
<210> 33
   <211> 236
   <212> PRT
   <213> Trichoderma reesei
<400> 33
   Met Asp Ala Ile Arg Ala Arg Ser Ala Ala Arg Arg Ser Asn Arg Phe
   1               5                   10                  15
   Gln Ala Gly Ser Ser Lys Asn Val Asn Gly Thr Ala Asp Val Glu Ser
               20                  25                  30
   Thr Asn Trp Ala Gly Ala Ala Ile Thr Thr Ser Gly Val Thr Glu Val
           35                  40                  45
   Ser Gly Thr Phe Thr Val Pro Arg Pro Ser Val Pro Ala Gly Gly Ser
       50                  55                  60
   Ser Arg Glu Glu Tyr Cys Gly Ala Ala Trp Val Gly Ile Asp Gly Tyr
   65                  70                  75                  80
   Ser Asp Ala Asp Leu Ile Gln Thr Gly Val Leu Trp Cys Val Glu Asp
                   85                  90                  95
   Gly Glu Tyr Leu Tyr Glu Ala Trp Tyr Glu Tyr Leu Pro Ala Ala Leu
               100                 105                 110
   Val Glu Tyr Ser Gly Ile Ser Val Thr Ala Gly Ser Val Val Thr Val
           115                 120                 125
   Thr Ala Thr Lys Thr Gly Thr Asn Ser Gly Val Thr Thr Leu Thr Ser
       130                 135                 140
   Gly Gly Lys Thr Val Ser His Thr Phe Ser Arg Gln Asn Ser Pro Leu
   145                 150                 155                 160
   Pro Gly Thr Ser Ala Glu Trp Ile Val Glu Asp Phe Thr Ser Gly Ser
                   165                 170                 175
   Ser Leu Val Pro Phe Ala Asp Phe Gly Ser Val Thr Phe Thr Gly Ala
               180                 185                 190
   Thr Ala Val Val Asn Gly Ala Thr Val Thr Ala Gly Gly Asp Ser Pro
           195                 200                 205
   Val Ile Ile Asp Leu Glu Asp Ser Arg Gly Asp Ile Leu Thr Ser Thr
       210                 215                 220
   Thr Val Ser Gly Ser Thr Val Thr Val Glu Tyr Glu
   225                 230                 235
<210> 34
   <211> 612
   <212> PRT
   <213> Trichoderma reesei
<400> 34
   Met Ala Lys Leu Ser Thr Leu Arg Leu Ala Ser Leu Leu Ser Leu Val
   1               5                   10                  15
   Ser Val Gln Val Ser Ala Ser Val His Leu Leu Glu Ser Leu Glu Lys
               20                  25                  30
   Leu Pro His Gly Trp Lys Ala Ala Glu Thr Pro Ser Pro Ser Ser Gln
           35                  40                  45
   Ile Val Leu Gln Val Ala Leu Thr Gln Gln Asn Ile Asp Gln Leu Glu
       50                  55                  60
   Ser Arg Leu Ala Ala Val Ser Thr Pro Thr Ser Ser Thr Tyr Gly Lys
   65                  70                  75                  80
   Tyr Leu Asp Val Asp Glu Ile Asn Ser Ile Phe Ala Pro Ser Asp Ala
                   85                  90                  95
   Ser Ser Ser Ala Val Glu Ser Trp Leu Gln Ser His Gly Val Thr Ser
               100                 105                 110
   Tyr Thr Lys Gln Gly Ser Ser Ile Trp Phe Gln Thr Asn Ile Ser Thr
   115 120 125
   Ala Asn Ala Met Leu Ser Thr Asn Phe His Thr Tyr Ser Asp Leu Thr
   130 135 140
   Gly Ala Lys Lys Val Arg Thr Leu Lys Tyr Ser Ile Pro Glu Ser Leu
   145 150 155 160
   Ile Gly His Val Asp Leu Ile Ser Pro Thr Thr Tyr Phe Gly Thr Thr
   165 170 175
   Lys Ala Met Arg Lys Leu Lys Ser Ser Gly Val Ser Pro Ala Ala Asp
   180 185 190
   Ala Leu Ala Ala Arg Gln Glu Pro Ser Ser Cys Lys Gly Thr Leu Val
   195 200 205
   Phe Glu Gly Glu Thr Phe Asn Val Phe Gln Pro Asp Cys Leu Arg Thr
   210 215 220
   Glu Tyr Ser Val Asp Gly Tyr Thr Pro Ser Val Lys Ser Gly Ser Arg
   225 230 235 240
   Ile Gly Phe Gly Ser Phe Leu Asn Glu Ser Ala Ser Phe Ala Asp Gln
   245 250 255
   Ala Leu Phe Glu Lys His Phe Asn Ile Pro Ser Gln Asn Phe Ser Val
   260 265 270
   Val Leu Ile Asn Gly Gly Thr Asp Leu Pro Gln Pro Pro Ser Asp Ala
   275 280 285
   Asn Asp Gly Glu Ala Asn Leu Asp Ala Gln Thr Ile Leu Thr Ile Ala
   290 295 300
   His Pro Leu Pro Ile Thr Glu Phe Ile Thr Ala Gly Ser Pro Pro Tyr
   305 310 315 320
   Phe Pro Asp Pro Val Glu Pro Ala Gly Thr Pro Asn Glu Asn Glu Pro
   325 330 335
   Tyr Leu Gln Tyr Tyr Glu Phe Leu Leu Ser Lys Ser Asn Ala Glu Ile
   340 345 350
   Pro Gln Val Ile Thr Asn Ser Tyr Gly Asp Glu Glu Gln Thr Val Pro
   355 360 365
   Arg Ser Tyr Ala Val Arg Val Cys Asn Leu Ile Gly Leu Leu Gly Leu
   370 375 380
   Arg Gly Ile Ser Val Leu His Ser Ser Gly Asp Glu Gly Val Gly Ala
   385 390 395 400
   Ser Cys Val Ala Thr Asn Ser Thr Thr Pro Gln Phe Asn Pro Ile Phe
   405 410 415
   Pro Ala Thr Cys Pro Tyr Val Thr Ser Val Gly Gly Thr Val Ser Phe
   420 425 430
   Asn Pro Glu Val Ala Trp Ala Gly Ser Ser Gly Gly Phe Ser Tyr Tyr
   435 440 445
   Phe Ser Arg Pro Trp Tyr Gln Gln Glu Ala Val Gly Thr Tyr Leu Glu
   450 455 460
   Lys Tyr Val Ser Ala Glu Thr Lys Lys Tyr Tyr Gly Pro Tyr Val Asp
   465 470 475 480
   Phe Ser Gly Arg Gly Phe Pro Asp Val Ala Ala His Ser Val Ser Pro
   485 490 495
   Asp Tyr Pro Val Phe Gln Gly Gly Glu Leu Thr Pro Ser Gly Gly Thr
   500 505 510
   Ser Ala Ala Ser Pro Val Val Ala Ala Ile Val Ala Leu Leu Asn Asp
   515 520 525
   Ala Arg Leu Arg Glu Gly Lys Pro Thr Leu Gly Phe Leu Asn Pro Leu
   530 535 540
   Ile Tyr Leu His Ala Ser Lys Gly Phe Thr Asp Ile Thr Ser Gly Gln
   545 550 555 560
   Ser Glu Gly Cys Asn Gly Asn Asn Thr Gln Thr Gly Ser Pro Leu Pro
   565 570 575
   Gly Ala Gly Phe Ile Ala Gly Ala His Trp Asn Ala Thr Lys Gly Trp
   580 585 590
   Asp Pro Thr Thr Gly Phe Gly Val Pro Asn Leu Lys Lys Leu Leu Ala
   595 600 605
   Leu Val Arg Phe
   610
<210> 35
   <211> 477
   <212> PRT
   <213> Trichoderma reesei
<400> 35
   Met Arg Phe Val Gln Tyr Val Ser Leu Ala Gly Leu Phe Ala Ala Ala
   1               5                   10                  15
   Thr Val Ser Ala Gly Val Val Thr Val Pro Phe Glu Lys Arg Asn Leu
               20                  25                  30
   Asn Pro Asp Phe Ala Pro Ser Leu Leu Arg Arg Asp Gly Ser Val Ser
           35                  40                  45
   Leu Asp Ala Ile Asn Asn Leu Thr Gly Gly Gly Tyr Tyr Ala Gln Phe
       50                  55                  60
   Ser Val Gly Thr Pro Pro Gln Lys Leu Ser Phe Leu Leu Asp Thr Gly
   65                  70                  75                  80
   Ser Ser Asp Thr Trp Val Asn Ser Val Thr Ala Asp Leu Cys Thr Asp
                   85                  90                  95
   Glu Phe Thr Gln Gln Thr Val Gly Glu Tyr Cys Phe Arg Gln Phe Asn
               100                 105                 110
   Pro Arg Arg Ser Ser Ser Tyr Lys Ala Ser Thr Glu Val Phe Asp Ile
           115                 120                 125
   Thr Tyr Leu Asp Gly Arg Arg Ile Arg Gly Asn Tyr Phe Thr Asp Thr
       130                 135                 140
   Val Thr Ile Asn Gln Ala Asn Ile Thr Gly Gln Lys Ile Gly Leu Ala
   145                 150                 155                 160
   Leu Gln Ser Val Arg Gly Thr Gly Ile Leu Gly Leu Gly Phe Arg Glu
                   165                 170                 175
   Asn Glu Ala Ala Asp Thr Lys Tyr Pro Thr Val Ile Asp Asn Leu Val
               180                 185                 190
   Ser Gln Lys Val Ile Pro Val Pro Ala Phe Ser Leu Tyr Leu Asn Asp
           195                 200                 205
   Leu Gln Thr Ser Gln Gly Ile Leu Leu Phe Gly Gly Val Asp Thr Asp
       210                 215                 220
   Lys Phe His Gly Gly Leu Ala Thr Leu Pro Leu Gln Ser Leu Pro Pro
   225                 230                 235                 240
   Ser Ile Ala Glu Thr Gln Asp Ile Val Met Tyr Ser Val Asn Leu Asp
                   245                 250                 255
   Gly Phe Ser Ala Ser Asp Val Asp Thr Pro Asp Val Ser Ala Lys Ala
               260                 265                 270
   Val Leu Asp Ser Gly Ser Thr Ile Thr Leu Leu Pro Asp Ala Val Val
           275                 280                 285
   Gln Glu Leu Phe Asp Glu Tyr Asp Val Leu Asn Ile Gln Gly Leu Pro
       290                 295                 300
   Val Pro Phe Ile Asp Cys Ala Lys Ala Asn Ile Lys Asp Ala Thr Phe
   305                 310                 315                 320
   Asn Phe Lys Phe Asp Gly Lys Thr Ile Lys Val Pro Ile Asp Glu Met
                   325                 330                 335
   Val Leu Asn Asn Leu Ala Ala Ala Ser Asp Glu Ile Met Ser Asp Pro
               340                 345                 350
   Ser Leu Ser Lys Phe Phe Lys Gly Trp Ser Gly Val Cys Thr Phe Gly
           355                 360                 365
   Met Gly Ser Thr Lys Thr Phe Gly Ile Gln Ser Asp Glu Phe Val Leu
       370                 375                 380
   Leu Gly Asp Thr Phe Leu Arg Ser Ala Tyr Val Val Tyr Asp Leu Gln
   385                 390                 395                 400
   Asn Lys Gln Ile Gly Ile Ala Gln Ala Thr Leu Asn Ser Thr Ser Ser
                   405                 410                 415
   Thr Ile Val Glu Phe Lys Ala Gly Ser Lys Thr Ile Pro Gly Pro Ala
               420                 425                 430
   Ser Thr Gly Asp Asp Ser Asp Asp Ser Ser Asp Asp Ser Asp Glu Asp
           435                 440                 445
   Ser Ala Gly Ala Ala Leu His Pro Thr Phe Ser Ile Ala Leu Ala Gly
       450                 455                 460
   Thr Leu Phe Thr Ala Val Ser Met Met Met Ser Val Leu
   465 470 475
<210> 36
   <211> 1263
   <212> DNA
   <213> Trichoderma reesei
<400> 36
   atggcgtcac tcatcaaaac tgccgtggac attgccaacg gccgccatgc gctgtccaga 60
   tatgtcatct ttgggctctg gcttgcggat gcggtgctgt gcgggctgat tatctggaaa 120
   gtgccttata cggaaatcga ctgggtcgcc tacatggagc aagtcaccca gttcgtccac 180
   ggagagcgag actaccccaa gatggagggc ggcacagggc ccctggtgta tcccgcggcc 240
   catgtgtaca tctacacagg gctctactac ctgacgaaca agggcaccga catcctgctg 300
   gcgcagcagc tctttgccgt gctctacatg gctactctgg cggtcgtcat gacatgctac 360
   tccaaggcca aggtcccgcc gtacatcttc ccgcttctca tcctctccaa aagacttcac 420
   agcgtcttcg tcctgagatg cttcaacgac tgcttcgccg ccttcttcct ctggctctgc 480
   atcttcttct tccagaggcg agagtggacc atcggagctc tcgcatacag catcggcctg 540
   ggcgtcaaaa tgtcgctgct actggttctc cccgccgtgg tcatcgtcct ctacctcggc 600
   cgcggcttca agggcgccct gcggctgctc tggctcatgg tgcaggtcca gctcctcctc 660
   gccataccct tcatcacgac aaattggcgc ggctacctcg gccgtgcatt cgagctctcg 720
   aggcagttca agtttgaatg gacagtcaat tggcgcatgc tgggcgagga tctgttcctc 780
   agccggggct tctctatcac gctactggca tttcacgcca tcttcctcct cgcctttatc 840
   ctcggccggt ggctgaagat tagggaacgg accgtactcg ggatgatccc ctatgtcatc 900
   cgattcagat cgccctttac cgagcaggaa gagcgcgcca tctccaaccg cgtcgtcacg 960
   cccggctatg tcatgtccac catcttgtcg gccaacgtgg tgggactgct gtttgcccgg 1020
   tctctgcact accagttcta tgcatatctg gcgtgggcga ccccctatct cctgtggacg 1080
   gcctgcccca atcttttggt ggtggccccc ctctgggcgg cgcaagaatg ggcctggaac 1140
   gtcttcccca gcacgcctct tagctcgagc gtcgtggtga gcgtgctggc cgtgacggtg 1200
   gccatggcgt ttgcaggttc aaatccgcag ccacgtgaaa catcgaagcc gaagcagcac 1260
   taa 1263
<210> 37
   <211> 420
   <212> PRT
   <213> Trichoderma reesei
<400> 37
   Met Ala Ser Leu Ile Lys Thr Ala Val Asp Ile Ala Asn Gly Arg His
   1               5                   10                  15
   Ala Leu Ser Arg Tyr Val Ile Phe Gly Leu Trp Leu Ala Asp Ala Val
               20                  25                  30
   Leu Cys Gly Leu Ile Ile Trp Lys Val Pro Tyr Thr Glu Ile Asp Trp
           35                  40                  45
   Val Ala Tyr Met Glu Gln Val Thr Gln Phe Val His Gly Glu Arg Asp
       50                  55                  60
   Tyr Pro Lys Met Glu Gly Gly Thr Gly Pro Leu Val Tyr Pro Ala Ala
   65                  70                  75                  80
   His Val Tyr Ile Tyr Thr Gly Leu Tyr Tyr Leu Thr Asn Lys Gly Thr
                   85                  90                  95
   Asp Ile Leu Leu Ala Gln Gln Leu Phe Ala Val Leu Tyr Met Ala Thr
               100                 105                 110
   Leu Ala Val Val Met Thr Cys Tyr Ser Lys Ala Lys Val Pro Pro Tyr
           115                 120                 125
   Ile Phe Pro Leu Leu Ile Leu Ser Lys Arg Leu His Ser Val Phe Val
       130                 135                 140
   Leu Arg Cys Phe Asn Asp Cys Phe Ala Ala Phe Phe Leu Trp Leu Cys
   145                 150                 155                 160
   Ile Phe Phe Phe Gln Arg Arg Glu Trp Thr Ile Gly Ala Leu Ala Tyr
                   165                 170                 175
   Ser Ile Gly Leu Gly Val Lys Met Ser Leu Leu Leu Val Leu Pro Ala
               180                 185                 190
   Val Val Ile Val Leu Tyr Leu Gly Arg Gly Phe Lys Gly Ala Leu Arg
           195                 200                 205
   Leu Leu Trp Leu Met Val Gln Val Gln Leu Leu Leu Ala Ile Pro Phe
       210                 215                 220
   Ile Thr Thr Asn Trp Arg Gly Tyr Leu Gly Arg Ala Phe Glu Leu Ser
   225                 230                 235                 240
   Arg Gln Phe Lys Phe Glu Trp Thr Val Asn Trp Arg Met Leu Gly Glu
                   245                 250                 255
   Asp Leu Phe Leu Ser Arg Gly Phe Ser Ile Thr Leu Leu Ala Phe His
   260 265 270
   Ala Ile Phe Leu Leu Ala Phe Ile Leu Gly Arg Trp Leu Lys Ile Arg
   275 280 285
   Glu Arg Thr Val Leu Gly Met Ile Pro Tyr Val Ile Arg Phe Arg Ser
   290 295 300
   Pro Phe Thr Glu Gln Glu Glu Arg Ala Ile Ser Asn Arg Val Val Thr
   305 310 315 320
   Pro Gly Tyr Val Met Ser Thr Ile Leu Ser Ala Asn Val Val Gly Leu
   325 330 335
   Leu Phe Ala Arg Ser Leu His Tyr Gln Phe Tyr Ala Tyr Leu Ala Trp
   340 345 350
   Ala Thr Pro Tyr Leu Leu Trp Thr Ala Cys Pro Asn Leu Leu Val Val
   355 360 365
   Ala Pro Leu Trp Ala Ala Gln Glu Trp Ala Trp Asn Val Phe Pro Ser
   370 375 380
   Thr Pro Leu Ser Ser Ser Val Val Val Ser Val Leu Ala Val Thr Val
   385 390 395 400
   Ala Met Ala Phe Ala Gly Ser Asn Pro Gln Pro Arg Glu Thr Ser Lys
   405 410 415
   Pro Lys Gln His
   420
<210> 38
   <211> 445
   <212> PRT
   <213> Homo sapiens
<400> 38
   Met Leu Lys Lys Gln Ser Ala Gly Leu Val Leu Trp Gly Ala Ile Leu
   1 5 10 15
   Phe Val Ala Trp Asn Ala Leu Leu Leu Leu Phe Phe Trp Thr Arg Pro
   20 25 30
   Ala Pro Gly Arg Pro Pro Ser Val Ser Ala Leu Asp Gly Asp Pro Ala
   35 40 45
   Ser Leu Thr Arg Glu Val Ile Arg Leu Ala Gln Asp Ala Glu Val Glu
       50                  55                  60
   Leu Glu Arg Gln Arg Gly Leu Leu Gln Gln Ile Gly Asp Ala Leu Ser
   65                  70                  75                  80
   Ser Gln Arg Gly Arg Val Pro Thr Ala Ala Pro Pro Ala Gln Pro Arg
                   85                  90                  95
   Val Pro Val Thr Pro Ala Pro Ala Val Ile Pro Ile Leu Val Ile Ala
               100                 105                 110
   Cys Asp Arg Ser Thr Val Arg Arg Cys Leu Asp Lys Leu Leu His Tyr
           115                 120                 125
   Arg Pro Ser Ala Glu Leu Phe Pro Ile Ile Val Ser Gln Asp Cys Gly
       130                 135                 140
   His Glu Glu Thr Ala Gln Ala Ile Ala Ser Tyr Gly Ser Ala Val Thr
   145                 150                 155                 160
   His Ile Arg Gln Pro Asp Leu Ser Ser Ile Ala Val Pro Pro Asp His
                   165                 170                 175
   Arg Lys Phe Gln Gly Tyr Tyr Lys Ile Ala Arg His Tyr Arg Trp Ala
               180                 185                 190
   Leu Gly Gln Val Phe Arg Gln Phe Arg Phe Pro Ala Ala Val Val Val
           195                 200                 205
   Glu Asp Asp Leu Glu Val Ala Pro Asp Phe Phe Glu Tyr Phe Arg Ala
       210                 215                 220
   Thr Tyr Pro Leu Leu Lys Ala Asp Pro Ser Leu Trp Cys Val Ser Ala
   225                 230                 235                 240
   Trp Asn Asp Asn Gly Lys Glu Gln Met Val Asp Ala Ser Arg Pro Glu
                   245                 250                 255
   Leu Leu Tyr Arg Thr Asp Phe Phe Pro Gly Leu Gly Trp Leu Leu Leu
               260                 265                 270
   Ala Glu Leu Trp Ala Glu Leu Glu Pro Lys Trp Pro Lys Ala Phe Trp
           275                 280                 285
   Asp Asp Trp Met Arg Arg Pro Glu Gln Arg Gln Gly Arg Ala Cys Ile
       290                 295                 300
   Arg Pro Glu Ile Ser Arg Thr Met Thr Phe Gly Arg Lys Gly Val Ser
   305                 310                 315                 320
   His Gly Gln Phe Phe Asp Gln His Leu Lys Phe Ile Lys Leu Asn Gln
                   325                 330                 335
   Gln Phe Val His Phe Thr Gln Leu Asp Leu Ser Tyr Leu Gln Arg Glu
               340                 345                 350
   Ala Tyr Asp Arg Asp Phe Leu Ala Arg Val Tyr Gly Ala Pro Gln Leu
           355                 360                 365
   Gln Val Glu Lys Val Arg Thr Asn Asp Arg Lys Glu Leu Gly Glu Val
       370                 375                 380
   Arg Val Gln Tyr Thr Gly Arg Asp Ser Phe Lys Ala Phe Ala Lys Ala
   385                 390                 395                 400
   Leu Gly Val Met Asp Asp Leu Lys Ser Gly Val Pro Arg Ala Gly Tyr
                   405                 410                 415
   Arg Gly Ile Val Thr Phe Gln Phe Arg Gly Arg Arg Val His Leu Ala
               420                 425                 430
   Pro Pro Leu Thr Trp Glu Gly Tyr Asp Pro Ser Trp Asn
           435                 440                 445
<210> 39
   <211> 447
   <212> PRT
   <213> Homo sapiens
<400> 39
   Met Arg Phe Arg Ile Tyr Lys Arg Lys Val Leu Ile Leu Thr Leu Val
   1               5                   10                  15
   Val Ala Ala Cys Gly Phe Val Leu Trp Ser Ser Asn Gly Arg Gln Arg
               20                  25                  30
   Lys Asn Glu Ala Leu Ala Pro Pro Leu Leu Asp Ala Glu Pro Ala Arg
           35                  40                  45
   Gly Ala Gly Gly Arg Gly Gly Asp His Pro Ser Val Ala Val Gly Ile
       50                  55                  60
   Arg Arg Val Ser Asn Val Ser Ala Ala Ser Leu Val Pro Ala Val Pro
   65                  70                  75                  80
   Gln Pro Glu Ala Asp Asn Leu Thr Leu Arg Tyr Arg Ser Leu Val Tyr
                   85                  90                  95
   Gln Leu Asn Phe Asp Gln Thr Leu Arg Asn Val Asp Lys Ala Gly Thr
               100                 105                 110
   Trp Ala Pro Arg Glu Leu Val Leu Val Val Gln Val His Asn Arg Pro
           115                 120                 125
   Glu Tyr Leu Arg Leu Leu Leu Asp Ser Leu Arg Lys Ala Gln Gly Ile
       130                 135                 140
   Asp Asn Val Leu Val Ile Phe Ser His Asp Phe Trp Ser Thr Glu Ile
   145                 150                 155                 160
   Asn Gln Leu Ile Ala Gly Val Asn Phe Cys Pro Val Leu Gln Val Phe
                   165                 170                 175
   Phe Pro Phe Ser Ile Gln Leu Tyr Pro Asn Glu Phe Pro Gly Ser Asp
               180                 185                 190
   Pro Arg Asp Cys Pro Arg Asp Leu Pro Lys Asn Ala Ala Leu Lys Leu
           195                 200                 205
   Gly Cys Ile Asn Ala Glu Tyr Pro Asp Ser Phe Gly His Tyr Arg Glu
       210                 215                 220
   Ala Lys Phe Ser Gln Thr Lys His His Trp Trp Trp Lys Leu His Phe
   225                 230                 235                 240
   Val Trp Glu Arg Val Lys Ile Leu Arg Asp Tyr Ala Gly Leu Ile Leu
                   245                 250                 255
   Phe Leu Glu Glu Asp His Tyr Leu Ala Pro Asp Phe Tyr His Val Phe
               260                 265                 270
   Lys Lys Met Trp Lys Leu Lys Gln Gln Glu Cys Pro Glu Cys Asp Val
           275                 280                 285
   Leu Ser Leu Gly Thr Tyr Ser Ala Ser Arg Ser Phe Tyr Gly Met Ala
       290                 295                 300
   Asp Lys Val Asp Val Lys Thr Trp Lys Ser Thr Glu His Asn Met Gly
   305                 310                 315                 320
   Leu Ala Leu Thr Arg Asn Ala Tyr Gln Lys Leu Ile Glu Cys Thr Asp
                   325                 330                 335
   Thr Phe Cys Thr Tyr Asp Asp Tyr Asn Trp Asp Trp Thr Leu Gln Tyr
   340 345 350
   Leu Thr Val Ser Cys Leu Pro Lys Phe Trp Lys Val Leu Val Pro Gln
   355 360 365
   Ile Pro Arg Ile Phe His Ala Gly Asp Cys Gly Met His His Lys Lys
   370 375 380
   Thr Cys Arg Pro Ser Thr Gln Ser Ala Gln Ile Glu Ser Leu Leu Asn
   385 390 395 400
   Asn Asn Lys Gln Tyr Met Phe Pro Glu Thr Leu Thr Ile Ser Glu Lys
   405 410 415
   Phe Thr Val Val Ala Ile Ser Pro Pro Arg Lys Asn Gly Gly Trp Gly
   420 425 430
   Asp Ile Arg Asp His Glu Leu Cys Lys Ser Tyr Arg Arg Leu Gln
   435 440 445
<210> 40
   <211> 85
   <212> PRT
   <213> Trichoderma reesei
<400> 40
   Met Ala Ser Thr Asn Ala Arg Tyr Val Arg Tyr Leu Leu Ile Ala Phe
   1 5 10 15
   Phe Thr Ile Leu Val Phe Tyr Phe Val Ser Asn Ser Lys Tyr Glu Gly
   20 25 30
   Val Asp Leu Asn Lys Gly Thr Phe Thr Ala Pro Asp Ser Thr Lys Thr
   35 40 45
   Thr Pro Lys Pro Pro Ala Thr Gly Asp Ala Lys Asp Phe Pro Leu Ala
   50 55 60
   Leu Thr Pro Asn Asp Pro Gly Phe Asn Asp Leu Val Gly Ile Ala Pro
   65 70 75 80
   Gly Pro Arg Met Asn
   85
<210> 41
   <211> 255
   <212> DNA
   <213> Trichoderma reesei
<400> 41
   atggcgtcaa caaatgcgcg ctatgtgcgc tatctactaa tcgccttctt cacaatcctc      60
   gtcttctact ttgtctccaa ttcaaagtat gagggcgtcg atctcaacaa gggcaccttc     120
   acagctccgg attcgaccaa gacgacacca aagccgccag ccactggcga tgccaaagac     180
   tttcctctgg ccctgacgcc gaacgatcca ggcttcaacg acctcgtcgg catcgctccc     240
   ggccctcgaa tgaac                                                      255
<210> 42
   <211> 58
   <212> PRT
   <213> Homo sapiens
<400> 42
   Met Arg Phe Arg Ile Tyr Lys Arg Lys Val Leu Ile Leu Thr Leu Val
   1               5                   10                  15
   Val Ala Ala Cys Gly Phe Val Leu Trp Ser Ser Asn Gly Arg Gln Arg
               20                  25                  30
   Lys Asn Glu Ala Leu Ala Pro Pro Leu Leu Asp Ala Glu Pro Ala Arg
           35                  40                  45
   Gly Ala Gly Gly Arg Gly Gly Asp His Pro
       50                  55
<210> 43
   <211> 51
   <212> PRT
   <213> Trichoderma reesei
<400> 43
   Met Ala Ser Thr Asn Ala Arg Tyr Val Arg Tyr Leu Leu Ile Ala Phe
   1               5                   10                  15
   Phe Thr Ile Leu Val Phe Tyr Phe Val Ser Asn Ser Lys Tyr Glu Gly
               20                  25                  30
   Val Asp Leu Asn Lys Gly Thr Phe Thr Ala Pro Asp Ser Thr Lys Thr
           35                  40                  45
   Thr Pro Lys
       50
<210> 44
   <211> 52
   <212> PRT
   <213> Trichoderma reesei
<400> 44
   Met Ala Ile Ala Arg Pro Val Arg Ala Leu Gly Gly Leu Ala Ala Ile
   1               5                   10                  15
   Leu Trp Cys Phe Phe Leu Tyr Gln Leu Leu Arg Pro Ser Ser Ser Tyr
               20                  25                  30
   Asn Ser Pro Gly Asp Arg Tyr Ile Asn Phe Glu Arg Asp Pro Asn Leu
           35                  40                  45
   Asp Pro Thr Gly
       50
<210> 45
   <211> 33
   <212> PRT
   <213> Trichoderma reesei
<400> 45
   Met Leu Asn Pro Arg Arg Ala Leu Ile Ala Ala Ala Phe Ile Leu Thr
   1               5                   10                  15
   Val Phe Phe Leu Ile Ser Arg Ser His Asn Ser Glu Ser Ala Ser Thr
               20                  25                  30
   Ser
<210> 46
   <211> 84
   <212> PRT
   <213> Trichoderma reesei
<400> 46
   Met Met Pro Arg His His Ser Ser Gly Phe Ser Asn Gly Tyr Pro Arg
   1               5                   10                  15
   Ala Asp Thr Phe Glu Ile Ser Pro His Arg Phe Gln Pro Arg Ala Thr
               20                  25                  30
   Leu Pro Pro His Arg Lys Arg Lys Arg Thr Ala Ile Arg Val Gly Ile
           35                  40                  45
   Ala Val Val Val Ile Leu Val Leu Val Leu Trp Phe Gly Gln Pro Arg
       50                  55                  60
   Ser Val Ala Ser Leu Ile Ser Leu Gly Ile Leu Ser Gly Tyr Asp Asp
   65                  70                  75                  80
   Leu Lys Leu Glu
<210> 47
   <211> 55
   <212> PRT
   <213> Trichoderma reesei
<400> 47
   Met Leu Leu Pro Lys Gly Gly Leu Asp Trp Arg Ser Ala Arg Ala Gln
   1               5                   10                  15
   Ile Pro Pro Thr Arg Ala Leu Trp Asn Ala Val Thr Arg Thr Arg Phe
               20                  25                  30
   Ile Leu Leu Val Gly Ile Thr Gly Leu Ile Leu Leu Leu Trp Arg Gly
           35                  40                  45
   Val Ser Thr Ser Ala Ser Glu
       50                  55
<210> 48
   <211> 20
   <212> DNA
   <213> Artificial
<220>
   <223> Primer
<400> 48
   ccgcgttgaa cggcttccca 20
<210> 49
   <211> 24
   <212> DNA
   <213> Artificial
<220>
   <223> Primer
<400> 49
   taacttgtac gctctcagtt cgag 24
<210> 50
   <211> 20
   <212> DNA
   <213> Artificial
<220>
   <223> Primer
<400> 50
   gcgacggcga cccattagca 20
<210> 51
   <211> 19
   <212> DNA
   <213> Artificial
<220>
   <223> Primer
<400> 51
   catcctcaag gcctcagac 19
<210> 52
   <211> 20
   <212> DNA
   <213> Artificial
<220>
   <223> Primer
<400> 52
   tgcgctctca ccagcatcgc 20
<210> 53
   <211> 20
   <212> DNA
   <213> Artificial
<220>
   <223> Primer
<400> 53
   gtcctgggcg agttccgcac 20
<210> 54
   <211> 63
   <212> DNA
   <213> Artificial
<220>
   <223> Primer
<400> 54
   agatttcagt ctctcaccac tcacctgagt tgcctctctc ggtctgaagg acgtggaatg 60
   atg 63
<210> 55
   <211> 66
   <212> DNA
   <213> Artificial
<220>
   <223> Primer
<400> 55
   gcagggtgat gagctggatc accttgacgg tgttgcccat gttgagagaa gttgttggat 60
   tgatca 66
<210> 56
   <211> 63
   <212> DNA
   <213> Artificial
<220>
   <223> Primer
<400> 56
   agatttcagt ctctcaccac tcacctgagt tgcctctctc ggtctgaagg acgtggaatg 60
   atg 63
<210> 57
   <211> 66
   <212> DNA
   <213> Artificial
<220>
   <223> Primer
<400> 57
   cagagccgct atcgccgagg aggttgccct tcttgcccat gttgagagaa gttgttggat 60
   tgatca 66
<210> 58
   <211> 63
   <212> DNA
   <213> Artificial
<220>
   <223> Primer
<400> 58
   agatttcagt ctctcaccac tcacctgagt tgcctctctc ggtctgaagg acgtggaatg 60
   atg 63
<210> 59
   <211> 66
   <212> DNA
   <213> Artificial
<220>
   <223> Primer
<400> 59
   tcttgaggat gagctggacg agggtcttga aaaagcccat gttgagagaa gttgttggat 60
   tgatca 66
<210> 60
   <211> 21
   <212> DNA
   <213> Artificial
<220>
   <223> Primer
<400> 60
   agctccgtgg cgaaagcctg a 21
<210> 61
   <211> 66
   <212> DNA
   <213> Artificial
<220>
   <223> Primer
<400> 61
   cagccgcagc ctcagcctct ctcagcctca tcagccgcgg ccgccaactt tgcgtccctt 60
   gtgacg 66
<210> 62
   <211> 76
   <212> DNA
   <213> Artificial
<220>
   <223> Primer
<400> 62
   gcaacgagag cagagcagca gtagtcgatg ctaggcggcc gcgggcagta tgccggatgg 60
   ctggcttata caggca 76
<210> 63
   <211> 76
   <212> DNA
   <213> Artificial
<220>
   <223> Primer
<400> 63
   tgcctgtata agccagccat ccggcatact gcccgcggcc gcctagcatc gactactgct 60
   gctctgctct cgttgc 76
<210> 64
   <211> 20
   <212> DNA
   <213> Artificial
<220>
   <223> Primer
<400> 64
   tgcgtcgccg tctcgctcct 20
<210> 65
   <211> 20
   <212> DNA
   <213> Artificial
<220>
   <223> Primer
<400> 65
   ttaggcgacc tctttttcca 20
<210> 66
   <211> 20
   <212> DNA
   <213> Artificial
<220>
   <223> Primer
<400> 66
   cgaggaagtc tcgtgaggat 20
<210> 67
   <211> 19
   <212> DNA
   <213> Artificial
<220>
   <223> Primer
<400> 67
   cagctaaacc gacgggcca 19
<210> 68
   <211> 20
   <212> DNA
   <213> Artificial
<220>
   <223> Primer
<400> 68
   gaccgtatat ttgaaaaggg 20
<210> 69
   <211> 20
   <212> DNA
   <213> Artificial
<220>
   <223> Primer
<400> 69
   gatgttgcgc ctgggttgac 20
<210> 70
   <211> 23
   <212> DNA
   <213> Artificial
<220>
   <223> Primer
<400> 70
   taacttgtac gctctcagtt cga 23
<210> 71
   <211> 20
   <212> DNA
   <213> Artificial
<220>
   <223> Primer
<400> 71
   ccatgagctt gaacaggtaa 20
<210> 72
   <211> 20
   <212> DNA
   <213> Artificial
<220>
   <223> Primer
<400> 72
   gattgtcatg gtgtacgtga 20
<210> 73
   <211> 20
   <212> DNA
   <213> Artificial
<220>
   <223> Primer
<400> 73
   caagatggag ggcggcacag 20
<210> 74
   <211> 22
   <212> DNA
   <213> Artificial
<220>
   <223> Primer
<400> 74
   gccagtagcg tgatagagaa gc 22
<210> 75
   <211> 20
   <212> DNA
   <213> Artificial
<220>
   <223> Primer
<400> 75
   gcgtcactca tcaaaactgc 20
<210> 76
   <211> 19
   <212> DNA
   <213> Artificial
<220>
   <223> Primer
<400> 76
   cttcggcttc gatgtttca 19
<210> 77
   <211> 20
   <212> DNA
   <213> Artificial
<220>
   <223> Primer
<400> 77
   tgcgtcgccg tctcgctcct 20
<210> 78
   <211> 20
   <212> DNA
   <213> Artificial
<220>
   <223> Primer
<400> 78
   tgacgtacca gttgggatga 20
<210> 79
   <211> 20
   <212> DNA
   <213> Artificial
<220>
   <223> Primer
<400> 79
   gatgttgcgc ctgggttgac 20
<210> 80
   <211> 20
   <212> DNA
   <213> Artificial
<220>
   <223> Primer
<400> 80
   tgacgtacca gttgggatga 20
<210> 81
   <211> 20
   <212> DNA
   <213> Artificial
<220>
   <223> Primer
<400> 81
   tgcgtcgccg tctcgctcct 20
<210> 82
   <211> 20
   <212> DNA
   <213> Artificial
<220>
   <223> Primer
<400> 82
   gattgtcatg gtgtacgtga 20
<210> 83
   <211> 20
   <212> DNA
   <213> Artificial
<220>
   <223> Primer
<400> 83
   caagatggag ggcggcacag 20
<210> 84
   <211> 22
   <212> DNA
   <213> Artificial
<220>
   <223> Primer
<400> 84
   gccagtagcg tgatagagaa gc 22
<210> 85
   <211> 488
   <212> PRT
   <213> Trichoderma reesei
<400> 85
   Met Arg Ala Ser Pro Leu Ala Val Ala Gly Val Ala Leu Ala Ser Ala
   1               5                   10                  15
   Ala Gln Ala Gln Val Val Gln Phe Asp Ile Glu Lys Arg His Ala Pro
               20                  25                  30
   Arg Leu Ser Arg Arg Asp Gly Thr Ile Asp Gly Thr Leu Ser Asn Gln
           35                  40                  45
   Arg Val Gln Gly Gly Tyr Phe Ile Asn Val Gln Val Gly Ser Pro Gly
       50                  55                  60
   Gln Asn Ile Thr Leu Gln Leu Asp Thr Gly Ser Ser Asp Val Trp Val
   65                  70                  75                  80
   Pro Ser Ser Thr Ala Ala Ile Cys Thr Gln Val Ser Glu Arg Asn Pro
                   85                  90                  95
   Gly Cys Gln Phe Gly Ser Phe Asn Pro Asp Asp Ser Asp Thr Phe Asp
               100                 105                 110
   Glu Val Gly Gln Gly Leu Phe Asp Ile Thr Tyr Val Asp Gly Ser Ser
           115                 120                 125
   Ser Lys Gly Asp Tyr Phe Gln Asp Asn Phe Gln Ile Asn Gly Val Thr
       130                 135                 140
   Val Lys Asn Leu Thr Met Gly Leu Gly Leu Ser Ser Ser Ile Pro Asn
   145                 150                 155                 160
   Gly Leu Ile Gly Val Gly Tyr Met Asn Asp Glu Ala Ser Val Ser Thr
                   165                 170                 175
   Thr Arg Ser Thr Tyr Pro Asn Leu Pro Ile Val Leu Gln Gln Gln Lys
               180                 185                 190
   Leu Ile Asn Ser Val Ala Phe Ser Leu Trp Leu Asn Asp Leu Asp Ala
           195                 200                 205
   Ser Thr Gly Ser Ile Leu Phe Gly Gly Ile Asp Thr Glu Lys Tyr His
       210                 215                 220
   Gly Asp Leu Thr Ser Ile Asp Ile Ile Ser Pro Asn Gly Gly Lys Thr
   225                 230                 235                 240
   Phe Thr Glu Phe Ala Val Asn Leu Tyr Ser Val Gln Ala Thr Ser Pro
                   245                 250                 255
   Ser Gly Thr Asp Thr Leu Ser Thr Ser Glu Asp Thr Leu Ile Ala Val
               260                 265                 270
   Leu Asp Ser Gly Thr Thr Leu Thr Tyr Leu Pro Gln Asp Met Ala Glu
   275 280 285
   Glu Ala Trp Asn Glu Val Gly Ala Glu Tyr Ser Asn Glu Leu Gly Leu
   290 295 300
   Ala Val Val Pro Cys Ser Val Gly Asn Thr Asn Gly Phe Phe Ser Phe
   305 310 315 320
   Thr Phe Ala Gly Thr Asp Gly Pro Thr Ile Asn Val Thr Leu Ser Glu
   325 330 335
   Leu Val Leu Asp Leu Phe Ser Gly Gly Pro Ala Pro Arg Phe Ser Ser
   340 345 350
   Gly Pro Asn Lys Gly Gln Ser Ile Cys Glu Phe Gly Ile Gln Asn Gly
   355 360 365
   Thr Gly Ser Pro Phe Leu Leu Gly Asp Thr Phe Leu Arg Ser Ala Phe
   370 375 380
   Val Val Tyr Asp Leu Val Asn Asn Gln Ile Ala Ile Ala Pro Thr Asn
   385 390 395 400
   Phe Asn Ser Thr Arg Thr Asn Val Val Ala Phe Ala Ser Ser Gly Ala
   405 410 415
   Pro Ile Pro Ser Ala Thr Ala Ala Pro Asn Gln Ser Arg Thr Gly His
   420 425 430
   Ser Ser Ser Thr His Ser Gly Leu Ser Ala Ala Ser Gly Phe His Asp
   435 440 445
   Gly Asp Asp Glu Asn Ala Gly Ser Leu Thr Ser Val Phe Ser Gly Pro
   450 455 460
   Gly Met Ala Val Val Gly Met Thr Ile Cys Tyr Thr Leu Leu Gly Ser
   465 470 475 480
   Ala Ile Phe Gly Ile Gly Trp Leu
   485
<210> 86
   <211> 761
   <212> PRT
   <213> Trichoderma reesei
<400> 86
   Met Arg Ser Thr Leu Tyr Gly Leu Ala Ala Leu Pro Leu Ala Ala Gln
   1               5                   10                  15
   Ala Leu Glu Phe Ile Asp Asp Thr Val Ala Gln Gln Asn Gly Ile Met
               20                  25                  30
   Arg Tyr Thr Leu Thr Thr Thr Lys Gly Ala Thr Ser Lys His Leu His
           35                  40                  45
   Arg Arg Gln Asp Ser Ala Asp Leu Met Ser Gln Gln Thr Gly Tyr Phe
       50                  55                  60
   Tyr Ser Ile Gln Leu Glu Ile Gly Thr Pro Pro Gln Ala Val Ser Val
   65                  70                  75                  80
   Asn Phe Asp Thr Gly Ser Ser Glu Leu Trp Val Asn Pro Val Cys Ser
                   85                  90                  95
   Lys Ala Thr Asp Pro Ala Phe Cys Lys Thr Phe Gly Gln Tyr Asn His
               100                 105                 110
   Ser Thr Thr Phe Val Asp Ala Lys Ala Pro Gly Gly Ile Lys Tyr Gly
           115                 120                 125
   Thr Gly Phe Val Asp Phe Asn Tyr Gly Tyr Asp Tyr Val Gln Leu Gly
       130                 135                 140
   Ser Leu Arg Ile Asn Gln Gln Val Phe Gly Val Ala Thr Asp Ser Glu
   145                 150                 155                 160
   Phe Ala Ser Val Gly Ile Leu Gly Ala Gly Pro Asp Leu Ser Gly Trp
                   165                 170                 175
   Thr Ser Pro Tyr Pro Phe Val Ile Asp Asn Leu Val Lys Gln Gly Phe
               180                 185                 190
   Ile Lys Ser Arg Ala Phe Ser Leu Asp Ile Arg Gly Leu Asp Ser Asp
           195                 200                 205
   Arg Gly Ser Val Thr Tyr Gly Gly Ile Asp Ile Lys Lys Phe Ser Gly
       210                 215                 220
   Pro Leu Ala Lys Lys Pro Ile Ile Pro Ala Ala Gln Ser Pro Asp Gly
   225                 230                 235                 240
   Tyr Thr Arg Tyr Trp Val His Met Asp Gly Met Ser Ile Thr Lys Glu
                   245                 250                 255
   Asp Gly Ser Lys Phe Glu Ile Phe Asp Lys Pro Asn Gly Gln Pro Val
   260 265 270
   Leu Leu Asp Ser Gly Tyr Thr Val Ser Thr Leu Pro Gly Pro Leu Met
   275 280 285
   Asp Lys Ile Leu Glu Ala Phe Pro Ser Ala Arg Leu Glu Ser Thr Ser
   290 295 300
   Gly Asp Tyr Ile Val Asp Cys Asp Ile Ile Asp Thr Pro Gly Arg Val
   305 310 315 320
   Asn Phe Lys Phe Gly Asn Val Val Val Asp Val Glu Tyr Lys Asp Phe
   325 330 335
   Ile Trp Gln Gln Pro Asp Leu Gly Ile Cys Lys Leu Gly Val Ser Gln
   340 345 350
   Asp Asp Asn Phe Pro Val Leu Gly Asp Thr Phe Leu Arg Ala Ala Tyr
   355 360 365
   Val Val Phe Asp Trp Asp Asn Gln Glu Val His Ile Ala Ala Asn Glu
   370 375 380
   Asp Cys Gly Asp Glu Leu Ile Pro Ile Gly Ser Gly Pro Asp Ala Ile
   385 390 395 400
   Pro Ala Ser Ala Ile Gly Lys Cys Ser Pro Ser Val Lys Thr Asp Thr
   405 410 415
   Thr Thr Ser Val Ala Glu Thr Thr Ala Thr Ser Ala Ala Ala Ser Thr
   420 425 430
   Ser Glu Leu Ala Ala Thr Thr Ser Glu Ala Ala Thr Thr Ser Ser Glu
   435 440 445
   Ala Ala Thr Thr Ser Ala Ala Ala Glu Thr Thr Ser Val Pro Leu Asn
   450 455 460
   Thr Ala Pro Ala Thr Thr Gly Leu Leu Pro Thr Thr Ser His Arg Phe
   465 470 475 480
   Ser Asn Gly Thr Ala Pro Tyr Pro Ile Pro Ser Leu Ser Ser Val Ala
   485 490 495
   Ala Ala Ala Gly Ser Ser Thr Val Pro Ser Glu Ser Ser Thr Gly Ala
   500 505 510
   Ala Ala Ala Gly Thr Thr Ser Ala Ala Thr Gly Ser Gly Ser Gly Ser
   515 520 525
   Gly Ser Gly Asp Ala Thr Thr Ala Ser Ala Thr Tyr Thr Ser Thr Phe
   530 535 540
   Thr Thr Thr Asn Val Tyr Thr Val Thr Ser Cys Pro Pro Ser Val Thr
   545 550 555 560
   Asn Cys Pro Val Gly His Val Thr Thr Glu Val Val Val Ala Tyr Thr
   565 570 575
   Thr Trp Cys Pro Val Glu Asn Gly Pro His Pro Thr Ala Pro Pro Lys
   580 585 590
   Pro Ala Ala Pro Glu Ile Thr Ala Thr Phe Thr Leu Pro Asn Thr Tyr
   595 600 605
   Thr Cys Ser Gln Gly Lys Asn Thr Cys Ser Asn Pro Lys Thr Ala Pro
   610 615 620
   Asn Val Ile Val Val Thr Pro Ile Val Thr Gln Thr Ala Pro Val Val
   625 630 635 640
   Ile Pro Gly Ile Ala Ala Pro Thr Pro Thr Pro Ser Val Ala Ala Ser
   645 650 655
   Ser Pro Ala Ser Pro Ser Val Val Pro Ser Pro Thr Ala Pro Val Ala
   660 665 670
   Thr Ser Pro Ala Gln Ser Ala Tyr Tyr Pro Pro Pro Pro Pro Pro Glu
   675 680 685
   His Ala Val Ser Thr Pro Val Ala Asn Pro Pro Ala Val Thr Pro Ala
   690 695 700
   Pro Ala Pro Phe Pro Ser Gly Gly Leu Thr Thr Val Ile Ala Pro Gly
   705 710 715 720
   Ser Thr Gly Val Pro Ser Gln Pro Ala Gln Ser Gly Leu Pro Pro Val
   725 730 735
   Pro Ala Gly Ala Ala Gly Phe Arg Ala Pro Ala Ala Val Ala Leu Leu
   740 745 750
   Ala Gly Ala Val Ala Ala Ala Leu Leu
               755                 760
<210> 87
   <211> 526
   <212> PRT
   <213> Trichoderma reesei
<400> 87
   Met Arg Pro Asn Ser Val Leu Leu Ala Pro Leu Ala Leu Tyr Ala Ser
   1               5                   10                  15
   Gly Ala Leu Ala Phe Tyr Pro Tyr Thr Pro Pro Trp Leu Lys Glu Leu
               20                  25                  30
   Glu Glu His Asn Ala Gly Glu Ala Lys Arg Ser Ala Asp Asn Gly Leu
           35                  40                  45
   Thr Phe Asp Ile Lys Arg Arg Ala Ser Arg Arg Ala Pro Ala Ser Gln
       50                  55                  60
   Glu Glu Lys Ala Ala Trp Gln Ala Ala Leu Leu Ser His Lys Tyr Ser
   65                  70                  75                  80
   Glu Ser Val Thr Pro Ser Pro Ser Pro Asp Thr Thr Leu Ser Lys Arg
                   85                  90                  95
   Asp Asn Gln Phe Ser Ile Leu Lys Ala Val Asp Pro Asp Ala Pro Asn
               100                 105                 110
   Thr Ala Gly Leu Ala Gln Asp Gly Thr Asp Tyr Ser Tyr Phe Val Gln
           115                 120                 125
   Ala Ser Leu Gly Ser Lys Lys Thr Lys Leu Tyr Met Leu Leu Asp Thr
       130                 135                 140
   Gly Ala Gly Ser Ser Trp Val Met Gly Thr Asp Cys Val Ser Glu Ala
   145                 150                 155                 160
   Cys Ser Leu His Asp Ser Phe Gly Pro Glu Asp Ser Asp Thr Leu Lys
                   165                 170                 175
   Thr Ser Thr Lys Asp Phe Ser Ile Ala Tyr Gly Ser Gly Ala Val Ser
               180                 185                 190
   Gly Ser Leu Val Asn Asp Thr Ile Glu Val Ala Gly Met Ser Leu Thr
           195                 200                 205
   Tyr Gln Phe Gly Leu Ala His Asn Thr Ser Ser Asp Phe Val His Phe
       210                 215                 220
   Ala Phe Asp Gly Ile Leu Gly Met Ser Met Asn Ser Gly Ala Asn Glu
   225                 230                 235                 240
   Asn Phe Leu Ser Ala Leu Glu Gly Ala Gly Leu Leu Asp Lys Ser Ile
                   245                 250                 255
   Phe Ser Val Ala Leu Ala Arg Ala Ser Asp Gly His Asn Asp Gly Glu
               260                 265                 270
   Val Thr Phe Gly Ala Thr Asn Pro Ser Arg Tyr Thr Gly Asp Ile Thr
           275                 280                 285
   Tyr Thr Pro Ile Pro Ser Gly Thr Asp Trp Ser Ile Pro Leu Asp Asp
       290                 295                 300
   Met Ser Tyr Asn Gly Lys Lys Gly Asn Val Gly Gly Ile Asn Ala Tyr
   305                 310                 315                 320
   Ile Asp Thr Gly Thr Ser Tyr Met Phe Gly Pro Ser Lys Asn Val Lys
                   325                 330                 335
   Ala Leu His Ala Val Ile Asp Gly Ala Lys Ser Ser Asp Gly Ile Thr
               340                 345                 350
   Trp Thr Val Pro Cys Asp Thr Thr Thr Pro Leu Val Val Thr Phe Ser
           355                 360                 365
   Gly Val Asp Phe Ala Ile Ser Pro Lys Asp Trp Ile Ser Pro Lys Asp
       370                 375                 380
   Ser Ser Gly Lys Cys Thr Ser Asn Val Tyr Gly Tyr Glu Val Val Ser
   385                 390                 395                 400
   Gly Ser Trp Leu Phe Gly Asp Thr Phe Leu Lys Asn Val Tyr Ala Val
                   405                 410                 415
   Phe Asp Lys Glu Gln Met Arg Ile Gly Lys Thr Ser Pro Arg Ala Thr
               420                 425                 430
   Ser Pro Ser Ser Pro Ala Pro Thr Arg Thr Pro Ser Pro Ala Thr Thr
           435                 440                 445
   Ser Pro Ser Ser Ala Ser Thr Pro Gly Ser Thr Pro Thr Thr Ser Ser
       450                 455                 460
   Thr Arg Thr Ala Arg Pro Ser Thr Ser Ala Pro Ser Gly Thr Ser Ser
   465 470 475 480
   Thr Gly Ala Pro Ser Pro Ser Ala Ser Ala Asn Arg Asp Val Leu Arg
   485 490 495
   Ala Lys Arg Ile Asn Met Leu Lys Ser Ile Ser Ser Phe Trp His Asp
   500 505 510
   Pro Cys Cys Cys Leu Phe Leu His Val Ser Ile Ser Ser Thr
   515 520 525
<210> 88
   <211> 2559
   <212> DNA
   <213> Leishmania mexicana
<400> 88
   atggggaaaa ataaggcaaa ttcagtggcc gactccggct ctgcggcaac cgcacctcgt      60
   gaagctcctg cccaagccaa agatgccgcc ccacaagccc agaccgcatc tccaccgcct     120
   aagaagactt tgttgcccaa aacgctaaca gatgagacgg aatttgtcgg catctttccg     180
   ttccctttct ggccagtacg gttcgtcgtt acggtggtgg cactcttcgg cttaggcgcc     240
   agctgcctcc aagccttcac ggttcgcatg acctcggtta agatttacgg atacctgatc     300
   cacgagttcg acccgtggtt caactaccgc gctgccgagt acatgtccac gcacggctgg     360
   tccgccttct tcagctggtt cgactacatg agctggtacc cgctgggccg ccccgtcggc     420
   tccaccacgt acccgggcct gcagttcact gccgtcgcca ttcaccgcgc actggcggct     480
   gccggcatcc cgatgtctct caacgacgtg tgtgtgctga tcccggcgtg gtttggcgcc     540
   atcgctaccg ctcttctggc tctttgcacg tacgaagcca gtgggtcgac ggtggcggcc     600
   gccgctgccg ccctctcctt ctccatcatc ccagcccacc tgatgcggtc catggcgggt     660
   gagttcgaca acgagtgcat cgccgtcgcc gccatgctgc tcaccttcta ctgctgggtg     720
   cgctcgctgc gcacgcggtc ctcgtggccc atcggcgtcc tcaccggtgt cgcctacggc     780
   tacatggtgg cggcgtgggg cggctacatt ttcgtgctca acatggttgc catgcatgcc     840
   ggcatatcat cgatggtgga ctgggcccgc aacacgtaca acccgtcgct gctgcgtgca     900
   tacacgctgt tctacgttgt cggcaccgcc atcgccgtgt gcgtgccgcc agtggggatg     960
   tcgcccttca agtcgctgga gcagctgggt gcgctgctgg tgcttgtctt cctgtgcggg    1020
   ctgcaggtgt gcgaggtgct gcgggcacgc gccggtgtcg aggttcgctc tcgcgcgaac    1080
   ttcaagatcc gcgcgcgcgt cttcagcgcg atggctggcg gggctgcgct tgcaatcgcg    1140
   ctgctggcac cgagggggta cttcgggccc ctttcggctc gtgtgcgtgc gctgttcgtg    1200
   gagcacacgc gcactggcaa tccgctggtc gactcggtcg ccgaacatca acccgccagc 1260
   cctgaggcaa tgtggtcgtt tcttcacgtg tgcggcgtga catggggctt gggcttcatt 1320
   gtgcttgctg tctcaacgtt cgtgaactac tccccgtcga aggtcttctg ggtactgaac 1380
   tctggtgccg tgtactactt cagcacccgc atggctcggc tgctgcttct ctccggtccc 1440
   gctgcgtgtc tgtccactgg cattttcgtg ggggcaattc tggaagcagc ggtgcagctc 1500
   agcttttggg acagtgatgc gacaaaggcc aagccccaga agcagaccca acgccaccag 1560
   aggggggctc gtaaggacaa caagcgaaat gacgctgaga gcggaatgac cgcgctctca 1620
   ctttgcgaca tcgtgtccgg tagctctctg gcttggggcc atcgtatggt gctgtgcatc 1680
   gctatgtggg ctctcgtgac gacaaccgtg gtgaccttca tcagttccgg tttcgcgtcc 1740
   cactcactaa aatttgcgga gcagtcgtca aatccgatga ttgttttcgc ggcctccgtg 1800
   ccaaaccgtg caacaggcaa gcctatgatg atattggtgg atgactacct gcacagctat 1860
   ctctggctgc gcgataacac acccaggagt gcgcgcattt tggcctggtg ggactacggc 1920
   taccagatca caggcatcgg caaccgcacc tcgctggccg atggcaacac ctggaaccac 1980
   gagcacatcg ccaccatcgg caagatgttg acgtcgcccg tggcggaggc gcactcgctg 2040
   gtgcgccaca tggccgacta cgtcctcatc tgggctgggc agagcggaga cttgatgaag 2100
   tcaccgcaca tggcgcgcat cggcaacagt gtgtaccacg acatctgccc caacgacccg 2160
   ctgtgccagc aattcggctt ttacagaaat gattaccatc gtccaacacc gatgatgcgg 2220
   gcgtcgctgc tgtacaacct gcacgaggcc gggaaaacag cggccgtgaa ggtggaccca 2280
   tccctctttc aggaggtgta ctcgtccaag tacggcctgg tgcgcatctt caaggtcatg 2340
   aacgtgagcg cggagagcaa gaagtgggtt gctgacccgg caaaccgcgt gtgccgcccg 2400
   cctgggtcgt ggatctgccc cgggcagtac ccgccggcga aggagatcca ggagatgctg 2460
   gcacaccggg tctccttcga tcaggtggac aaggacaaga agcgcaaggc gacgtaccac 2520
   gaggagtaca tgcgccggat gcgtgaaaac gagatctga 2559
<210> 89
   <211> 852
   <212> PRT
   <213> Leishmania mexicana
<400> 89
   Met Gly Lys Asn Lys Ala Asn Ser Val Ala Asp Ser Gly Ser Ala Ala
   1               5                   10                  15
   Thr Ala Pro Arg Glu Ala Pro Ala Gln Ala Lys Asp Ala Ala Pro Gln
               20                  25                  30
   Ala Gln Thr Ala Ser Pro Pro Pro Lys Lys Thr Leu Leu Pro Lys Thr
           35                  40                  45
   Leu Thr Asp Glu Thr Glu Phe Val Gly Ile Phe Pro Phe Pro Phe Trp
       50                  55                  60
   Pro Val Arg Phe Val Val Thr Val Val Ala Leu Phe Gly Leu Gly Ala
   65                  70                  75                  80
   Ser Cys Leu Gln Ala Phe Thr Val Arg Met Thr Ser Val Lys Ile Tyr
                   85                  90                  95
   Gly Tyr Leu Ile His Glu Phe Asp Pro Trp Phe Asn Tyr Arg Ala Ala
               100                 105                 110
   Glu Tyr Met Ser Thr His Gly Trp Ser Ala Phe Phe Ser Trp Phe Asp
           115                 120                 125
   Tyr Met Ser Trp Tyr Pro Leu Gly Arg Pro Val Gly Ser Thr Thr Tyr
       130                 135                 140
   Pro Gly Leu Gln Phe Thr Ala Val Ala Ile His Arg Ala Leu Ala Ala
   145                 150                 155                 160
   Ala Gly Ile Pro Met Ser Leu Asn Asp Val Cys Val Leu Ile Pro Ala
                   165                 170                 175
   Trp Phe Gly Ala Ile Ala Thr Ala Leu Leu Ala Leu Cys Thr Tyr Glu
               180                 185                 190
   Ala Ser Gly Ser Thr Val Ala Ala Ala Ala Ala Ala Leu Ser Phe Ser
           195                 200                 205
   Ile Ile Pro Ala His Leu Met Arg Ser Met Ala Gly Glu Phe Asp Asn
       210                 215                 220
   Glu Cys Ile Ala Val Ala Ala Met Leu Leu Thr Phe Tyr Cys Trp Val
   225                 230                 235                 240
   Arg Ser Leu Arg Thr Arg Ser Ser Trp Pro Ile Gly Val Leu Thr Gly
                   245                 250                 255
   Val Ala Tyr Gly Tyr Met Val Ala Ala Trp Gly Gly Tyr Ile Phe Val
               260                 265                 270
   Leu Asn Met Val Ala Met His Ala Gly Ile Ser Ser Met Val Asp Trp
           275                 280                 285
   Ala Arg Asn Thr Tyr Asn Pro Ser Leu Leu Arg Ala Tyr Thr Leu Phe
       290                 295                 300
   Tyr Val Val Gly Thr Ala Ile Ala Val Cys Val Pro Pro Val Gly Met
   305                 310                 315                 320
   Ser Pro Phe Lys Ser Leu Glu Gln Leu Gly Ala Leu Leu Val Leu Val
                   325                 330                 335
   Phe Leu Cys Gly Leu Gln Val Cys Glu Val Leu Arg Ala Arg Ala Gly
               340                 345                 350
   Val Glu Val Arg Ser Arg Ala Asn Phe Lys Ile Arg Ala Arg Val Phe
           355                 360                 365
   Ser Ala Met Ala Gly Gly Ala Ala Leu Ala Ile Ala Leu Leu Ala Pro
       370                 375                 380
   Arg Gly Tyr Phe Gly Pro Leu Ser Ala Arg Val Arg Ala Leu Phe Val
   385                 390                 395                 400
   Glu His Thr Arg Thr Gly Asn Pro Leu Val Asp Ser Val Ala Glu His
                   405                 410                 415
   Gln Pro Ala Ser Pro Glu Ala Met Trp Ser Phe Leu His Val Cys Gly
               420                 425                 430
   Val Thr Trp Gly Leu Gly Phe Ile Val Leu Ala Val Ser Thr Phe Val
           435                 440                 445
   Asn Tyr Ser Pro Ser Lys Val Phe Trp Val Leu Asn Ser Gly Ala Val
       450                 455                 460
   Tyr Tyr Phe Ser Thr Arg Met Ala Arg Leu Leu Leu Leu Ser Gly Pro
   465                 470                 475                 480
   Ala Ala Cys Leu Ser Thr Gly Ile Phe Val Gly Ala Ile Leu Glu Ala
                   485                 490                 495
   Ala Val Gln Leu Ser Phe Trp Asp Ser Asp Ala Thr Lys Ala Lys Pro
               500                 505                 510
   Gln Lys Gln Thr Gln Arg His Gln Arg Gly Ala Arg Lys Asp Asn Lys
           515                 520                 525
   Arg Asn Asp Ala Glu Ser Gly Met Thr Ala Leu Ser Leu Cys Asp Ile
       530                 535                 540
   Val Ser Gly Ser Ser Leu Ala Trp Gly His Arg Met Val Leu Cys Ile
   545 550 555 560
   Ala Met Trp Ala Leu Val Thr Thr Thr Val Val Thr Phe Ile Ser Ser
   565 570 575
   Gly Phe Ala Ser His Ser Leu Lys Phe Ala Glu Gln Ser Ser Asn Pro
   580 585 590
   Met Ile Val Phe Ala Ala Ser Val Pro Asn Arg Ala Thr Gly Lys Pro
   595 600 605
   Met Met Ile Leu Val Asp Asp Tyr Leu His Ser Tyr Leu Trp Leu Arg
   610 615 620
   Asp Asn Thr Pro Arg Ser Ala Arg Ile Leu Ala Trp Trp Asp Tyr Gly
   625 630 635 640
   Tyr Gln Ile Thr Gly Ile Gly Asn Arg Thr Ser Leu Ala Asp Gly Asn
   645 650 655
   Thr Trp Asn His Glu His Ile Ala Thr Ile Gly Lys Met Leu Thr Ser
   660 665 670
   Pro Val Ala Glu Ala His Ser Leu Val Arg His Met Ala Asp Tyr Val
   675 680 685
   Leu Ile Trp Ala Gly Gln Ser Gly Asp Leu Met Lys Ser Pro His Met
   690 695 700
   Ala Arg Ile Gly Asn Ser Val Tyr His Asp Ile Cys Pro Asn Asp Pro
   705 710 715 720
   Leu Cys Gln Gln Phe Gly Phe Tyr Arg Asn Asp Tyr His Arg Pro Thr
   725 730 735
   Pro Met Met Arg Ala Ser Leu Leu Tyr Asn Leu His Glu Ala Gly Lys
   740 745 750
   Thr Ala Ala Val Lys Val Asp Pro Ser Leu Phe Gln Glu Val Tyr Ser
   755 760 765
   Ser Lys Tyr Gly Leu Val Arg Ile Phe Lys Val Met Asn Val Ser Ala
   770 775 780
   Glu Ser Lys Lys Trp Val Ala Asp Pro Ala Asn Arg Val Cys Arg Pro
   785 790 795 800
   Pro Gly Ser Trp Ile Cys Pro Gly Gln Tyr Pro Pro Ala Lys Glu Ile
                   805                 810                 815
   Gln Glu Met Leu Ala His Arg Val Ser Phe Asp Gln Val Asp Lys Asp
               820                 825                 830
   Lys Lys Arg Lys Ala Thr Tyr His Glu Glu Tyr Met Arg Arg Met Arg
           835                 840                 845
   Glu Asn Glu Ile
       850
<210> 90
   <211> 2565
   <212> DNA
   <213> Leishmania braziliensis
<400> 90
   atgggtaaga agaaagcaat tccgtcgggc agcgtcggcc ctgcgacaac cacctcccgt      60
   gaagctccag gcaaagacga aggtgcctcc caacccgcca agactgcagc tctgccggtg     120
   aagccctttg tgttgcccaa cacgctgaca gacgaggagg agtttgttgg catctttccc     180
   tgccctttct ggccagtgcg atttgtcatc acagtgatgg cactcgtcct cttgggtgcc     240
   agctgtatcc gcgccttcac gattcgcatg ctatccgttc agctttatgg ctacatcatc     300
   cacgagttcg acccgtggtt caactaccgc gccgccgagt acatgtccgc gcacggctgg     360
   tccgccttct tcagctggtt cgactacatg agctggtacc cgctgggccg ccccgttggc     420
   accaccacgt acccgggcct gcagctcacc gccgttgcca tccaccgcgc attggcggct     480
   gccggggtgc cgatgtctct caacaacgtg tgcgtgctga tccccgcgtg gtatggtgcc     540
   atcgctactg ctatcctggc cctttgcgct tacgaggtca gtaggtcaat ggtagcggcg     600
   gctgttgctg cactctcatt ctccatcatt ccagcacacc tgatgcggtc catggcgggc     660
   gagttcgaca acgagtgcat cgccgttgca gccatgctcc tcaccttcta cttgtgggta     720
   cgctcgctgc gcacgcggtg ctcgtggccc atcggcatcc tcaccggtat cgcctacggc     780
   tacatggtgg cggcgtgggg cggatacatt tttgtgctca acatggttgc catgcacgcc     840
   ggcatatcat cgatggtcga ctgggctcgc aacacgtaca acccgtcgct gctgcgcgca     900
   tacgcgctgt tctacgttgt cggcaccgcc atcgccacgc gcgtgccgcc tgtggggatg     960
   tcgcccttca ggtcgctgga gcagctgggt gcgctggcgg tgctcctctt cctgtgcggg    1020
   ctgcaggcct gcgaggtgtt tcgcgcacgg gccgacgtcg aggttcgctc ccgcgcgaac    1080
   ttcaagatcc gcatgcgtgc cttcagcgtg atggctggcg tgggtgcgct tgcaatcgcg    1140
   gtgctgtcgc cgaccgggta ctttggcccc ctcacggctc gtgtgcgtgc gctgttcatg    1200
   gagcacacgc gcactggcaa tccgctggtc gactcggtcg ctgagcacca ccccgccagt 1260
   cctgaggcga tgtggacatt tcttcacgtg tgcggcgtga cttggggttt gggctccatt 1320
   gttcttcttg tgtcgttgct ggtggactac tcctcggcaa agctcttttg gctgatgaac 1380
   tctggtgccg tgtactattt cagcacccgc atgtcacgac tgctgcttct cacgggcccc 1440
   gctgcgtgtc tgtccactgg ctgtttcgtg gggacattac tggaagcggc gatacagttc 1500
   accttctggt ccagcgatgc aacaaaggcc aaaaaacagc aagagacaca acttcaccaa 1560
   aagggcgcgc gcaagcatag cgaccggagt aactctaaga atgcactgac tgtgcgtaca 1620
   ttgggcgacg tcttgaggag tacctctctg gcatggggtc atcgcatggt gctctgcttc 1680
   gctatgtggg ctcttgttat tacagtcgcg gtgtgcctct tgggttccga tttcacttcc 1740
   catgcaacga tgtttgcaag gcagacgtcg aacccgctga ttgtctttgc aaccgtgctg 1800
   cgagaccgcg ctaccggcaa gccaacacag gtattggtgg atgactacct gcgcagctat 1860
   ctctggctgc gcgacaacac gcccagaaat gcgcgcgtgc tgtcctggtg ggactacggc 1920
   taccagatca caggtatcgg caaccgcacc tcgctggccg atggcaacac ctggaaccac 1980
   gagcacatcg ccaccatcgg caagatgctg acgtcgcccg tggcggaggc gcactcactg 2040
   gtgcgccaca tggcggacta cgtcctcatc tgggctgggc agggcggaga cttgatgaag 2100
   tcgccgcaca tggcgcgcat tggcaacagc gtgtaccacg acatctgccc caacgacccg 2160
   ctttgccagc atttcggctt ttacaagaac gatcgcaatc gcccaaaacc gatgatgcgc 2220
   gcgtcgctgc tgtacaacct gcacgaggcc ggacgaagcg cgggtgtgaa ggtggacccg 2280
   tccctctttc aggaagtgta ctcatccaag tacggcctgg tgcgcatctt caaggtcatg 2340
   aacgtgagcg cggagagcaa gaagtgggtg gctgacccgg caaaccgcgt gtgccacccg 2400
   cctgggtcgt ggatctgccc cgggcagtac ccgccggcga aggagatcca ggagatgctg 2460
   gcgcaccgcg tcccctttga ccatgtgaac agcttcagtc ggaaaaaggc cgggtcttat 2520
   catgaagaat acatgcgccg gatgcgtgaa gagcaggacc gatga 2565
<210> 91
   <211> 854
   <212> PRT
   <213> Leishmania braziliensis
<400> 91
   Met Gly Lys Lys Lys Ala Ile Pro Ser Gly Ser Val Gly Pro Ala Thr
   1               5                   10                  15
   Thr Thr Ser Arg Glu Ala Pro Gly Lys Asp Glu Gly Ala Ser Gln Pro
               20                  25                  30
   Ala Lys Thr Ala Ala Leu Pro Val Lys Pro Phe Val Leu Pro Asn Thr
           35                  40                  45
   Leu Thr Asp Glu Glu Glu Phe Val Gly Ile Phe Pro Cys Pro Phe Trp
       50                  55                  60
   Pro Val Arg Phe Val Ile Thr Val Met Ala Leu Val Leu Leu Gly Ala
   65                  70                  75                  80
   Ser Cys Ile Arg Ala Phe Thr Ile Arg Met Leu Ser Val Gln Leu Tyr
                   85                  90                  95
   Gly Tyr Ile Ile His Glu Phe Asp Pro Trp Phe Asn Tyr Arg Ala Ala
               100                 105                 110
   Glu Tyr Met Ser Ala His Gly Trp Ser Ala Phe Phe Ser Trp Phe Asp
           115                 120                 125
   Tyr Met Ser Trp Tyr Pro Leu Gly Arg Pro Val Gly Thr Thr Thr Tyr
       130                 135                 140
   Pro Gly Leu Gln Leu Thr Ala Val Ala Ile His Arg Ala Leu Ala Ala
   145                 150                 155                 160
   Ala Gly Val Pro Met Ser Leu Asn Asn Val Cys Val Leu Ile Pro Ala
                   165                 170                 175
   Trp Tyr Gly Ala Ile Ala Thr Ala Ile Leu Ala Leu Cys Ala Tyr Glu
               180                 185                 190
   Val Ser Arg Ser Met Val Ala Ala Ala Val Ala Ala Leu Ser Phe Ser
           195                 200                 205
   Ile Ile Pro Ala His Leu Met Arg Ser Met Ala Gly Glu Phe Asp Asn
       210                 215                 220
   Glu Cys Ile Ala Val Ala Ala Met Leu Leu Thr Phe Tyr Leu Trp Val
   225                 230                 235                 240
   Arg Ser Leu Arg Thr Arg Cys Ser Trp Pro Ile Gly Ile Leu Thr Gly
                   245                 250                 255
   Ile Ala Tyr Gly Tyr Met Val Ala Ala Trp Gly Gly Tyr Ile Phe Val
               260                 265                 270
   Leu Asn Met Val Ala Met His Ala Gly Ile Ser Ser Met Val Asp Trp
           275                 280                 285
   Ala Arg Asn Thr Tyr Asn Pro Ser Leu Leu Arg Ala Tyr Ala Leu Phe
       290                 295                 300
   Tyr Val Val Gly Thr Ala Ile Ala Thr Arg Val Pro Pro Val Gly Met
   305                 310                 315                 320
   Ser Pro Phe Arg Ser Leu Glu Gln Leu Gly Ala Leu Ala Val Leu Leu
                   325                 330                 335
   Phe Leu Cys Gly Leu Gln Ala Cys Glu Val Phe Arg Ala Arg Ala Asp
               340                 345                 350
   Val Glu Val Arg Ser Arg Ala Asn Phe Lys Ile Arg Met Arg Ala Phe
           355                 360                 365
   Ser Val Met Ala Gly Val Gly Ala Leu Ala Ile Ala Val Leu Ser Pro
       370                 375                 380
   Thr Gly Tyr Phe Gly Pro Leu Thr Ala Arg Val Arg Ala Leu Phe Met
   385                 390                 395                 400
   Glu His Thr Arg Thr Gly Asn Pro Leu Val Asp Ser Val Ala Glu His
                   405                 410                 415
   His Pro Ala Ser Pro Glu Ala Met Trp Thr Phe Leu His Val Cys Gly
               420                 425                 430
   Val Thr Trp Gly Leu Gly Ser Ile Val Leu Leu Val Ser Leu Leu Val
           435                 440                 445
   Asp Tyr Ser Ser Ala Lys Leu Phe Trp Leu Met Asn Ser Gly Ala Val
       450                 455                 460
   Tyr Tyr Phe Ser Thr Arg Met Ser Arg Leu Leu Leu Leu Thr Gly Pro
   465                 470                 475                 480
   Ala Ala Cys Leu Ser Thr Gly Cys Phe Val Gly Thr Leu Leu Glu Ala
                   485                 490                 495
   Ala Ile Gln Phe Thr Phe Trp Ser Ser Asp Ala Thr Lys Ala Lys Lys
               500                 505                 510
   Gln Gln Glu Thr Gln Leu His Gln Lys Gly Ala Arg Lys His Ser Asp
           515                 520                 525
   Arg Ser Asn Ser Lys Asn Ala Leu Thr Val Arg Thr Leu Gly Asp Val
       530                 535                 540
   Leu Arg Ser Thr Ser Leu Ala Trp Gly His Arg Met Val Leu Cys Phe
   545 550 555 560
   Ala Met Trp Ala Leu Val Ile Thr Val Ala Val Cys Leu Leu Gly Ser
   565 570 575
   Asp Phe Thr Ser His Ala Thr Met Phe Ala Arg Gln Thr Ser Asn Pro
   580 585 590
   Leu Ile Val Phe Ala Thr Val Leu Arg Asp Arg Ala Thr Gly Lys Pro
   595 600 605
   Thr Gln Val Leu Val Asp Asp Tyr Leu Arg Ser Tyr Leu Trp Leu Arg
   610 615 620
   Asp Asn Thr Pro Arg Asn Ala Arg Val Leu Ser Trp Trp Asp Tyr Gly
   625 630 635 640
   Tyr Gln Ile Thr Gly Ile Gly Asn Arg Thr Ser Leu Ala Asp Gly Asn
   645 650 655
   Thr Trp Asn His Glu His Ile Ala Thr Ile Gly Lys Met Leu Thr Ser
   660 665 670
   Pro Val Ala Glu Ala His Ser Leu Val Arg His Met Ala Asp Tyr Val
   675 680 685
   Leu Ile Trp Ala Gly Gln Gly Gly Asp Leu Met Lys Ser Pro His Met
   690 695 700
   Ala Arg Ile Gly Asn Ser Val Tyr His Asp Ile Cys Pro Asn Asp Pro
   705 710 715 720
   Leu Cys Gln His Phe Gly Phe Tyr Lys Asn Asp Arg Asn Arg Pro Lys
   725 730 735
   Pro Met Met Arg Ala Ser Leu Leu Tyr Asn Leu His Glu Ala Gly Arg
   740 745 750
   Ser Ala Gly Val Lys Val Asp Pro Ser Leu Phe Gln Glu Val Tyr Ser
   755 760 765
   Ser Lys Tyr Gly Leu Val Arg Ile Phe Lys Val Met Asn Val Ser Ala
   770 775 780
   Glu Ser Lys Lys Trp Val Ala Asp Pro Ala Asn Arg Val Cys His Pro
   785                 790                 795                 800
   Pro Gly Ser Trp Ile Cys Pro Gly Gln Tyr Pro Pro Ala Lys Glu Ile
                   805                 810                 815
   Gln Glu Met Leu Ala His Arg Val Pro Phe Asp His Val Asn Ser Phe
               820                 825                 830
   Ser Arg Lys Lys Ala Gly Ser Tyr His Glu Glu Tyr Met Arg Arg Met
           835                 840                 845
   Arg Glu Glu Gln Asp Arg
       850